# EUROPEAN PATENT APPLICATION

(11) **EP 4 737 574 A2**
(43) Date of publication of application: **06.05.2026**
(21) Application number: 26165913.0
(22) Date of filing: 18.01.2023
(51) Int. Cl.: C12N 15/67

(54) **POLY-TAILED AND POLY-CAPPED MRNA AND USES THEREOF**

(30) Priority: 18.01.2022 US 202263300602 P
(62) Divisional of application: 23707228.5
(71) Applicant: The Broad Institute Inc., Cambridge, MA 02142 (US); Massachusetts Institute of Technology, Cambridge, MA 02139-4301 (US)
(72) Inventor: ADITHAM, Abhishek, Cambridge, 02139 (US); CHEN, Hongyu, Cambridge, 02139 (US); GUO, Jianting, Cambridge, 02139 (US); WANG, Xiao, Cambridge, 02142 (US)
(74) Representative: Impact Intellectual Property LLP

(57) **Abstract**

Disclosed herein are modified mRNAs with poly(A) tails containing one or more additional poly-A tails or 5' caps, which may be made by ligation of nucleic acids onto the 3' terminal end or 5' terminal end of an RNA, respectively. Also provided are compositions comprising one or more modified mRNAs provided herein, and methods of using said compositions for therapeutic or agricultural applications.

## Description

### REFERENCE TO AN ELECTRONIC SEQUENCE LISTING

The contents of the electronic sequence listing (B119570144WO00-SEQ-JQM.xml; Size: 61,200 bytes; and Date of Creation: January 18, 2023) is herein incorporated by reference in its entirety.

### BACKGROUND OF THE INVENTION

Messenger RNA (mRNA) technology is an emerging alternative to conventional small molecule, DNA, and protein therapeutics and conventional vaccine approaches because it is potent, programmable, and capable of rapid production of mRNAs with desired sequences. mRNA therapeutics are a rapidly developing field and have been used for the expression of therapeutic proteins, ranging from vascular regeneration factors (e.g., vascular endothelial growth factor A (VEGF-A), GATA Binding Protein 4 (GATA4), Myocyte Enhancer Factor 2C (MEF2C), T-Box Transcription Factor 5 (TBX5), myocardin (MYOCD)) to vaccines for COVID-19, influenza, and Zika virus. Despite recent clinical successes, mRNA therapeutics still faces challenges of instability, toxicity, short-term efficacy, and potential immunological responses. Increasing the stability of mRNAs to enhance their efficacy *in vivo* remains an important problem that must be solved to increase the feasibility of mRNA therapeutics for clinical applications.

### SUMMARY OF THE INVENTION

Provided herein are modified mRNAs with modified poly-A tails and/or modified 5' caps to improve the stability of the mRNA in cells and in *in vitro* translation systems, thereby enhancing protein production, as well as methods of making and using such modified mRNAs. Conventional mRNAs comprise poly-A tails which include approximately 100-250 adenosine nucleotides at the 3' end. Poly-A tails can be degraded or removed by cellular exonucleases, which remove 3' nucleotides. Once exonucleases remove the poly-A tail and begin removing nucleotides of the 3' untranslated region (UTR) and/or open reading frame (ORF), the mRNA is unable to be translated into an encoded protein. mRNAs that are more resistant to 3' exonuclease activity are degraded more slowly and are thus more stable, having increased half-lives in cells, and therefore more protein can be produced from a given mRNA molecule. A poly-A tail is also required for binding to the poly-A binding protein (PABP), which enhances translation of mRNA molecules by binding to the eukaryotic initiation factor 4 complex (eIF4G). Conventional mRNAs also include a 5' cap which, similar to the poly-A tail, protects mRNAs from 5' exonucleases and serves as a site for the recruitment of translation machinery. Modified nucleotides containing one or more structural modifications to the nucleobase, sugar, or phosphate linkage of the mRNA can interfere with 3' and 5' exonuclease activity, rendering the mRNA more stable. However, the same structural modifications that inhibit exonucleases can also hinder the ability of enzymes to incorporate these modified nucleotides into the mRNA, making the modified mRNA difficult to produce.

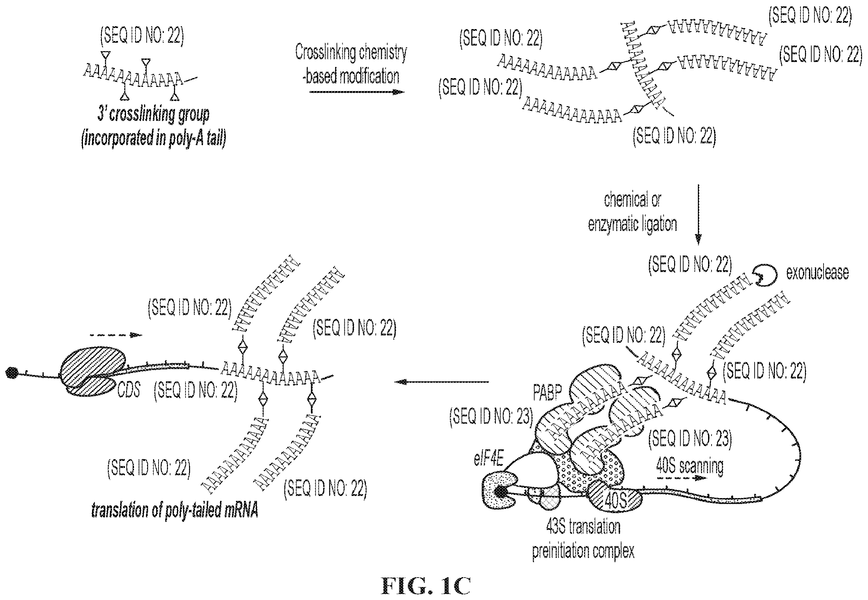

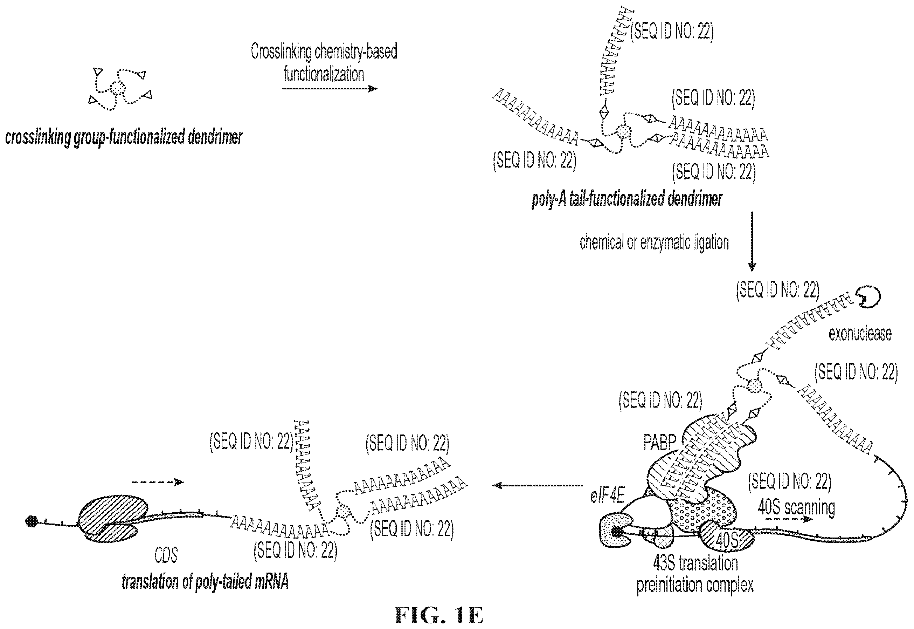

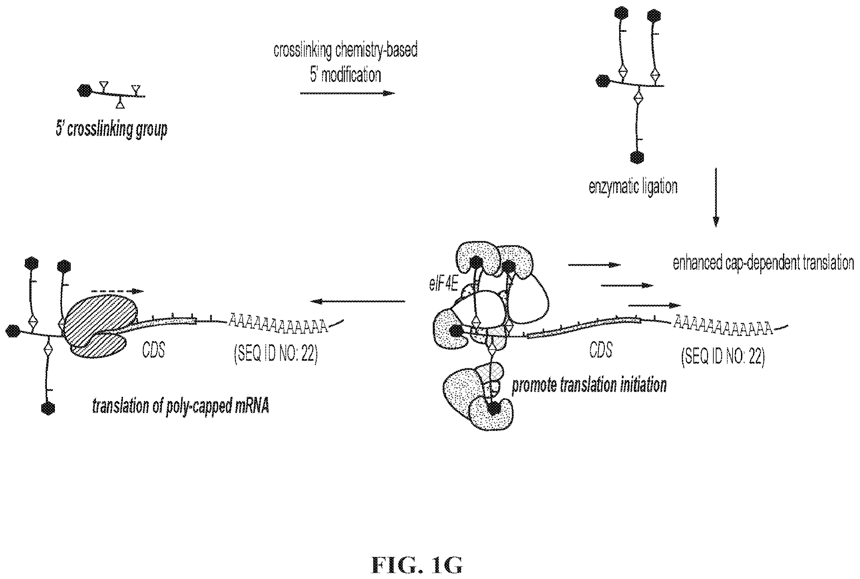

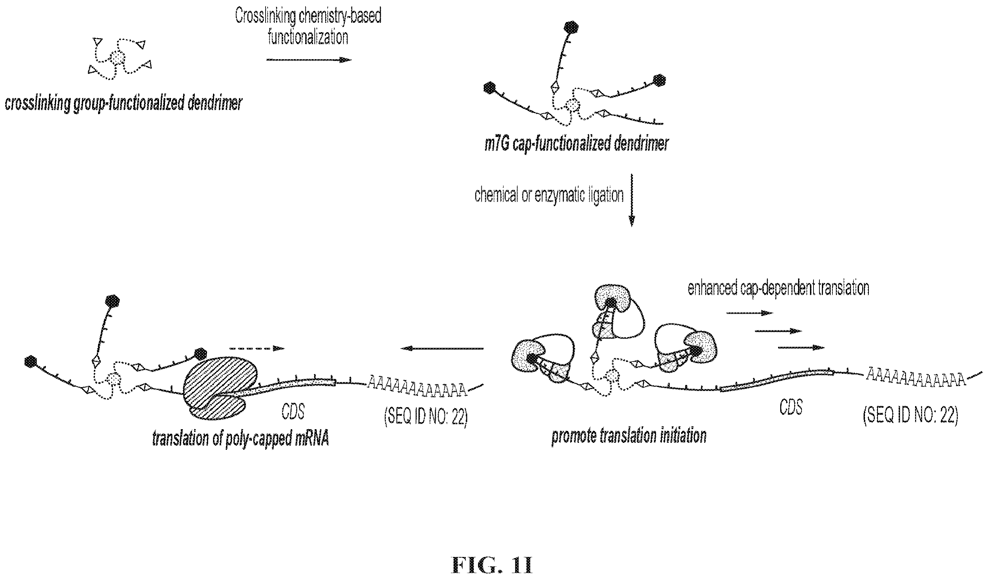

Nevertheless, alternate strategies for enhancing the stability of mRNA molecules without significantly impairing the synthesis and/or translation of the mRNAs have been demonstrated. Disclosed herein are strategies for enhancing mRNA stability by either ligating multiple poly-A tails onto the 3' end of a mRNA, and/or ligating multiple 5' caps onto the 5' end. One strategy involves ligating onto the 3' and/or 5' end of an existing mRNA molecule an oligonucleotide comprising one or more nucleotides modified with click chemistry handles. In turn, additional oligonucleotides comprising either 3' poly-A tails or 5' caps are ligated to the one or more modified nucleotides via click chemistry reactions (see **FIGs. 1C** and **1G**). Alternately, the same result may be achieved by ligating onto the 3' and/or 5' end of an existing mRNA molecule an oligonucleotide that is attached to a dendrimer through a click chemistry reaction. In turn, the dendrimer, which is also modified with click chemistry handles, is ligated to additional oligonucleotides comprising either 3' poly-A tails or 5' caps via click chemistry reactions (see **FIGs. 1E** and **1I**). In addition to prolonging mRNA lifetime by protecting existing mRNA molecules from exonuclease activity, thereby reducing degradation of the ORF, these strategies may also enhance translation of mRNAs directly, as each additional poly-A tail or 5' cap ligated to the mRNA can interact with and recruit translation machinery to the modified mRNA. These strategies are amenable with one another, and also with other techniques known for enhancing mRNA stability, including, for example, the introduction of modified nucleotides at the 3' and/or 5' ends of a mRNA, the introduction of modified nucleotides into the 3' and/or 5' untranslated regions (UTRs) of an mRNA, the introduction of exonuclease-resistant sequences into a mRNA, and/or circularization by ligating the terminal ends of a linear mRNA to produce a circular mRNA.

Accordingly, the present disclosure provides, in some aspects, a modified mRNA comprising:
(i) an open reading frame (ORF) encoding a protein;
(ii) a poly-A region, and
(iii) a 5' cap region;
wherein the poly-A region is 3' to the ORF and comprises 10 or more nucleotides and the 5' cap region is 5' to the ORF, and wherein the poly-A region comprises two or more poly-A tails and/or the 5' cap region comprises two or more 5' caps.

In some embodiments, the modified mRNA comprises a poly-A region comprising two or more poly-A tails and a 5' cap region comprising one 5' cap. In some embodiments, the modified mRNA comprises a poly-A region comprising one poly-A tail and a 5' cap region comprising two or more 5' caps. In some embodiments, the modified mRNA comprises a poly-A region comprising two or more poly-A tails and a 5' cap region comprising two or more 5' caps.

In some embodiments, the modified mRNA comprises a 5' untranslated region (5' UTR) and a 3' untranslated region (3' UTR), wherein the ORF is between the 5' UTR and the 3' UTR, wherein the 3' UTR is between the ORF and the poly-A region, and wherein the 5' UTR is between the 5' cap region and the ORF.

In some embodiments, the modified mRNA is a linear mRNA, wherein the 5' cap region is at the 5' end of the modified mRNA and the poly-A region is at the 3' end of the modified mRNA.

In some embodiments, the modified mRNA is a circular mRNA, wherein the poly-A region is between the 3' UTR and the 5' cap region.

In some embodiments, the poly-A region comprises two or more poly-A tails, and at least two instances of the poly-A tails are covalently linked by a first linker.

In some embodiments, the poly-A region comprises -(a first poly-A tail)-[(a first linker)-(a second poly-A tail)ₙ₁]ₙ₂; wherein each instance of n1 is independently an integer between 1 and 20, inclusive, n2 is an integer between 2 and 10, inclusive, the first poly-A tail is covalently linked to the 3' UTR, and the first linker is covalently linked to the first poly-A tail and to the second poly-A tail.

In some embodiments, each instance of n1 is 1.

In some embodiments, at least one instance of the first linker is covalently linked to an internal nucleotide of the first poly-A tail.

In some embodiments, at least one instance of the first linker is covalently linked to a second poly-A tail at the 3' nucleotide of the second poly-A tail.

In some embodiments, at least one instance of the first linker comprises at least one moiety formed by reacting two orthogonal click-chemistry handles.

In some embodiments, at least one moiety formed by reacting two orthogonal click-chemistry handles is of the formula:

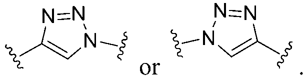

In some embodiments, each instance of the first linker is independently substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, substituted or unsubstituted, C₁₋₂₀₀ heteroalkynylene, substituted or unsubstituted carbocyclylene, substituted or unsubstituted heterocyclylene, substituted or unsubstituted arylene, or substituted or unsubstituted heteroarylene; and optionally one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with substituted or unsubstituted carbocyclylene, substituted or unsubstituted heterocyclylene, substituted or unsubstituted arylene, or substituted or unsubstituted heteroarylene.

In some embodiments, one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with

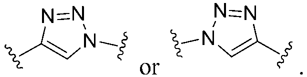

In some embodiments, at least one instance of the first linker is of the formula: 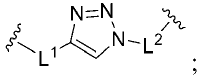 each instance of L¹ is independently substituted or unsubstituted, C₁₋₁₉₉ alkylene or substituted or unsubstituted, C₁₋₁₉₉ heteroalkylene; and each instance of L² is independently substituted or unsubstituted, C₁₋₁₉₉ alkylene or substituted or unsubstituted, C₁₋₁₉₉ heteroalkylene

In some embodiments, at least one instance of the first linker is of the formula:

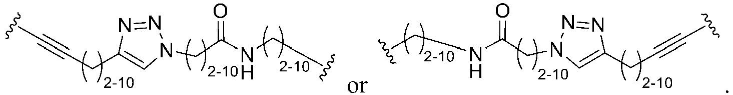

In some embodiments, each instance of n1 is independently an integer between 2 and 20, inclusive.

In some embodiments, at least one instance of the first linker comprises a dendrimer. In certain embodiments, at least one instance of the dendrimer is a first-generation dendrimer. In certain embodiments, at least one instance of the dendrimer is a second-generation dendrimer. In certain embodiments, at least one instance of the dendrimer is a third-generation dendrimer. In certain embodiments, at least one instance of the dendrimer is a fourth-generation dendrimer. In certain embodiments, at least one instance of the dendrimer comprises 2, 3, 4, 5, 6, 7, or 8 dendrons.

In certain embodiments, at least one instance of the dendrimer is a poly-propylene imine dendrimer, poly-propylene amine dendrimer, poly(glutamic acid) dendrimer, polymelamine dendrimer, polyester dendrimer, or pegylated dendrimer. In some embodiments, at least one instance of the dendrimer is a polyamidoamine (PAMAM) dendrimer.

In some embodiments, at least one instance of the PAMAM dendrimer is of the formula: 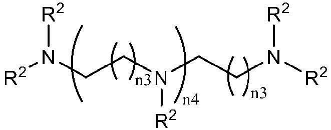 wherein each instance of n3 is independently an integer between 1 and 10, inclusive; each instance of n4 is independently an integer between 0 and 10, inclusive; each instance of R² is independently hydrogen or 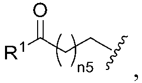 provided that at least three instances of R² is 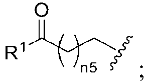 each instance of R¹ is substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, or substituted or unsubstituted, C₁₋₁₀ heteroalkynylene; optionally one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with substituted or unsubstituted carbocyclylene, substituted or unsubstituted heterocyclylene, substituted or unsubstituted arylene, or substituted or unsubstituted heteroarylene; and each instance of n5 is independently an integer between 0 and 10, inclusive.

In some embodiments, at least one instance of the PAMAM dendrimer comprises:

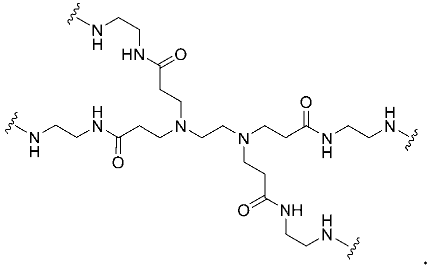

In some embodiments, one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with

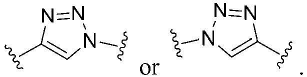

In some embodiments, at least one instance of the first linker is of the formula:

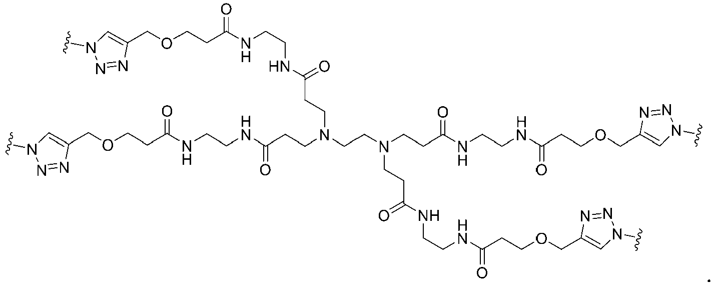

In some embodiments, at least one instance of the first linker is covalently linked to the 3' nucleotide of the first poly-A tail

In some embodiments, at least one instance of the first linker is covalently linked to the 3' nucleotide of the second poly-A tail.

In some embodiments, the poly-A region of the modified mRNA comprises one or more modified nucleotides.

In some embodiments, 3 or more of the last 10 nucleotides of at least one of the two or more poly-A tails are modified nucleotides and/or non-adenosine nucleotides.

In some embodiments, one or more modified nucleotides of the poly-A region are modified adenosine nucleotides.

In some embodiments, one or more modified nucleotides of the poly-A region are modified non-adenosine nucleotides.

In some embodiments, one or more modified nucleotides of the poly-A region comprise a modified nucleobase.

In some embodiments, the modified nucleobase comprised by one or more modified nucleotides of the poly-A region is selected from the group consisting of xanthine, allyaminouracil, allyaminothymidine, hypoxanthine, digoxigeninated adenine, digoxigeninated cytosine, digoxigeninated guanine, digoxigeninated uracil, 6-chloropurineriboside, N6-methyladenine, methylpseudouracil, 2-thiocytosine, 2-thiouracil, 5-methyluracil, 4-thiothymidine, 4-thiouracil, 5,6-dihydro-5-methyluracil, 5,6-dihydrouracil, 5-[(3-Indolyl)propionamide-N-allyl]uracil, 5-aminoallylcytosine, 5-aminoallyluracil, 5-bromouracil, 5-bromocytosine, 5-carboxycytosine, 5-carboxymethylesteruracil, 5-carboxyuracil, 5-fluorouracil, 5-formylcytosine, 5-formyluracil, 5-hydroxycytosine, 5-hydroxymethylcytosine, 5-hydroxymethyluracil, 5-hydroxyuracil, 5-iodocytosine, 5-iodouracil, 5-methoxycytosine, 5-methoxyuracil, 5-methylcytosine, 5-methyluracil, 5-propargylaminocytosine, 5-propargylaminouracil, 5-propynylcytosine, 5-propynyluracil, 6-azacytosine, 6-azauracil, 6-chloropurine, 6-thioguanine, 7-deazaadenine, 7-deazaguanine, 7-deaza-7-propargylaminoadenine, 7-deaza-7-propargylaminoguanine, 8-azaadenine, 8-azidoadenine, 8-chloroadenine, 8-oxoadenine, 8-oxoguanine, araadenine, aracytosine, araguanine, arauracil, biotin-16-7-deaza-7-propargylaminoguanine, biotin-16-aminoallylcytosine, biotin-16-aminoallyluracil, cyanine 3-5-propargylaminocytosine, cyanine 3-6-propargylaminouracil, cyanine 3-aminoallylcytosine, cyanine 3-aminoallyluracil, cyanine 5-6-propargylaminocytosine, cyanine 5-6-propargylaminouracil, cyanine 5-aminoallylcytosine, cyanine 5-aminoallyluracil, cyanine 7-aminoallyluracil, dabcyl-5-3-aminoallyluracil, desthiobiotin-16-aminoallyl-uracil, desthiobiotin-6-aminoallylcytosine, isoguanine, N1-ethylpseudouracil, N1-methoxymethylpseudouracil, N1-methyladenine, N1-methylpseudouracil, N1-propylpseudouracil, N2-methylguanine, N4-biotin-OBEA-cytosine, N4-methylcytosine, N6-methyladenine, O6-methylguanine, pseudoisocytosine, pseudouracil, thienocytosine, thienoguanine, thienouracil, xanthosine, 3-deazaadenine, 2,6-diaminoadenine, 2,6-daminoguanine, 5-carboxamide-uracil, 5-ethynyluracil, N6-isopentenyladenine (i6A), 2-methyl-thio-N6-isopentenyladenine (ms2i6A), 2-methylthio-N6-methyladenine (ms2m6A), N6-(cis-hydroxyisopentenyl)adenine (io6A), 2-methylthio-N6-(cis-hydroxyisopentenyl)adenine (ms2io6A), N6-glycinylcarbamoyladenine (g6A), N6-threonylcarbamoyladenine (t6A), 2-methylthio-N6-threonyl carbamoyladenine (ms2t6A), N6-methyl-N6-threonylcarbamoyladenine (m6t6A), N6-hydroxynorvalylcarbamoyladenine (hn6A), 2-methylthio-N6-hydroxynorvalyl carbamoyladenine (ms2hn6A), N6,N6-dimethyladenine (m62A), and N6-acetyladenine (ac6A).

In some embodiments, one or more modified nucleotides of the poly-A region comprise a modified sugar.

In some embodiments, the modified sugar comprised by one or more modified nucleotides of the poly-A region is selected from the group consisting of 2'-thioribose, 2',3'-dideoxyribose, 2'-amino-2'-deoxyribose, 2' deoxyribose, 2'-azido-2'-deoxyribose, 2'-fluoro-2'-deoxyribose, 2'-O-methylribose, 2'-O-methyldeoxyribose, 3'-amino-2',3'-dideoxyribose, 3'-azido-2',3'-dideoxyribose, 3'-deoxyribose, 3'-O-(2-nitrobenzyl)-2'-deoxyribose, 3'-O-methylribose, 5'-aminoribose, 5'-thioribose, 5-nitro-1-indolyl-2'-deoxyribose, 5'-biotin-ribose, 2'-O,4'-C-methylene-linked, 2'-O,4'-C-amino-linked ribose, and 2'-O,4'-C-thio-linked ribose.

In some embodiments, one or more modified nucleotides of the poly-A region comprise a modified phosphate.

In some embodiments, the modified phosphate comprised by one or more modified nucleotides of the poly-A region is selected from the group consisting of phosphorothioate (PS), thiophosphate, 5'-O-methylphosphonate, 3'-O-methylphosphonate, 5'-hydroxyphosphonate, hydroxyphosphanate, phosphoroselenoate, selenophosphate, phosphoramidate, carbophosphonate, methylphosphonate, phenylphosphonate, ethylphosphonate, H-phosphonate, guanidinium ring, triazole ring, boranophosphate (BP), methylphosphonate, and guanidinopropyl phosphoramidate.

In some embodiments, the poly-A region comprises between 1 and 3, between 3 and 5, between 5 and 10, between 10 and 15, between 15 and 30, between 30 and 50, between 50 and 100, or between 100 and 200 phosphorothioates.

In some embodiments, wherein the poly-A region comprises between 5 and 30 phosphorothioates.

In some embodiments, the poly-A region comprises between 3 and 5, between 5 and 10, between 10 and 15, between 15 and 30, between 30 and 50, between 50 and 100, or between 100 and 200 deoxyribose sugars.

In some embodiments, the poly-A region comprises between 5 and 30 deoxyribose sugars.

In some embodiments, the mRNA comprises a 3' terminal nucleotide wherein the 3' terminal nucleotide is dideoxyadenosine, dideoxycytidine, dideoxyguanosine, dideoxythymidine, dideoxyuridine, or inverted-deoxythymidine.

In some embodiments, the poly-A region comprises between 25 and 500 nucleotides.

In some embodiments, the poly-A region comprises between 50 and 100, between 100 and 150, between 150 and 200, between 200 and 300, between 300 and 400, or between 400 and 500 nucleotides.

In some embodiments, the poly-A region comprises 10 or more adenosine nucleotides.

In some embodiments, 25-100%, 30-100%, 40-100%, 50-100%, 60-100%, 70-100%, 80-100%, 90-100%, 95-100%, 96-100%, 97-100%, 98-100%, or 99-100% of nucleotides of the poly-A region are adenosine nucleotides.

In some embodiments, the first poly-A tail comprises between 10 and 50 adenosine ribonucleotides, between 1 and 10 modified uridine deoxyribonucleotides comprising an attachment point, and a 3' terminal dideoxyribonucleotide or inverted deoxyribonucleotide; and the second poly-A tails comprises between 10 and 50 adenosine ribonucleotides.

In some embodiments, the first poly-A tail comprises a nucleotide sequence set forth as: 5'-rArArArArArAdU(a1)rArArArArArArArArArArArAdU(a1)rArArArArArArArArArArArA dU(a1)ddC-3' (SEQ ID NO: 1); wherein at least one instance of the second poly-A tails comprises a nucleotide sequence set forth as: 5'-rArArArArArArArArArArArArArArArArArArArArArArA rArA*rA*rA*rA*rA*rA*(b1)-3' (SEQ ID NO: 2); and each instance of the first linker is independently 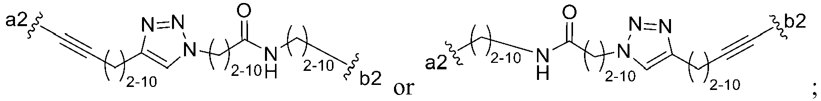 wherein "rA" represents an adenosine ribonucleotide; "dU(a1)" represents a modified uridine deoxyribonucleotide; "ddC" represents a cytosine dideoxyribonucleotide; "*" represents a phosphorothioate linkage; the a1 of each instance of dU(a1) represents an attachment point on the uridine of the dU(a1); the b1 of rA*(bl) represents an attachment point on the * of the rA*(b1); each instance of a1 is attached to an instance of a2; and each instance of b1 is attached to an instance of b2. In some embodiments, the modified uridine deoxyribonucleotide is 5-octadiynyl deoxyuridine.

In some embodiments, the first poly-A tail and the second poly-A tails comprise between 10 and 50 adenosine ribonucleotides and a 3' terminal azide moiety.

In some embodiments, the first poly-A tail and the second poly-A tails comprise between 10 and 50 adenosine ribonucleotides and a 5' terminal azide moiety.

In some embodiments, the first poly-A tail and the second poly-A tails comprise a nucleotide sequence set forth as 5'-rArArArArArArArArArArArArArArArArArArArArArArArArA*rA*rA* rA*rA*rA*/AzideN/- 3' (SEQ ID NO: 3); wherein "AzideN" represents a 3' azide moiety and "*" represents a phosphorothioate linkage.

In some embodiments, the first poly-A tail and the second poly-A tails comprise a nucleotide sequence set forth as 5'-5/AzideN/rArArArArArArArArArArArArArArArArArArArArArArArArA*rA*rA* rA*rA*rA-3' (SEQ ID NO: 27); wherein "AzideN" represents a 3' azide moiety and "*" represents a phosphorothioate linkage.

In some embodiments, the 5' cap region comprises two or more 5' caps, and at least two instances of the 5' caps are covalently linked by a second linker.

In some embodiments, the 5' cap region comprises -(a first 5' cap)-[(a second linker)-(a second 5' cap)ₘ₁]ₘ₂; wherein each instance of m1 is independently an integer between 1 and 20, inclusive; m2 is an integer between 2 and 10, inclusive; the first 5' cap is covalently linked to the 5' UTR; and the second linker is covalently linked to the first 5' cap and to the second 5' cap.

In some embodiments, each instance of m1 is 1.

In some embodiments, at least one instance of the second linker is covalently linked to an internal nucleotide of the first 5' cap.

In some embodiments, at least one instance of the second linker is covalently linked to a second 5' cap at the 3' nucleotide of the second 5' cap.

In some embodiments, at least one instance of the second linker comprises at least one moiety formed by reacting two orthogonal click-chemistry handles.

In some embodiments, at least one moiety formed by reacting two orthogonal click-chemistry handles is of the formula:

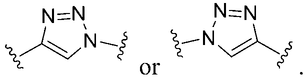

In some embodiments, each instance of the second linker is independently substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, substituted or unsubstituted, C₁₋₂₀₀ heteroalkynylene, substituted or unsubstituted carbocyclylene, substituted or unsubstituted heterocyclylene, substituted or unsubstituted arylene, or substituted or unsubstituted heteroarylene; and optionally one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with substituted or unsubstituted carbocyclylene, substituted or unsubstituted heterocyclylene, substituted or unsubstituted arylene, or substituted or unsubstituted heteroarylene.

In some embodiments, one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with

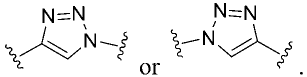

In some embodiments, at least one instance of the second linker is of the formula: 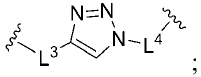 each instance of L³ is independently substituted or unsubstituted, C₁₋₁₉₉ alkylene or substituted or unsubstituted, C₁₋₁₉₉ heteroalkylene; and each instance of L⁴ is independently substituted or unsubstituted, C₁₋₁₉₉ alkylene or substituted or unsubstituted, C₁₋₁₉₉ heteroalkylene.

In some embodiments, at least one instance of the second linker is of the formula:

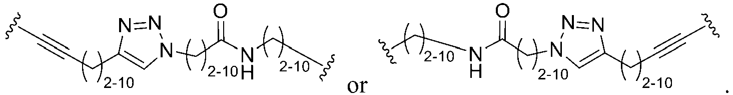

In some embodiments, each instance of m1 is independently an integer between 2 and 20, inclusive.

In some embodiments, at least one instance of the second linker comprises a dendrimer. In certain embodiments, at least one instance of the dendrimer is a first-generation dendrimer. In certain embodiments, at least one instance of the dendrimer is a second-generation dendrimer. In certain embodiments, at least one instance of the dendrimer is a third-generation dendrimer. In certain embodiments, at least one instance of the dendrimer is a fourth-generation dendrimer. In certain embodiments, at least one instance of the dendrimer comprises 2, 3, 4, 5, 6, 7, or 8 dendrons.

In certain embodiments, at least one instance of the dendrimer is a poly-propylene imine dendrimer, poly-propylene amine dendrimer, poly(glutamic acid) dendrimer, polymelamine dendrimer, polyester dendrimer, or pegylated dendrimer. In some embodiments, at least one instance of the dendrimer is a polyamidoamine (PAMAM) dendrimer.

In some embodiments, at least one instance of the PAMAM dendrimer is of the formula: 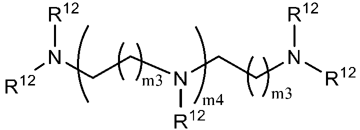 wherein each instance of m3 is independently an integer between 1 and 10, inclusive; each instance of m4 is independently an integer between 0 and 10, inclusive; each instance of R¹² is independently hydrogen or 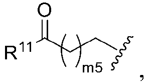 provided that at least three instances of R¹² is 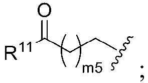 each instance of R¹¹ is substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, or substituted or unsubstituted, C₁₋₁₀ heteroalkynylene; optionally one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with substituted or unsubstituted carbocyclylene, substituted or unsubstituted heterocyclylene, substituted or unsubstituted arylene, or substituted or unsubstituted heteroarylene; and each instance of m5 is independently an integer between 0 and 10, inclusive.

In some embodiments, at least one instance of the PAMAM dendrimer comprises:

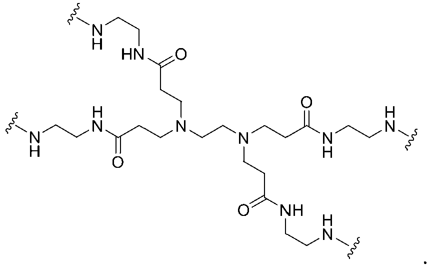

In some embodiments, one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with

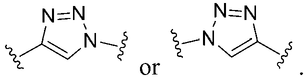

In some embodiments, at least one instance of the first linker is of the formula:

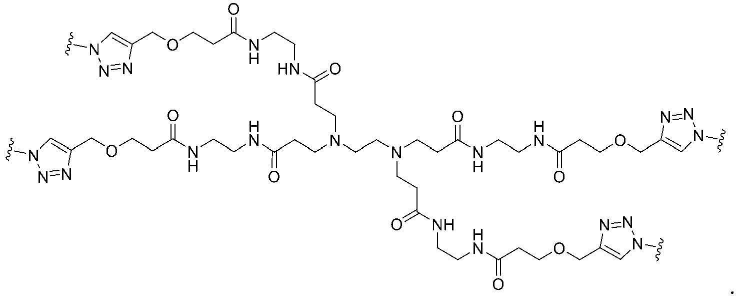

In some embodiments, at least one instance of the second linker is covalently linked to a 3' nucleotide of the first 5' cap.

In some embodiments, at least one instance of the second linker is covalently linked to a 3' nucleotide of the at least one instance of the second 5' caps.

In some embodiments, the 5' cap region of the modified mRNA comprises one or more modified nucleotides.

In some embodiments, one or more modified nucleotides of the 5' cap region comprise a modified nucleobase.

In some embodiments, the modified nucleobase is selected from the group consisting of xanthine, allyaminouracil, allyaminothymidine, hypoxanthine, digoxigeninated adenine, digoxigeninated cytosine, digoxigeninated guanine, digoxigeninated uracil, 6-chloropurineriboside, N6-methyladenine, methylpseudouracil, 2-thiocytosine, 2-thiouracil, 5-methyluracil, 4-thiothymidine, 4-thiouracil, 5,6-dihydro-5-methyluracil, 5,6-dihydrouracil, 5-[(3-Indolyl)propionamide-N-allyl]uracil, 5-aminoallylcytosine, 5-aminoallyluracil, 5-bromouracil, 5-bromocytosine, 5-carboxycytosine, 5-carboxymethylesteruracil, 5-carboxyuracil, 5-fluorouracil, 5-formylcytosine, 5-formyluracil, 5-hydroxycytosine, 5-hydroxymethylcytosine, 5-hydroxymethyluracil, 5-hydroxyuracil, 5-iodocytosine, 5-iodouracil, 5-methoxycytosine, 5-methoxyuracil, 5-methylcytosine, 5-methyluracil, 5-propargylaminocytosine, 5-propargylaminouracil, 5-propynylcytosine, 5-propynyluracil, 6-azacytosine, 6-azauracil, 6-chloropurine, 6-thioguanine, 7-deazaadenine, 7-deazaguanine, 7-deaza-7-propargylaminoadenine, 7-deaza-7-propargylaminoguanine, 8-azaadenine, 8-azidoadenine, 8-chloroadenine, 8-oxoadenine, 8-oxoguanine, araadenine, aracytosine, araguanine, arauracil, biotin-16-7-deaza-7-propargylaminoguanine, biotin-16-aminoallylcytosine, biotin-16-aminoallyluracil, cyanine 3-5-propargylaminocytosine, cyanine 3-6-propargylaminouracil, cyanine 3-aminoallylcytosine, cyanine 3-aminoallyluracil, cyanine 5-6-propargylaminocytosine, cyanine 5-6-propargylaminouracil, cyanine 5-aminoallylcytosine, cyanine 5-aminoallyluracil, cyanine 7-aminoallyluracil, dabcyl-5-3-aminoallyluracil, desthiobiotin-16-aminoallyl-uracil, desthiobiotin-6-aminoallylcytosine, isoguanine, N1-ethylpseudouracil, N1-methoxymethylpseudouracil, N1-methyladenine, N1-methylpseudouracil, N1-propylpseudouracil, N2-methylguanine, N4-biotin-OBEA-cytosine, N4-methylcytosine, N6-methyladenine, O6-methylguanine, pseudoisocytosine, pseudouracil, thienocytosine, thienoguanine, thienouracil, xanthosine, 3-deazaadenine, 2,6-diaminoadenine, 2,6-daminoguanine, 5-carboxamide-uracil, 5-ethynyluracil, N6-isopentenyladenine (i6A), 2-methyl-thio-N6-isopentenyladenine (ms2i6A), 2-methylthio-N6-methyladenine (ms2m6A), N6-(cis-hydroxyisopentenyl)adenine (io6A), 2-methylthio-N6-(cis-hydroxyisopentenyl)adenine (ms2io6A), N6-glycinylcarbamoyladenine (g6A), N6-threonylcarbamoyladenine (t6A), 2-methylthio-N6-threonyl carbamoyladenine (ms2t6A), N6-methyl-N6-threonylcarbamoyladenine (m6t6A), N6-hydroxynorvalylcarbamoyladenine (hn6A), 2-methylthio-N6-hydroxynorvalyl carbamoyladenine (ms2hn6A), N6,N6-dimethyladenine (m62A), and N6-acetyladenine (ac6A).

In some embodiments, one or more modified nucleotides of the 5' cap region comprise a modified sugar.

In some embodiments, the modified sugar comprised by one or more modified nucleotides of the 5' cap region is selected from the group consisting of 2'-thioribose, 2',3'-dideoxyribose, 2'-amino-2'-deoxyribose, 2' deoxyribose, 2'-azido-2'-deoxyribose, 2'-fluoro-2'-deoxyribose, 2'-O-methylribose, 2'-O-methyldeoxyribose, 3'-amino-2',3'-dideoxyribose, 3'-azido-2',3'-dideoxyribose, 3'-deoxyribose, 3'-O-(2-nitrobenzyl)-2'-deoxyribose, 3'-O-methylribose, 5'-aminoribose, 5'-thioribose, 5-nitro-1-indolyl-2'-deoxyribose, 5'-biotin-ribose, 2'-O,4'-C-methylene-linked, 2'-O,4'-C-amino-linked ribose, and 2'-O,4'-C-thio-linked ribose.

In some embodiments, one or more modified nucleotides of the 5' cap region comprise a modified phosphate.

In some embodiments, the modified phosphate comprised by one or more modified nucleotides of the 5' cap region is selected from the group consisting of phosphorothioate (PS), thiophosphate, 5'-O-methylphosphonate, 3'-O-methylphosphonate, 5'-hydroxyphosphonate, hydroxyphosphanate, phosphoroselenoate, selenophosphate, phosphoramidate, carbophosphonate, methylphosphonate, phenylphosphonate, ethylphosphonate, H-phosphonate, guanidinium ring, triazole ring, boranophosphate (BP), methylphosphonate, and guanidinopropyl phosphoramidate.

In some embodiments, the 5' cap region comprises between 1 and 3, between 3 and 5, between 5 and 10, between 10 and 15, between 15 and 30, between 30 and 50, between 50 and 100, or between 100 and 200 phosphorothioates.

In some embodiments, the 5' cap region comprises between 5 and 30 phosphorothioates.

In some embodiments, the 5' cap region comprises between 3 and 5, between 5 and 10, between 10 and 15, between 15 and 30, between 30 and 50, between 50 and 100, or between 100 and 200 deoxyribose sugars.

In some embodiments, the 5' cap region comprises between 5 and 30 deoxyribose sugars.

In some embodiments, the mRNA comprises a 3' terminal nucleotide wherein the 3' terminal nucleotide is dideoxyadenosine, dideoxycytidine, dideoxyguanosine, dideoxythymidine, dideoxyuridine, or inverted-deoxythymidine.

In some embodiments, the 5' cap region comprises between 25-500 nucleotides.

In some embodiments, the 5' cap region comprises between 50 and 100, between 100 and 150, between 150 and 200, between 200 and 300, between 300 and 400, or between 400 and 500 nucleotides.

In some embodiments, the 5' cap region comprises between 1 and 3, between 3 and 5, between 5 and 7, or between 7 and 10 5' caps.

In some embodiments, the 5' cap region comprises one or more 7-methylguanylate caps comprising a 5'-5' triphosphate linkage.

In some embodiments, the 5' cap region comprises one or more 2,2,7-trimethylguianosine caps comprising a 5'-5' triphosphate linkage.

In some embodiments, the 5' cap region comprises one or more modified 5' caps.

In some embodiments, one or more modified 5' caps are a locked nucleic acid (LNA)-modified cap, a 5' cap comprising a 5'-5' triphosphate linkage that is modified by 5'-phosphorothiolate, or a 5' cap comprising a 5'-5' tetraphosphate linkage.

In some embodiments, the 5' cap region comprises one or more 5' caps comprising one or more modified nucleotides.

In some embodiments, one or more modified nucleotides comprised by the 5' caps are a 2'-O-methylated nucleotide.

In some embodiments, the first 5' cap comprises between 10 and 50 ribonucleotides, between 1 and 10 modified uridine deoxyribonucleotides comprising an attachment point, and a 5' cap; and the second 5' cap comprises between 10 and 50 ribonucleotides and a 5' cap.

In some embodiments, the first 5' cap comprises a nucleotide sequence set forth as:
5'-/Cap/rGrGrGrArArAdU(c1)rArArGrArGrArGrArArArArGrArArGrArGdU(c1)rArArGrArA rGrArArAdU(c1)rA-3' (SEQ ID NO: 4); the second 5' cap comprises a nucleotide sequence set forth as: 5'-/Cap/rGrGrGrArGrArCrTrGrCrCrArCrCrA*rA*rA*rA*rA*rA*(d1)-3' (SEQ ID NO: 5);
and each instance of the second linker is independently 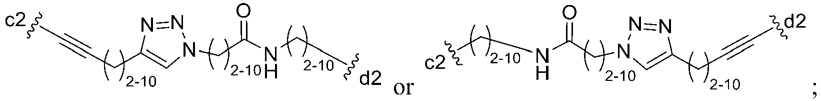
"rA" represents an adenosine ribonucleotide; "rT" represents a thymidine ribonucleotide;
"rC" represents a cytidine ribonucleotide; "rG" represents a guanosine ribonucleotide;
"dU(c1)" represents a modified uridine deoxyribonucleotide; "dU(c1)" represents a modified uridine deoxyribonucleotide; wherein "*" represents a phosphorothioate linkage; "Cap" represents a 5' cap; the c1 of each instance of dU(c1) represents an attachment point on the uridine of the dU(c1); the d1 of rA*(d1) represents an attachment point on the * of the rA*(dl); each instance of c1 is attached to an instance of c2; and each instance of d1 is attached to an instance of d2. In some embodiments, the modified uridine deoxynucleotide is 5-octadiynyl deoxyuridine.

In some embodiments, the first 5' cap comprises between 5 and 10 ribonucleotides and a 5' azide moiety; and the second 5' cap comprises between 10 and 50 ribonucleotides, a 3' azide moiety, and a 5' cap.

In some embodiments, the first 5' cap comprises a nucleotide sequence set forth as: 5'-/5AzideN/rArArA rArA-3'; and the second 5' cap comprise a nucleotide sequence set forth as: 5'-/Cap/rGrGrGrArGrArCrTrGrCrCrArCrCrA*rA*rA*rA*rA*rA* /3AzideN/-3' (SEQ ID NO: 6); wherein "rA" represents an adenosine ribonucleotide; "rT" represents a thymidine ribonucleotide; "rC" represents a cytidine ribonucleotide; "rG" represents a guanosine ribonucleotide; "dU(c1)" represents a modified uridine deoxyribonucleotide; "5AzideN" represents a 5' azide moiety; "3AzideN" represents a 3' azide moiety, "*" represents a phosphorothioate linkage, and "Cap" represents a 5' cap.

In some aspects, the present disclosure provides a method of producing any one of the modified mRNAs described herein, the method comprising ligating a first RNA comprising an open reading frame (ORF) encoding a protein to a tailing nucleic acid comprising two or more poly-A tails and/or a capping nucleic acid comprising two or more 5' caps, in the presence of an RNA ligase, whereby the RNA ligase forms a covalent bond between the 3' nucleotide of the first RNA and the 5' nucleotide of the tailing nucleic acid and/or a covalent bond between the 5' nucleotide of the first RNA and the 3' nucleotide of the capping nucleic acid to produce the modified mRNA.

In some embodiments, the modified mRNA comprises a 5' untranslated region (5' UTR) and a 3' untranslated region (3' UTR), wherein the ORF is between the 5' UTR and the 3' UTR, wherein the 3' UTR is between the ORF and the ligated tailing nucleic acid, and wherein the 5' UTR is between the ORF and the ligated capping nucleic acid.

In some embodiments, the tailing nucleic acid comprises a first poly-A tail and one or more second poly-A tails, wherein the first poly-A tail is covalently linked to the 3' UTR.

In some aspects, the present disclosure provides a modified mRNA produced by a method of producing a modified mRNA provided herein.

In some aspects, the present disclosure provides a delivery agent comprising any one of the modified mRNAs provided herein, wherein the delivery agent comprises a lipid, a peptide, a protein, an antibody, a carbohydrate, a nanoparticle, or a microparticle.

In some embodiments, a nanoparticle or microparticle provided herein is a lipid nanoparticle or a lipid microparticle, a polymer nanoparticle or a polymer microparticle, a protein nanoparticle or a protein microparticle, or a solid nanoparticle or a solid microparticle.

In some aspects, the present disclosure provides a cell comprising any one of the modified mRNAs provided herein.

In some embodiments, the cell is a mammalian cell.

In some aspects, the present disclosure provides a composition comprising any one of the modified mRNAs provided herein, a delivery agent provided herein, or a cell provided herein.

In some embodiments, the composition further comprises an additional agent. In some embodiments, the additional agent is an agent which has a therapeutic effect when administered to a subject. In some embodiments, the additional agent is a nucleotide, a nucleic acid, an amino acid, a peptide, a protein, a small molecule, an aptamer, a lipid, or a carbohydrate. In some embodiments, the additional agent is a shRNA, a siRNA, or an ASO. In some embodiments, the additional agent is an antigen or adjuvant.

In some embodiments, the composition is a pharmaceutical composition, wherein the pharmaceutical composition further comprises a pharmaceutically acceptable excipient.

In some aspects, the present disclosure provides a method comprising introducing any one of the modified mRNAs provided herein, a delivery agent provided herein, or a composition provided herein, into a cell.

In some aspects, the present disclosure provides a method comprising introducing any one of the modified mRNAs provided herein, a delivery agent provided herein, a cell provided herein, or a composition provided herein into a subject.

In some aspects, the present disclosure provides a method of preventing a disease in a subject in need thereof comprising introducing an effective amount any one of the modified mRNAs provided herein, a delivery agent provided herein, a cell provided herein, or a composition provided herein into the subject, wherein the open reading frame of the modified mRNA encodes a protein.

In some aspects, the present disclosure provides a method of treating a disease in a subject in need thereof comprising introducing an effective amount any one of the modified mRNAs provided herein, a delivery agent provided herein, a cell provided herein, or a composition provided herein into the subject, wherein the open reading frame of the modified mRNA encodes a protein.

In some embodiments, the protein is a protein antigen or a fragment thereof from a cell or virus that causes the disease.

In some embodiments, the protein is a protein that is expressed in the subject at a level that is lower than that of a reference value.

In some aspects, the present disclosure provides a method of replacing an enzyme in a subject comprising introducing any one of the modified mRNAs provided herein, a delivery agent provided herein, a cell provided herein, or a composition provided herein into the subject, wherein the open reading frame of the modified mRNA encodes an enzyme.

In some embodiments, the subject is a human.

In some aspects, the present disclosure provides any one of the modified mRNAs provided herein, a delivery agent provided herein, a cell provided herein, or a composition provided herein for use in treating a disease in a subject in need thereof.

In some aspects, the present disclosure provides a kit comprising the first RNA, and the tailing nucleic acid and/or capping nucleic acid of a method of producing any one of the modified mRNAs provided herein.

In some embodiments, the kit further comprises an RNA ligase.

In some aspects, the present disclosure provides a kit comprising a pharmaceutical composition provided herein, a device for administering the pharmaceutical composition to a subject, and instructions for administering the pharmaceutical composition to a subject.

### BRIEF DESCRIPTION OF THE DRAWINGS

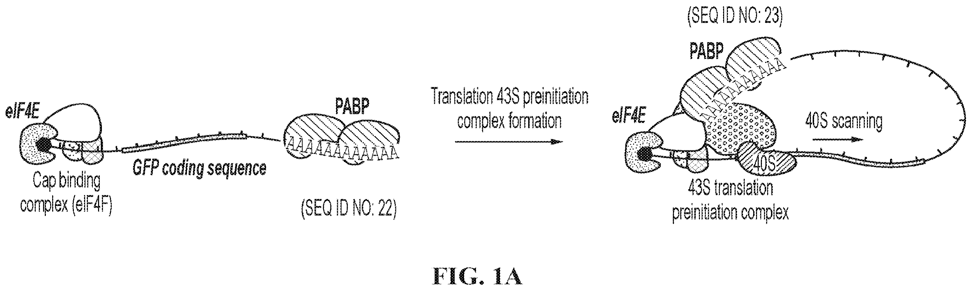

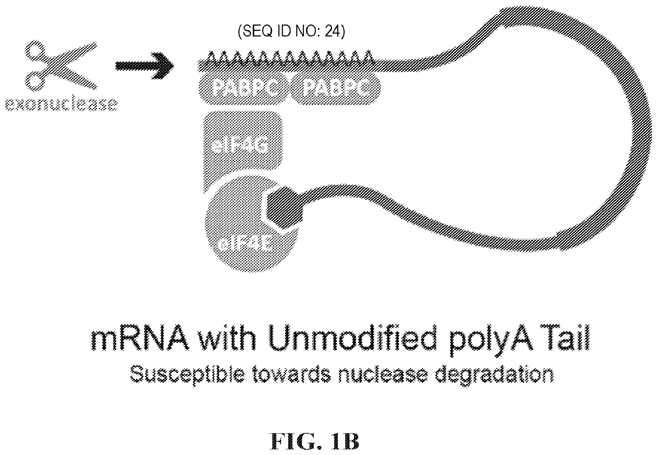

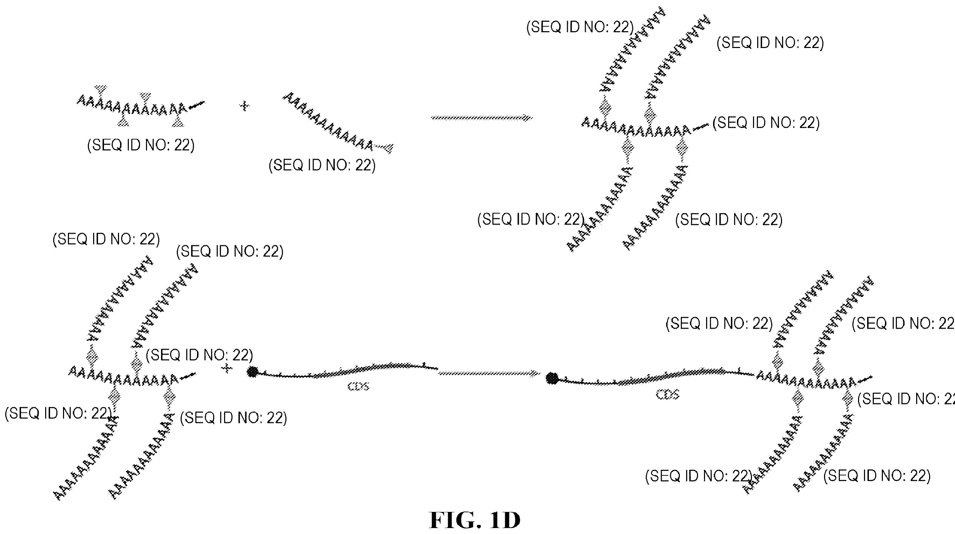

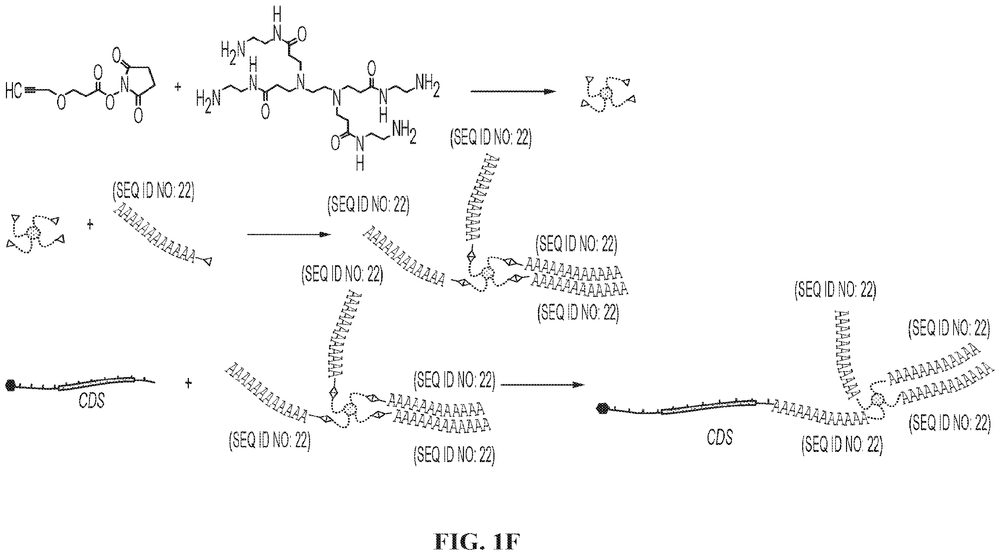

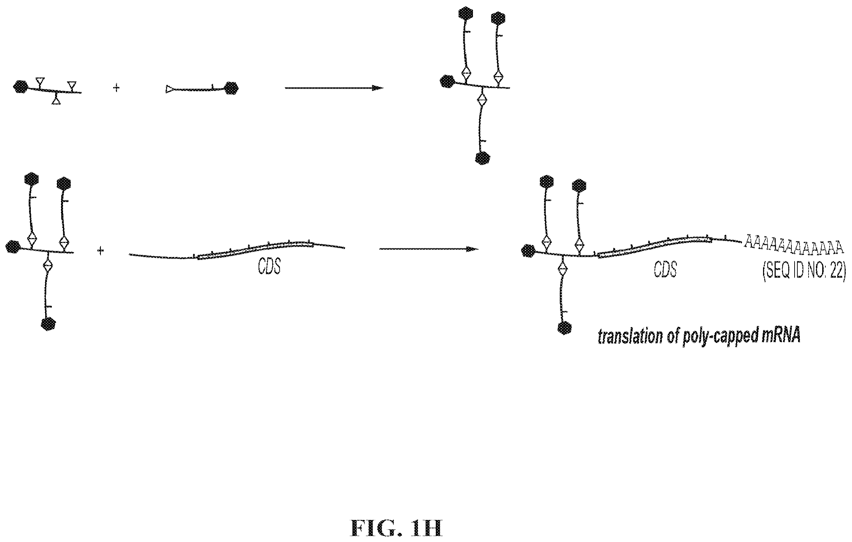

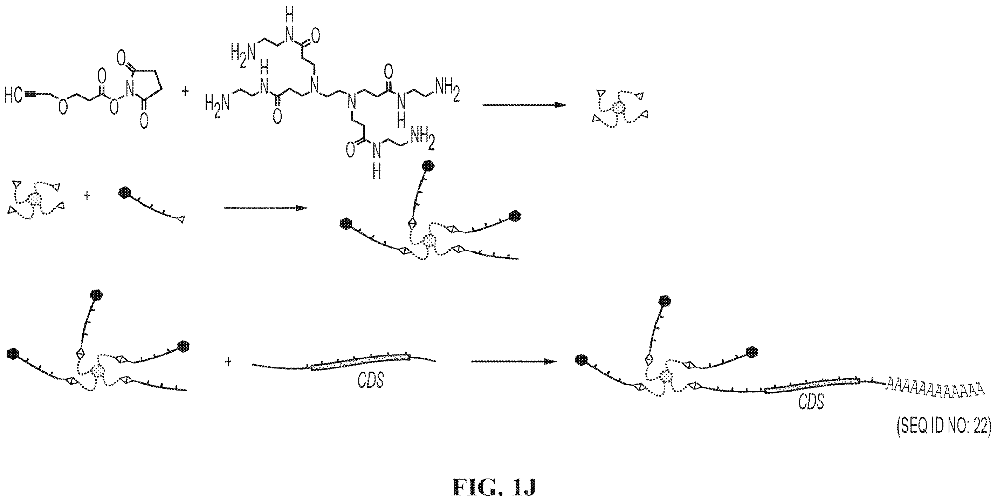

**FIGs.** 1A-J show schematics depicting alternate design pathways and routes of synthesizing poly-tailed and poly-capped mRNA. FIG. 1A shows the canonical pathway of eukaryotic translation initiation. **FIG. 1B** shows a pseudo closed loop model of actively translating mRNA, wherein the poly(A) tail is being degraded through exonuclease activity. **FIG. 1C** shows a first orthogonal design pathway for synthesizing poly-tailed mRNAs, where crosslinking chemistry (e.g., click chemistry) is used to generate poly-tailed mRNA. The poly-A branches are linked to the mRNA using site-specific "crosslinking" groups, which are incorporated into the 3' end of the mRNA transcript as modified nucleotides (e.g., added by a polymerase or site-specific modification by enzymatic treatment or through ligation of molecules/oligos containing crosslinking groups). **FIG. 1D** shows a schematic depicting the route of synthesizing a poly-tailed mRNA construct starting from a poly-A oligo modified with several internal 5-Octadiynyl dU sites to which further poly-A oligos are attached via a click chemistry reaction (at triangles) to produce a brushed poly-A tail. The brushed poly-A tail is then ligated to the 3' end of an RNA comprising a coding sequence (CDS). **FIG. 1E** shows a second orthogonal design pathway for synthesizing poly-tailed mRNAs, where chemical or enzymatic ligation is used to attach a poly-A tail-functionalized dendrimer onto the 3' end of a linear mRNA. **FIG. 1F** shows a schematic depicting the route of synthesizing a poly-tailed mRNA construct starting from a PAMAM dendrimer that is functionalized with alkyne groups, to which poly-A oligos are attached via a click chemistry reaction (at triangles). One of the poly-A oligos of the dendrimer is then ligated to the 3' end of an RNA comprising a coding sequence (CDS). **FIG. 1G** shows a first orthogonal design pathway for synthesizing poly-capped mRNAs, where crosslinking chemistry (e.g., click chemistry) is used to generate poly-capped mRNA. The poly-capped branches are linked to the mRNA using site-specific "crosslinking" groups, which are incorporated into the 5' end of the mRNA transcript as modified nucleotides (e.g., through ligation of molecules/oligos containing crosslinking groups). **FIG. 1H** shows a schematic depicting the route of synthesizing a poly-capped mRNA construct starting from a 5' capped oligo modified with several internal 5-Octadiynyl dU sites to which further 5' capped oligos are attached via a click chemistry reaction (at triangles) to produce a brushed 5' capping sequence. The brushed 5' capping sequence is then ligated to the 5' end of an RNA comprising a coding sequence (CDS). **FIG. 1I** shows a second orthogonal design pathway for synthesizing poly-capped mRNAs, where chemical or enzymatic ligation is used to attach a poly-capped-functionalized dendrimer onto the 5' end of a linear mRNA. **FIG. 1J** shows a schematic depicting the route of synthesizing a poly-capped mRNA construct starting from a PAMAM dendrimer that is functionalized with alkyne groups, to which 5' capping oligos are attached via a click chemistry reaction (at triangles). One of the 5' capping oligos of the dendrimer is then ligated to the 5' end of an RNA comprising a coding sequence (CDS).

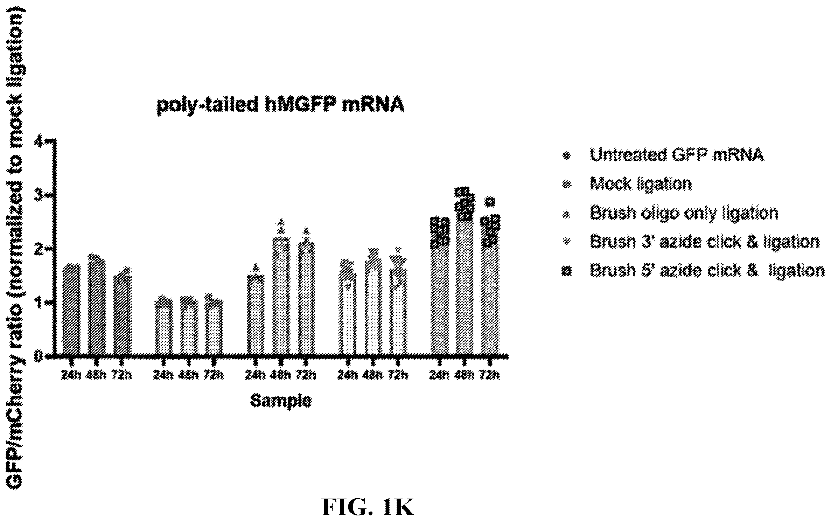

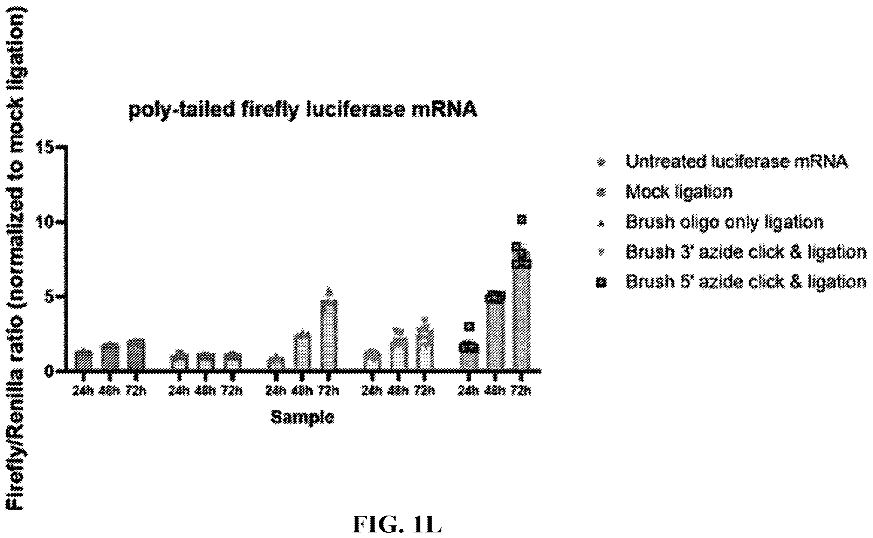

**FIGs. 1K** **and** **1L** show protein expression from different poly-tailed mRNA constructs. **FIG. 1K** shows GFP production over time from different poly-tailed mRNA constructs. Untreated GFP mRNA: GFP mRNA obtained from *in vitro* transcription. Mock ligation: products of the ligation reaction between GFP mRNA and no oligo. Brushed oligo only ligation: products of the ligation reaction between GFP mRNA and the brushed oligo precursor shown in Table 1. Brush 3' azide click & ligation: products of the ligation reaction between GFP mRNA and a brushed oligo synthesized from the click reaction between the brushed oligo precursor and the 3' azide poly(A) oligo of Table 1. Brush 5' azide click & ligation: products of the ligation reaction between GFP mRNA and a brushed oligo synthesized from the click reaction between the brushed oligo precursor and the 5' azide poly(A) oligo of Table 1. mCherry mRNA was always unligated and thus used as an internal control and co-transfected with GFP mRNA. The GFP/mCherry ratio was calculated from the intensities of GFP and mCherry signals, respectively. **FIG. 1L** shows firefly luciferase production over time from different poly-tailed mRNA constructs. Untreated luciferase mRNA: firefly luciferase mRNA obtained from *in vitro* transcription. Mock ligation: products of the ligation reaction between firefly luciferase mRNA and no oligo. Brushed oligo only ligation: products of the ligation reaction between firefly luciferase mRNA and the brushed oligo precursor shown in Table 1. Brush 3' azide click & ligation: products of the ligation reaction between firefly luciferase mRNA and a brushed oligo synthesized from the click reaction between the brushed oligo precursor and the 3' azide poly(A) oligo of Table 1. Brush 5' azide click & ligation: products of the ligation reaction between firefly luciferase mRNA and a brushed oligo synthesized from the click reaction between the brushed oligo precursor and the 5' azide poly(A) oligo of Table 1. Renilla luciferase mRNA was always unligated and thus used as an internal control and co-transfected with firefly luciferase mRNA. The Firefly/Renilla ratio was calculated from the intensities of bioluminescence signals induced by firefly luciferase and renilla luciferase respectively.

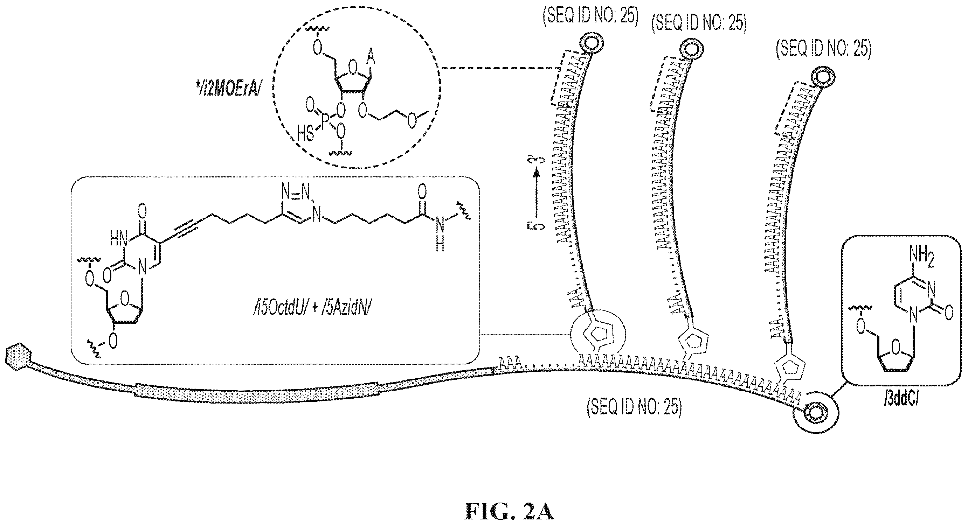

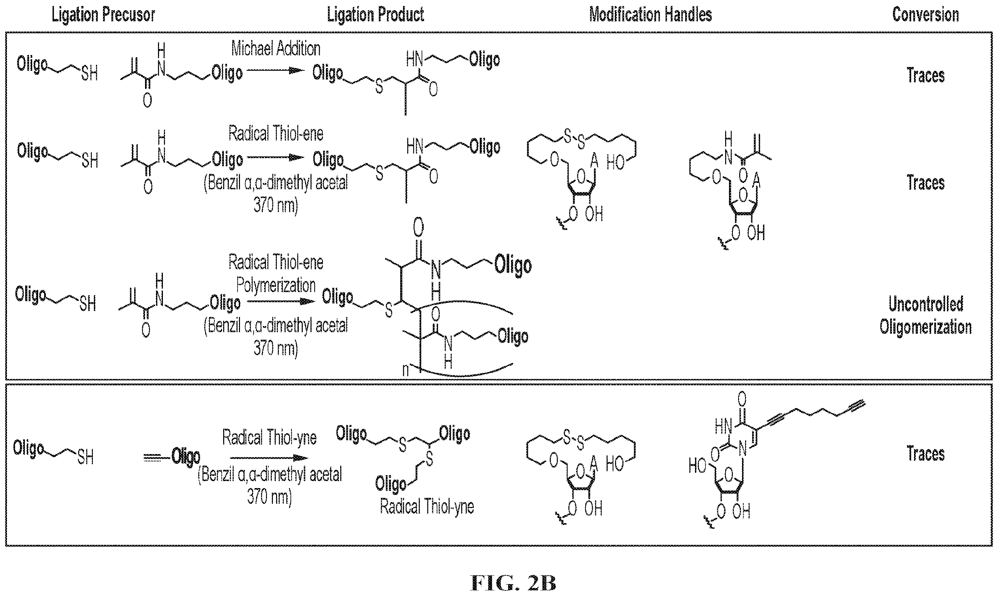

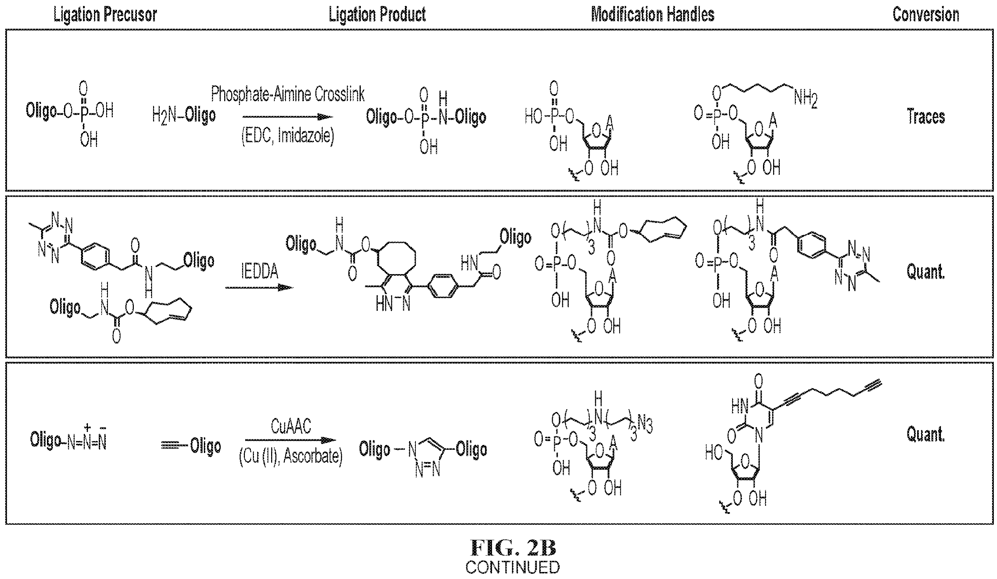

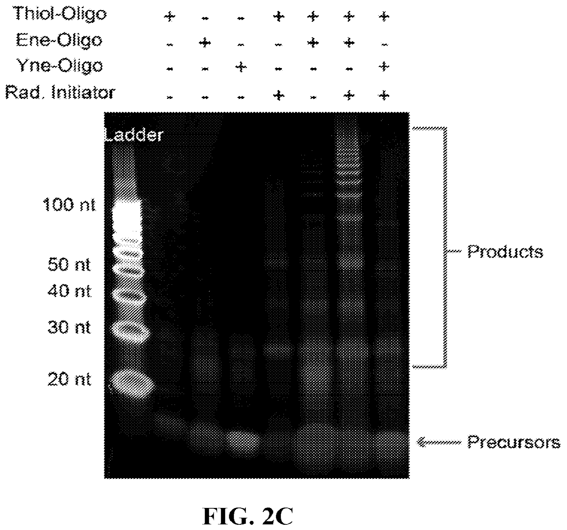

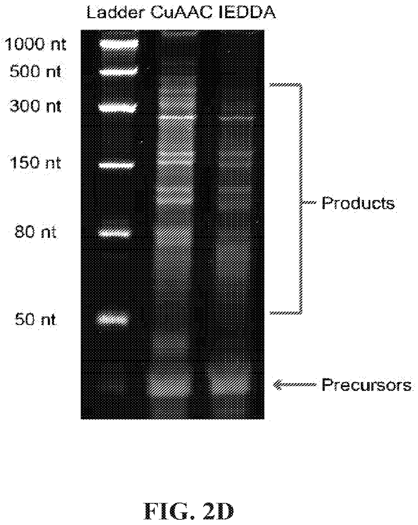

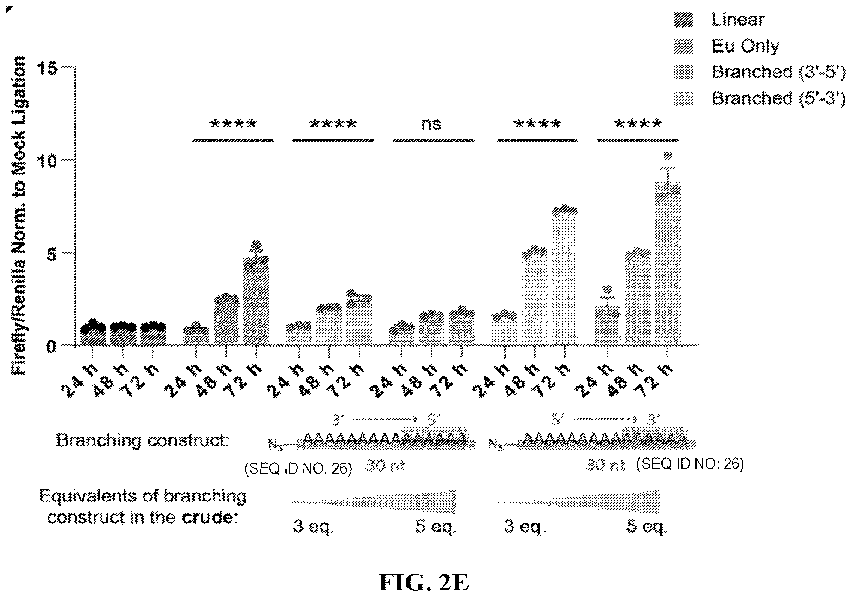

**FIGs. 2A-2D** show the conceptualization of chimeric mRNA bearing multiple poly(A) tails. **FIG. 2A** shows the pseudo closed loop model of actively translating mRNA. **FIG. 2B** shows a summary of oligonucleotide chemical conjugation methods. Screening was performed using 15-nt dA model substrates at micromolar concentrations. Modification handles were incorporated through solid phase synthesis, followed by amine-NHS labeling and HPLC purification if necessary. **FIG. 2C** shows a gel electrophoresis of crude thiol-ene/yne oligonucleotide conjugation of 15-nt model substrates containing only one conjugation handle. **FIG. 2D** shows gel electrophoresis of crude CuAAC and IEDDA 30-nt oligonucleotides bearing three EU/TCO handles reacting with 30-nt N₃/Tz modified oligo. **FIG. 2E** shows the preliminary dual luciferase assay using branched mRNA prepared from unpurified CuAAC mixture enriched by different equivalents of branched oligos. Branching oligos contained natural (5' to 3') or reversed (3' to 5') directionality and were modified on the last six bases. Time-course assay was performed as outlined in Fig. 1. *n* = 3 independent transfections in each biological condition. Mean ± sem. *P* values were calculated by ordinary two-way ANOVA (alpha = 0.05) (Dunnett's multiple comparison test, comparison of means across time points), with multiple comparisons to the linear construct. **** *P <* 0.0001, n.s. *P* > 0.1234.

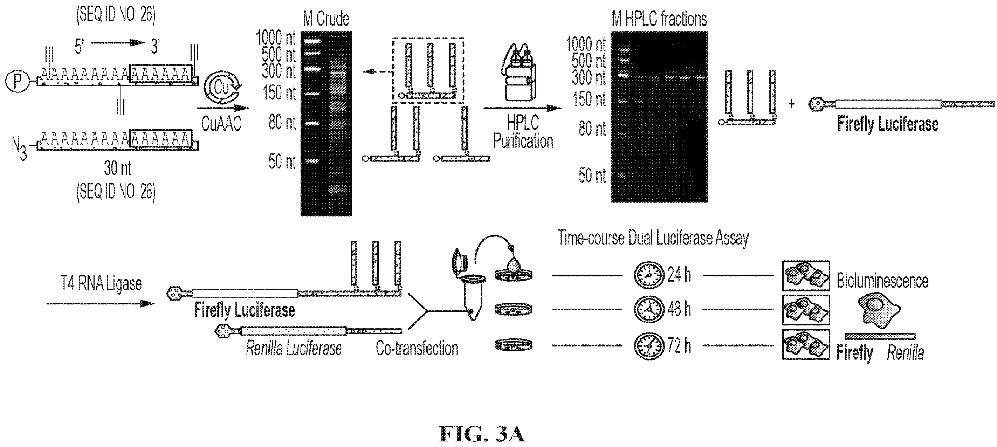

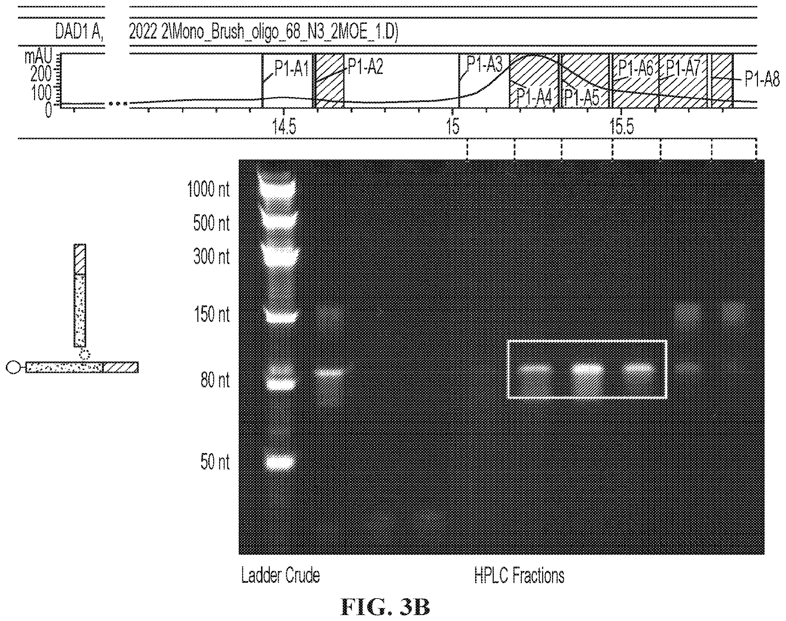

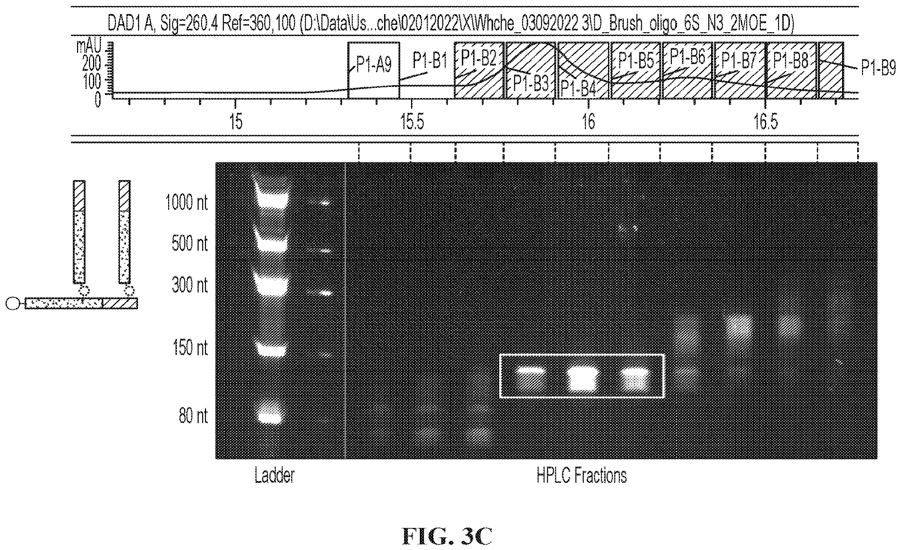

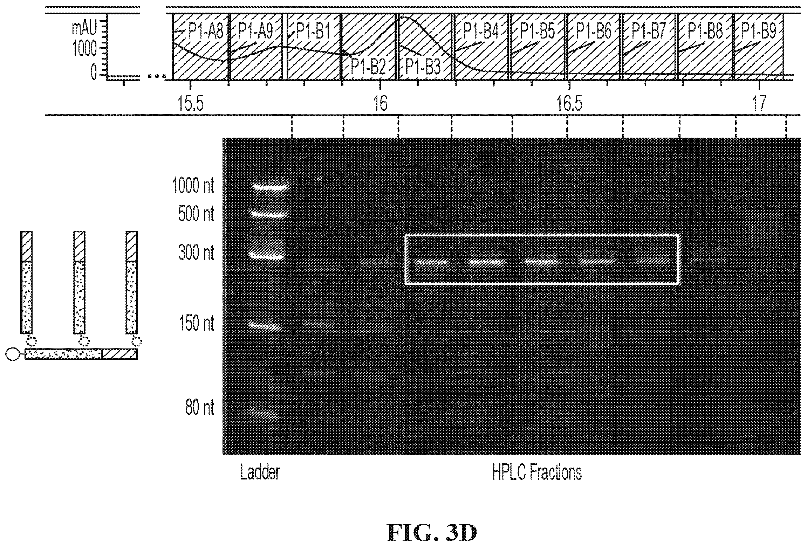

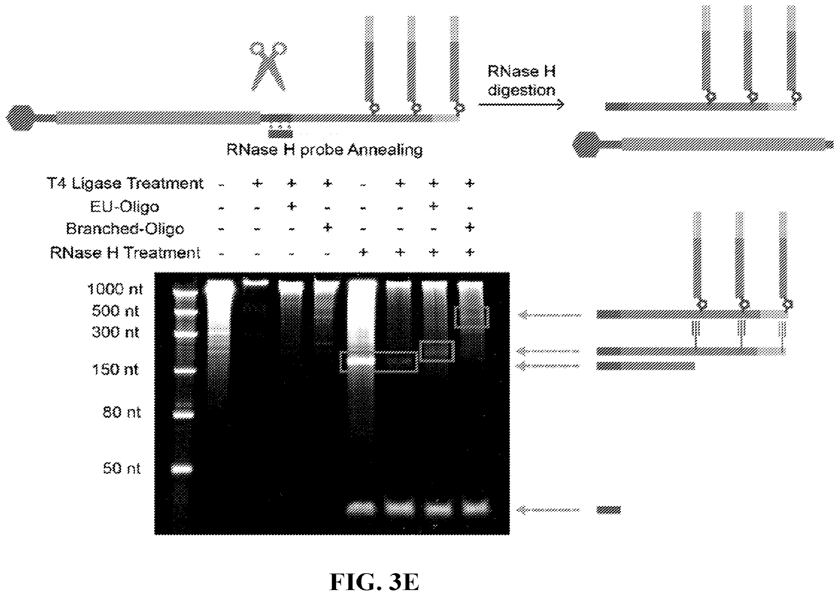

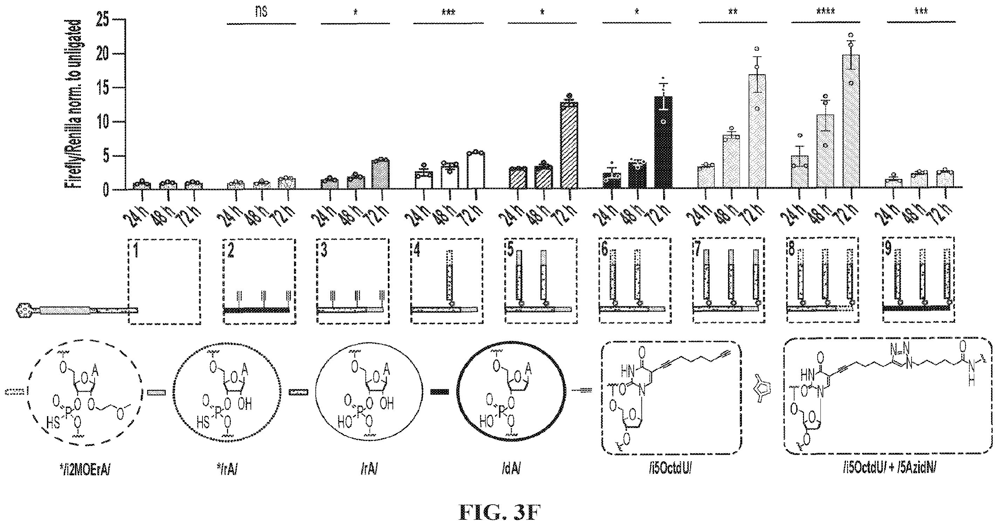

**FIGs. 3A-F** show the conceptualization of chimeric mRNA molecules bearing multiple poly-A tails. **FIG. 3A** shows the synthesis and screening pipeline of branched mRNA. EU and azide containing oligos were chemically conjugated, HPLC purified, and enzymatically ligated to 3'-end of mRNA to produce translatable branched mRNA coding Firefly luciferase reporter. Constructs were screened in a dual luciferase assay with Renilla luciferase as transfection control over a 72-hour time course. M, markers. **FIGs. 3B-3D** show HPLC purification chromatograms and gel electrophoresis characterizations of oligos containing 1/2/3 branching poly(A)'s. Fractions containing the desired products (boxed) were pooled and isolated. **FIG. 3E** shows representative RNase H characterization chimeric mRNA-branched poly(A) conjugates. RNase H probe was designed 200 nt upstream from the 3'-end of mRNA and conjugation products (boxed) were characterized by the corresponding band shifts post RNase H digestion on gel electrophoresis. **FIG. 3F** shows barplots of time-course dual luciferase assay screening chemical modifications on multimerized poly(A) tails. Protein expression was measured by Firefly luciferase luminescence normalized to Renilla luciferase luminescence and the linear mRNA control at 24, 48, 72 hrs post transfection. Mean ± sem. *P* values were calculated by ordinary two-way ANOVA (alpha = 0.05) (Dunnett's multiple comparison test, comparison of means across time points), with multiple comparisons to the linear construct. * *P* < 0.0332, ** *P* < 0.0021, *** *P* < 0.0002, **** *P* < 0.0001, n.s. *P* > 0.1234.

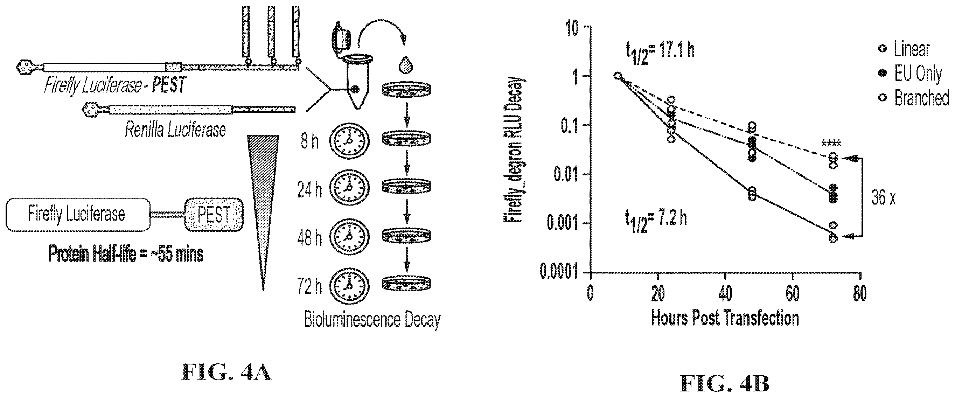

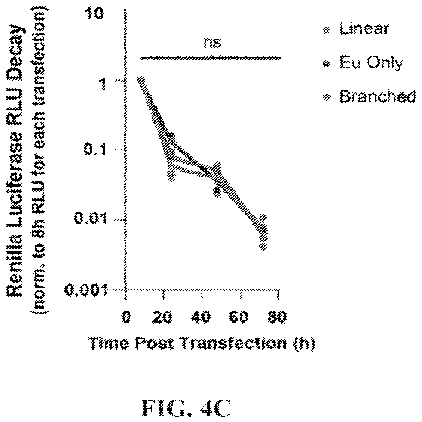

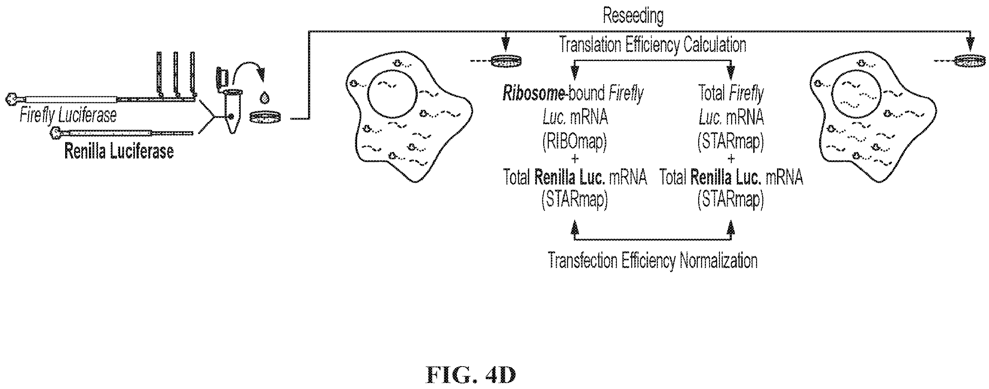

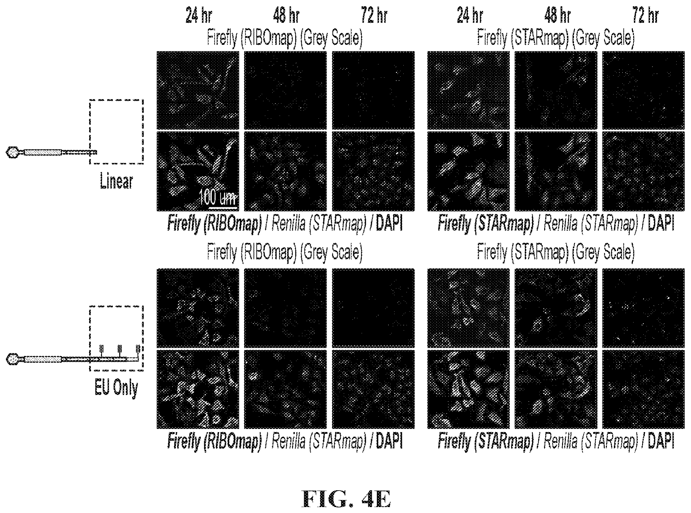

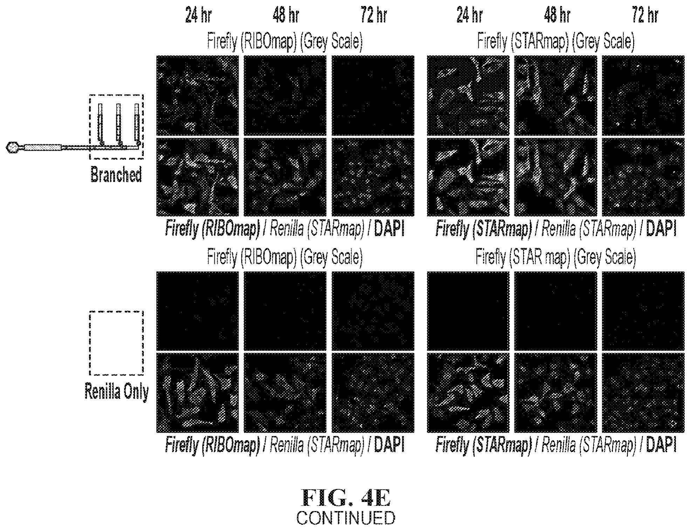

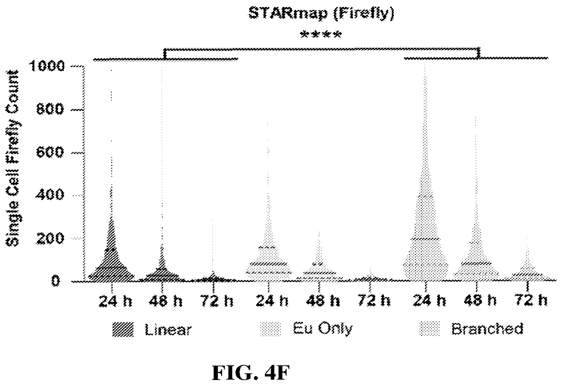

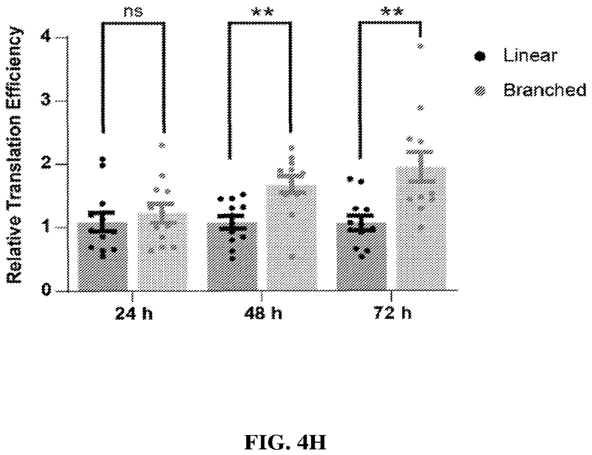

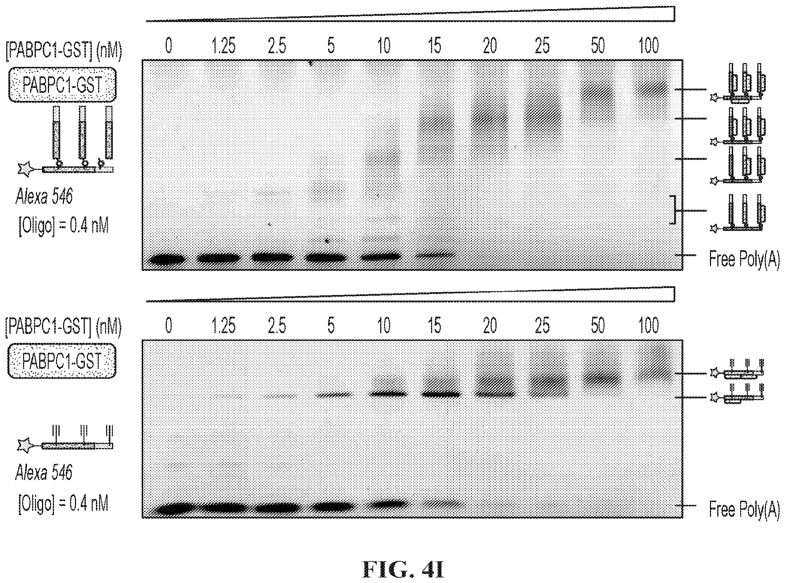

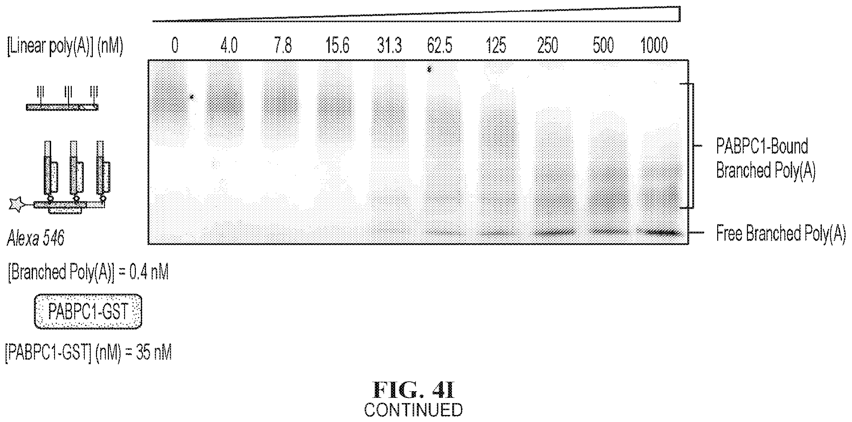

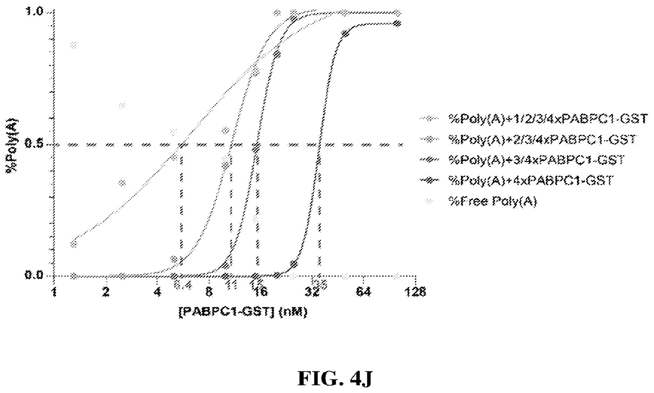

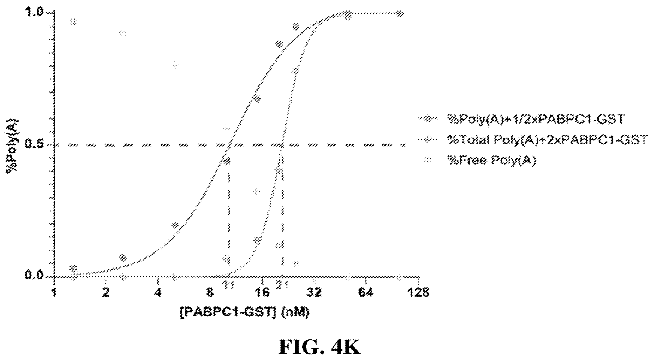

**FIGs. 4A-4K** show the mechanistic characterization of mRNA stabilization by multimerized poly-A tails. **FIGs. 4A-4B** show kinetic characterization of Firefly-degron reporter mRNAs. Firefly RLU of each construct at corresponding time points were normalized against Renilla luciferase control by Firefly RLU/Renilla RLU*average(Renilla RLU) and against 8 hrs normalized Firefly RLU for each construct. Decay halflife was calculated by fitting to an exponential curve. *P* values were calculated by ordinary two-way ANOVA (alpha = 0.05) (Dunnett's multiple comparison test, comparison of means across time points), with multiple comparisons to the linear construct. **** *P* < 0.0001. **FIG. 4C** shows decay kinetics of internal control Renilla luciferase RLU outlined in Fig 2C. *P* values were calculated by ordinary two-way ANOVA (alpha = 0.05) (Dunnett's multiple comparison test, comparison of means across time points), with multiple comparisons to the linear construct. n.s. *P* > 0.1234. **FIG. 4D** shows a schematic illustration of synergistic *in situ* sequencing characterization of targeted mRNA transcripts using STARmap + RIBOmap. Cells were reseeded into two wells per condition per time point 6 hrs after cotransfection of Firefly and Renilla luciferase mRNAs. RIBOmap and STARmap characterization of engineered Firefly luciferase transcript in each duplicate measured ribosome-bound mRNA or total mRNA, respectively. Unmodified Renilla luciferase mRNA was sequenced by STARmap in all replicates to account for transfection efficiency. Representative STARmap/RIBOmap images were acquired under the same confocal imaging settings. For each condition, at least three independent transfections were conducted, of which four FOVs were imaged from each. DAPI (blue), nuclei; Firefly luciferase amplicons (magenta); Renilla luciferase amplicons (yellow). Colocalized Firefly/Renilla amplicons in STARmap (white dots) were lipid transfection vesicles and were excluded from downstream quantification. **FIG. 4E** shows representative STARmap/RIBOmap images were acquired under the same confocal imaging settings. STARmap/RIBOmap characterization of modified Firefly luciferase mRNA and STARmap characterization of internal control Renilla luciferase mRNA were performed as outlined in Fig 2. DAPI (blue), nuclei; Firefly luciferase amplicons (magenta); Renilla luciferase amplicons (yellow). Colocalized Firefly/Renilla amplicons in STARmap (white dots) were lipid transfection vesicles and were excluded from downstream quantification. **FIGs. 4F-4G** show violin plots of single cell quantification of firefly luciferase STARmap/RIBOmap amplicons. *P* values were calculated by ordinary two-way ANOVA (alpha = 0.05). **** *P* < 0.0001. **FIG. 4H** shows comparison of translation efficiency (TE) of branched and linear mRNA across different time points. For each single cell, total Firefly mRNA abundance was measured by log10(Firefly STARmap/Renilla STARmap); ribosome-bound Firefly mRNA abundance was measured by log10(Firefly RIBOmap/Renilla STARmap). TE was calculated by ratio of ribosome-bound Firefly/total Firefly and median values of each FOV were normalized against linear Firefly mRNA. Mean ± sem. *P* values were calculated by unpaired t test with Welch's correction. ** *P* < 0.004; n.s. *P* > 0.1234. **FIG. 4I** shows the gel shift assays of three branched poly-A oligos with varying concentrations of PABPC1-GST, where the stem oligos were labeled with Alexa Fluor 546 at the 5' end. Both stem and branching poly-A's were 30 nt and modified with PS-2MOE at last six bases and ddC at the 3'end (top). FIG. 4I also shows the gel shift assay of the same modified poly-A oligo without branching (middle). Finally, FIG. 4I shows the competitive binding assay of Alexa 546 labeled branched poly(A) oligo with unlabeled linear poly(A) oligo against PABCP1-GST (bottom). **FIGs. 4J-4K** show the apparent K_{d} calculation of branched/linear poly(A) oligo. The percentage of poly(A) oligos bonded to x mers of PABP were calculated as the total percentages of oligos bonded to >x mers of PABP and the apparent K_{d} was calculated by plotting percentage of PABP-bonded poly (A) oligos against PABP concentration using GraphPad Prism (Specific binding with Hill slope).

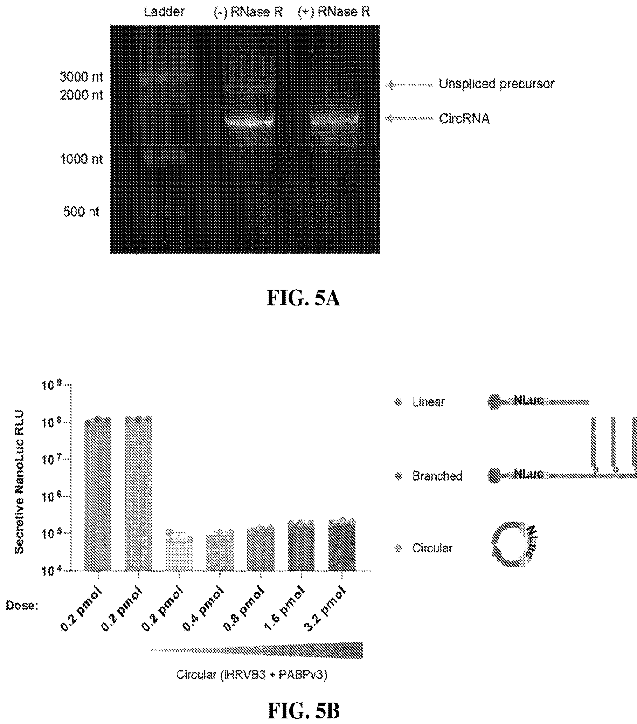

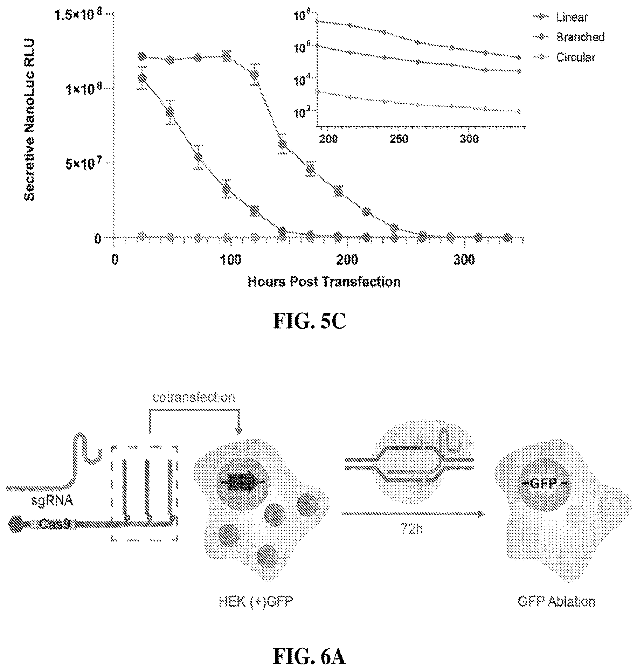

**FIGs. 5A-5C** show the comparison of branched versus circular mRNA. **FIG. 5A** shows a gel electrophoresis of circRNA generated through IVT, backsplicing, and enriched by RNase R treatment. **FIG. 5B** shows the comparison of linear/branched/circular mRNA with optimized UTRs at different dosage of circRNA using secretive Nano luciferase 24 hours after transfection in HeLa cells. Both branched and linear mRNAs were cotranscriptionally capped and tailed, and contained human alpha globin UTRs. Branched mRNAs were ligated with optimized branched construct with 6*PS-2MOE modifications. CircRNAs were designed to encode HRV IRES (with proximal loop Apt-eIF4G insertion) and 3'-PABP binding motif. **FIG. 5C** shows the time course comparison of linear vs branched vs circular mRNAs encoding secretive NanoLuc. Both branched and linear mRNAs were cotranscriptionally capped and tailed, and contained human alpha globin UTRs. Branched mRNAs were ligated with optimized branched construct with 6*PS-2MOE modifications. CircRNAs were designed to encode HRV IRES (with proximal loop Apt-eIF4G insertion) and 3'-PABP binding motif. Cells were transfected with equal molar (0.2 pmol) of each construct (three independent lipofections for each condition). Cells were cultured in phenol red-free DMEM media. On each day, media was completely renewed and 50 uL of old media was used for luciferase assay. Mean ± sem. *n* = 3 independent transfections for each biological condition.

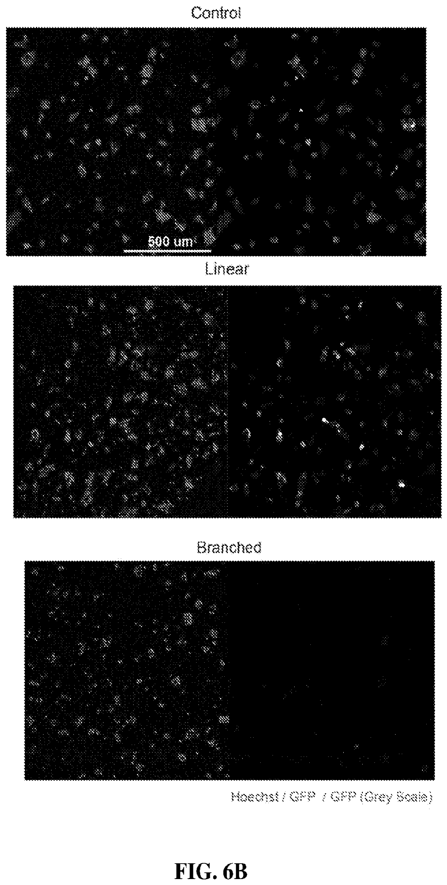

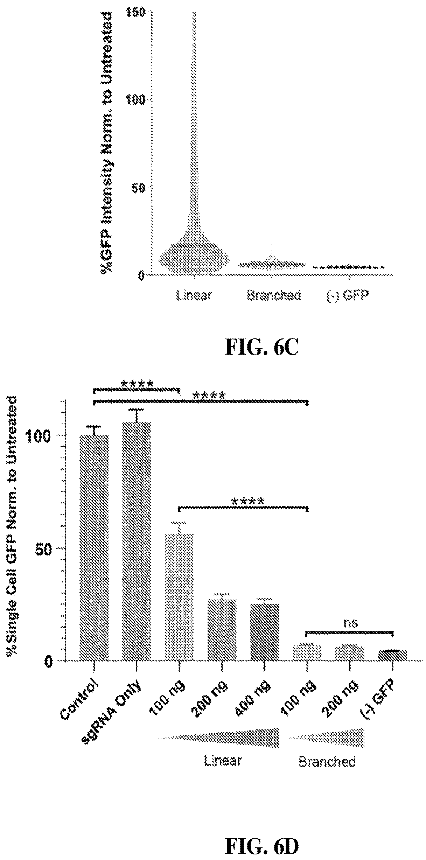

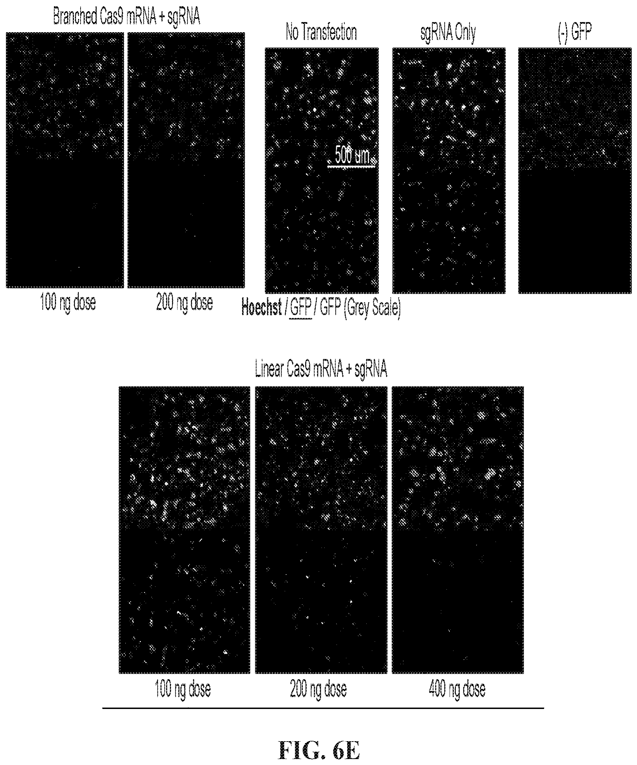

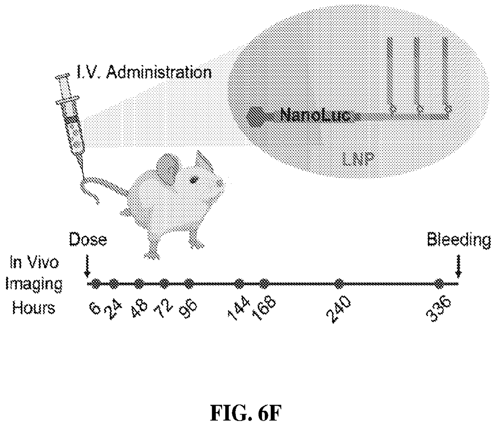

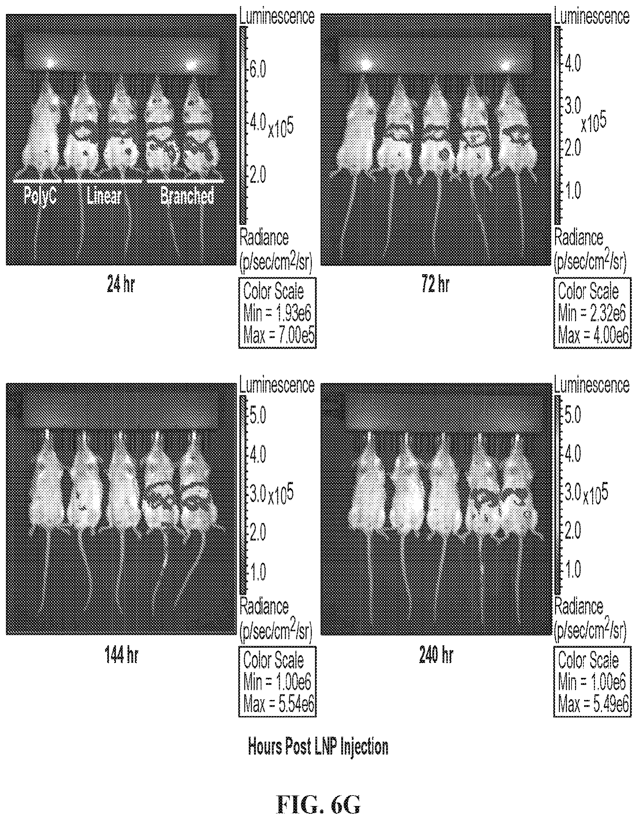

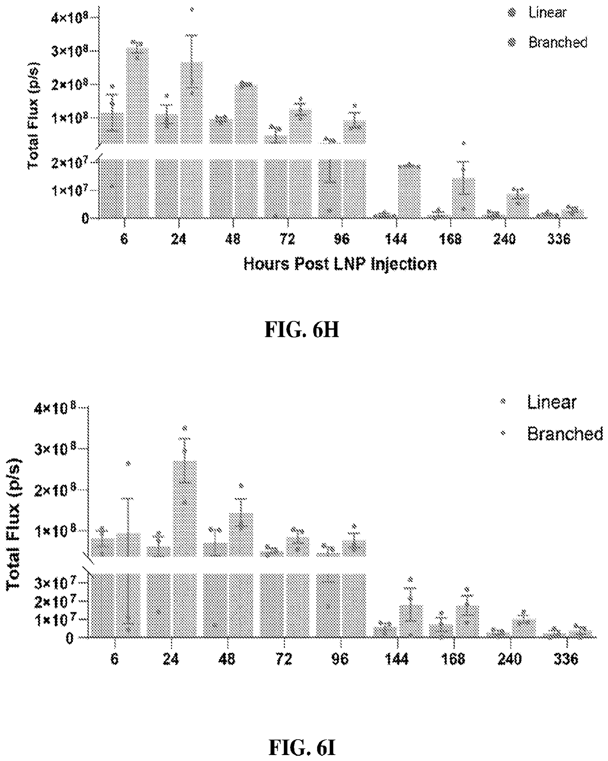

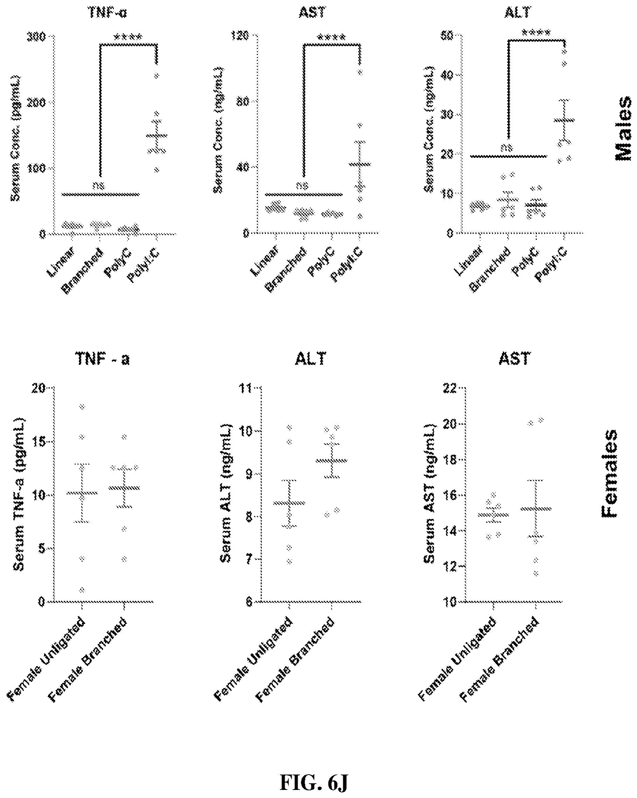

**FIGs. 6A-6J** show GFP ablation using branched Cas9 mRNA. **FIG. 6A** shows an illustration of GFP ablation experiments. Branched/linear Cas9 mRNAs were co-transfected with sgRNA targeting the GFP CDS. **FIG. 6B** shows representative images acquired under the same confocal imaging setting. Hoechst (blue), nuclei; GFP (green). **FIG. 6C** shows violin plots of %GFP intensity normalized to control of HEK293-uGFP cells treated with 100 ng branched/linear Cas9 mRNA after 72 hrs.FIG. **6D** shows barplots of single cell GFP intensities 72 hrs post transfection. Average GFP intensity in individual cells were measured and normalized against average GFP intensity of untreated cells. Mean ± sem. *P* values were calculated by ordinary one-way ANOVA (alpha = 0.05), (Tukey's multiple comparison test), with multiple comparisons between each group. **** *P* < 0.0001. n.s. *P* > 0.1234. **FIG. 6E** shows Representative images acquired under the same confocal imaging setting 72 hrs after transfection. For each condition, the same amount of sgRNA was transfected while the dose of Cas9 mRNA was varied. Hoechst (blue), nuclei; GFP (green). **FIG. 6F** shows schematics of *in vivo* expression of branched/linear NanoLuc mRNAs. mRNAs were formulated in LNPs and equal molars of branched/linear mRNA-LNP complex were administered through retro orbital injection. Mice were imaged at indicated time points and serum was collected 15 days post injection. **FIG. 6G** shows *in vivo* luminescence images of mice treated with polyC/linear/branched mRNA-LNPs at 24/72/144/240 hrs post LNP administration. **FIGs. 6H-6I** show *in vivo* luminescence signals of linear/branched NanoLuc mRNAs. Luminescence was measured by integration of total flux for each mouse normalized by subtraction of background (poly-C treated mice). Mean ± sem. *n* = 3 male mice for each condition. **FIG. 6J** shows serum level of immunogenicity and liver toxicity biomarkers. Serum concentrations of TNF-α, AST, and ALT were quantified by ELISA. Mean ± sem. *n =* 3 mice with each biological condition measured as duplicates. *P* values were calculated by ordinary one-way ANOVA (alpha = 0.05) (Tukey's multiple comparison test), with multiple comparisons between each group. ** *P* < 0.004; **** *P* < 0.0001; n.s. *P* > 0.1234.

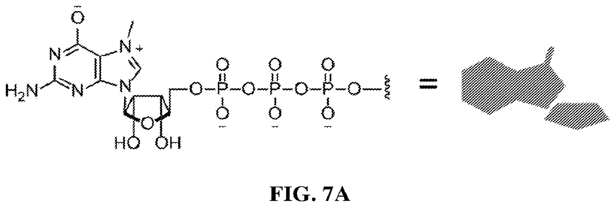

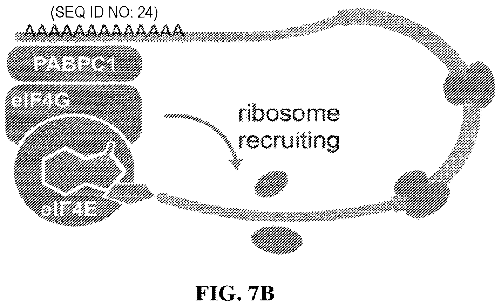

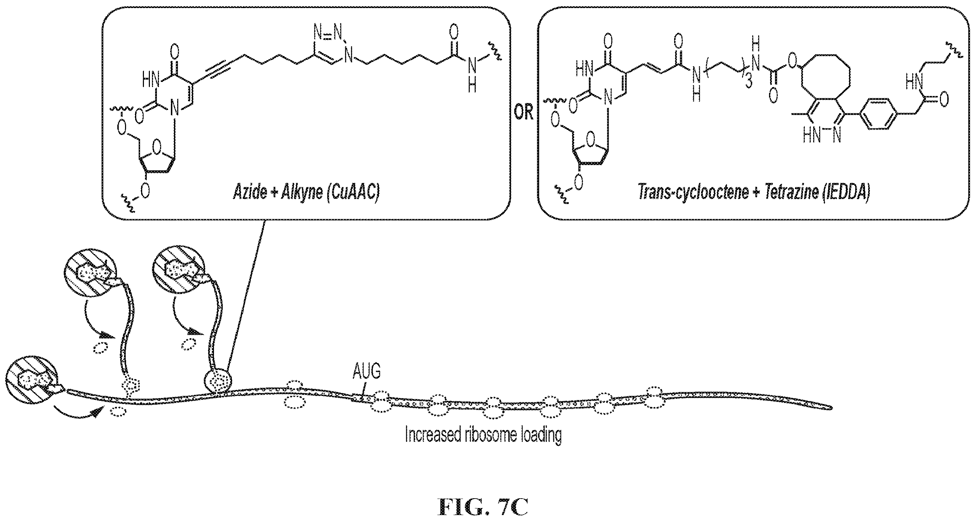

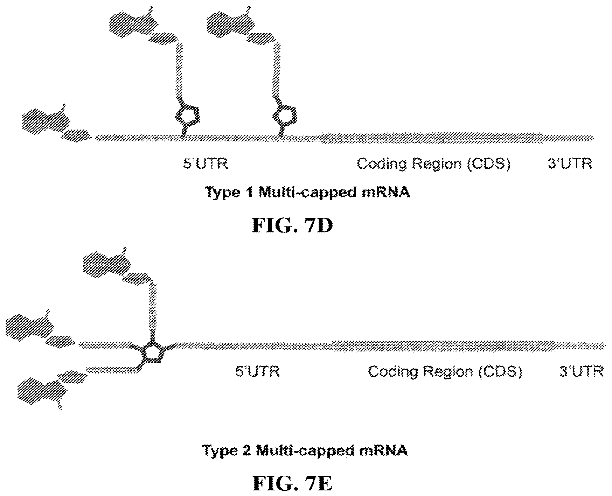

**FIGs. 7A-7E** show the conceptualization of chimeric mRNA bearing multiple 5'-caps. **FIG. 7A** depicts the chemical structure of the eukaryotic mRNA 5' cap. **FIG. 7B** shows a pseudo closed-loop model of cap dependent mRNA translation initiation. The 5' cap is recognized by eIF4E leading to formation of translation initiation complex and ribosome recruitment. **FIG. 7C** shows mocRNA bearing chemical conjugation handles are ligated to capped oligonucleotide via click chemistry. Multiple m7G caps result in increased binding to eIF4E and higher ribosome loading on the mRNA transcript. **FIG. 7D** shows Type 1 multi-capped mocRNA is featured by branching in the 5'UTR by chemical conjugation to capped oligonucleotides. **FIG. 7E** shows Type 2 multi-capped mocRNA is featured by ligation to a multivalent small molecule handle, which is conjugated to multiple capped oligonucleotides.

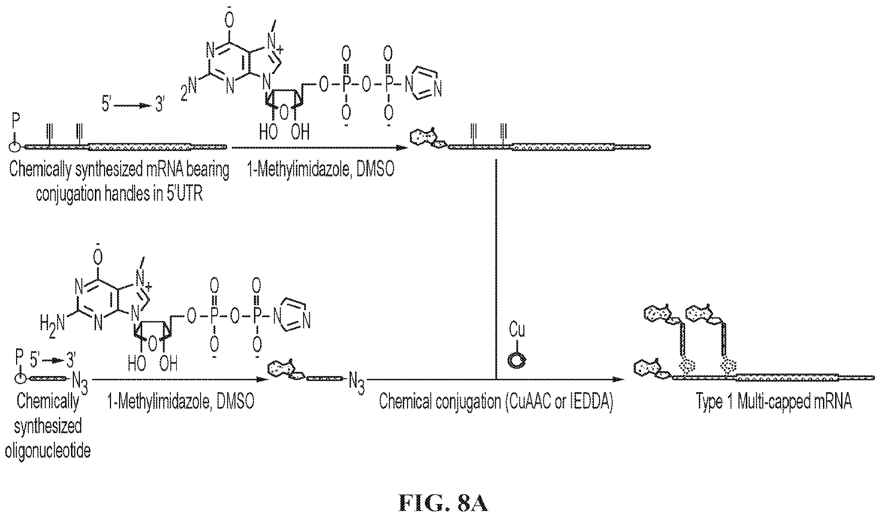

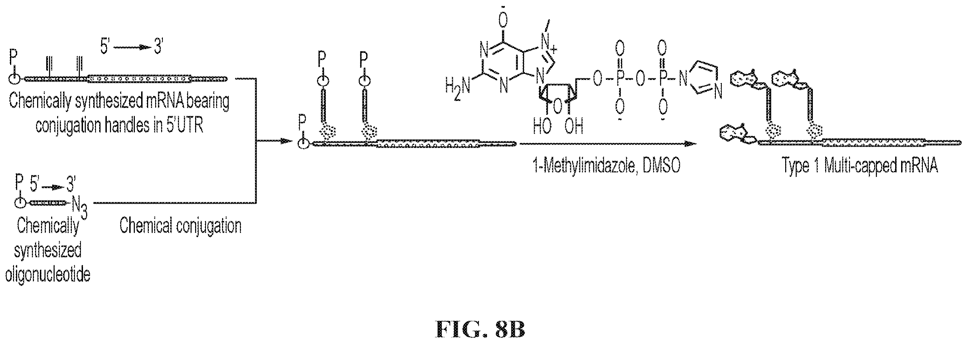

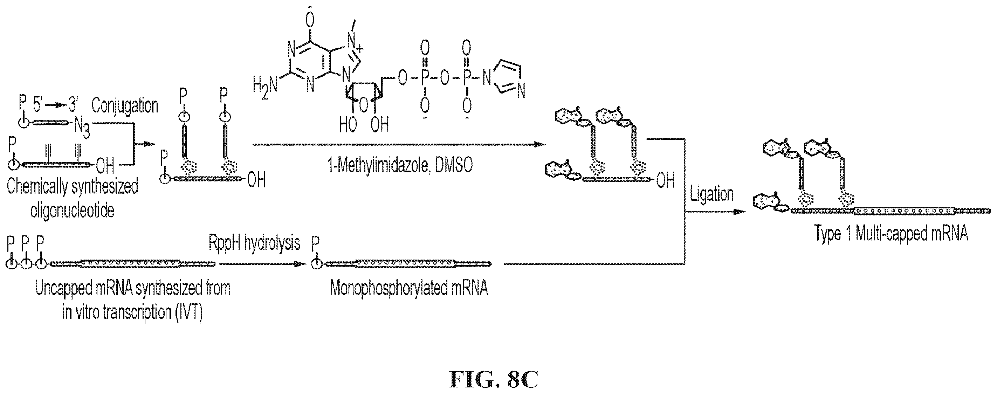

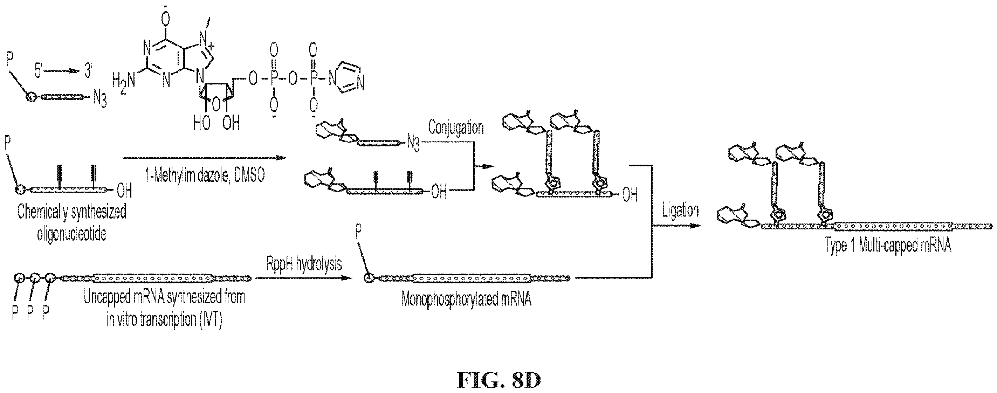

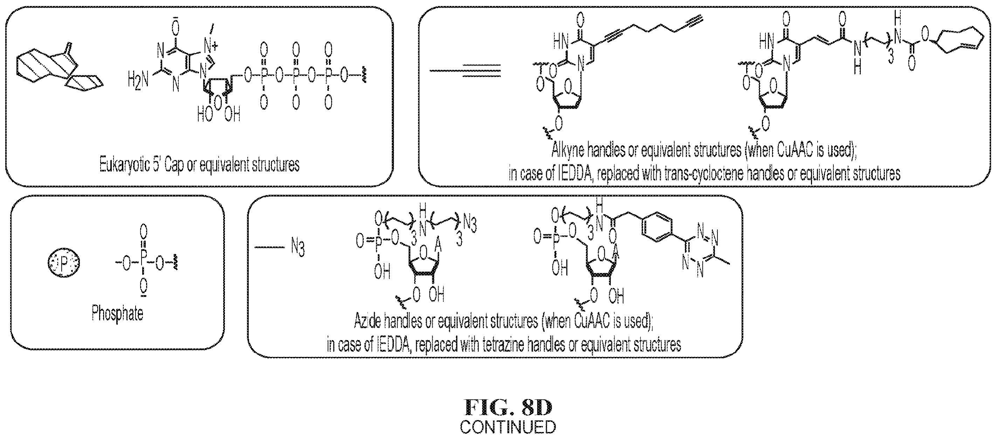

**FIGs. 8A-8D** show the general synthesis workflow of Type 1 multi-capped mRNA. **FIG. 8A** shows the chemically synthesized mRNA transcript and oligos bearing click chemistry handles were chemically capped with m7G-imidazolide, HPLC purified, and finally crosslinked by corresponding click reactions. **FIG. 8B** shows the chemically synthesized mRNA transcript and oligos bearing click chemistry handles were crosslinked by click reactions and HPLC purified. The conjugation product was subsequently capped using m7G-imidazolide to incorporate multiple caps. **FIG. 8C** shows the chemically synthesized oligos bearing click chemistry handles were crosslinked and HPLC purified to generate the branched oligo, which is subsequently capped by m7G-imidazolide to incorporate multiple caps. The multi-capped oligo was ligated to a 5'-monophosphorylated mRNA transcript generated by IVT and RppH hydrolysis. **FIG. 8D** shows an alternative workflow of **FIG. 8C****,** where the oligos were chemically capped first, and then crosslinked.

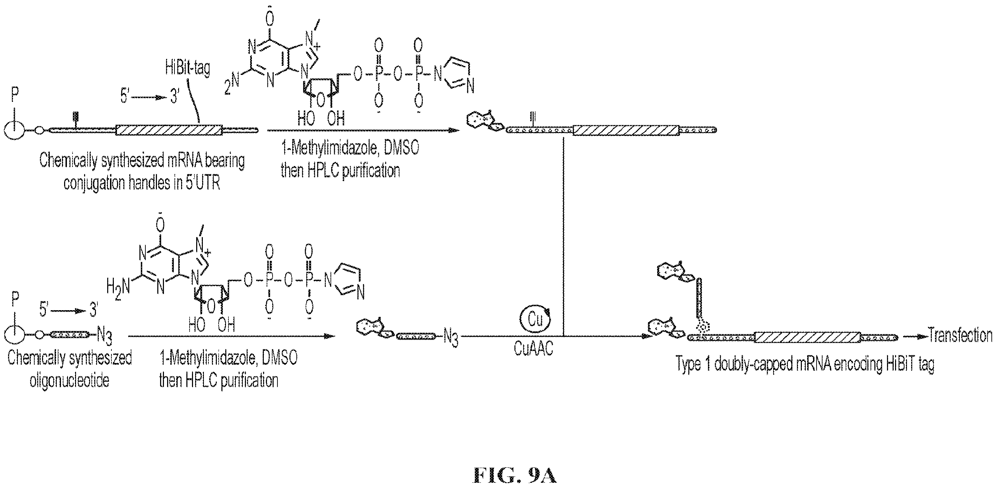

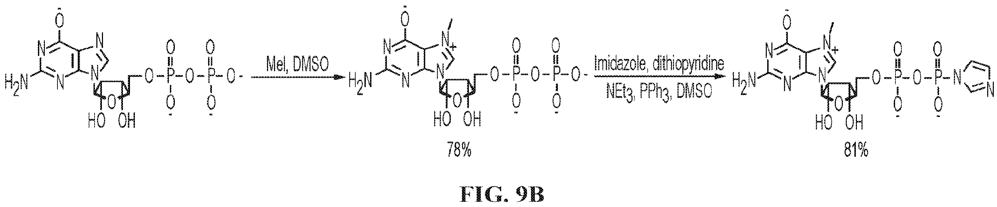

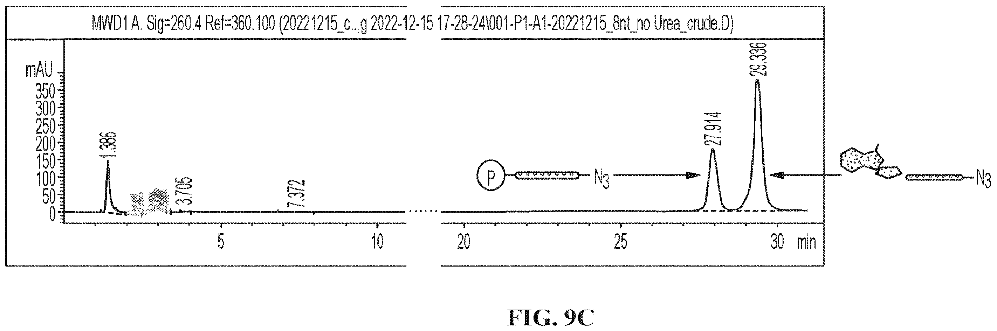

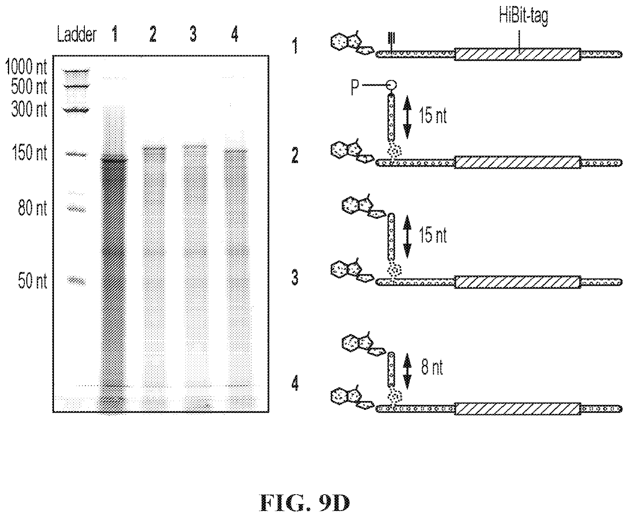

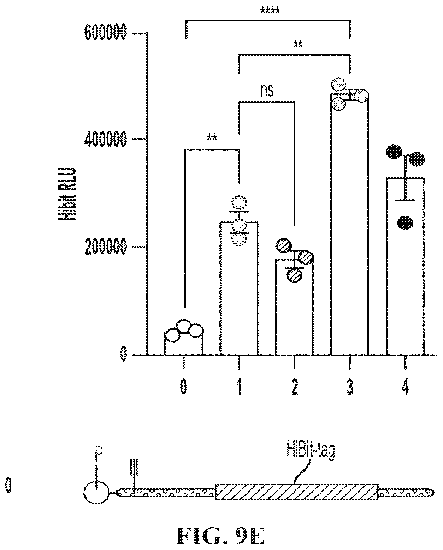

**FIGs. 9A-9D** show proof of concept experiment using Type 1 multi-capped mRNA encoding HiBit tag. **FIG. 9A** shows the synthetic HiBit mRNA having one EU handle in the 5'UTR was chemically capped, and ligated to a 5'-capped, 3'-azide oligonucleotide. The doubly-capped HiBit mRNA was then transfected to HeLa cell and evaluated by luciferase assay. **FIG. 9B** shows the synthesis of the m7G-imidazolide chemical capping reagent. **FIG. 9C** shows the representative HPLC traces for purifying uncapped and capped oligo with click chemistry handles. **FIG. 9D** shows an agarose gel electrophoresis of singly-capped/single-capped and branched/doubly-capped HiBit mRNAs. **FIG. 9E** shows Hibit bioluminescence 12 hrs post mRNA transfection. Mean ± sem. P values were calculated by unpaired t test with Welch's correction. **** P < 0.0001; ** P < 0.004; n.s. P > 0.1234.

### DETAILED DESCRIPTION

Provided herein are modified mRNAs comprising additional poly-A tails and/or additional 5' caps in order to improve stability of the modified mRNA in cells and thereby enhance the production of encoded gene products, such as proteins. Also provided are methods of making the modified mRNAs described herein by adding to the 3' end of a mRNA a poly-A region comprising multiple poly-A tails, and/or by adding to the 5' end of an mRNA a 5' cap region comprising multiple 5' caps. Various methods for producing poly-tailed and poly-capped mRNAs are contemplated herein. In one method, an oligonucleotide comprising a poly-A tail or an oligonucleotide comprising 5' cap oligonucleotide is ligated to the 3' or 5' end of a mRNA molecule, respectively. The ligated oligonucleotide is in turn attached to additional poly-A tailed or 5' capped oligonucleotides (e.g., via click chemistry reactions with modified oligonucleotides), depending on whether it is present at the 3' end or the 5' end of the modified mRNA (**FIGs. 1B** and **1D**). In an alternative method, a dendrimer that is attached to multiple oligonucleotides comprising either poly-A tails or 5' caps (e.g., via click chemistry reactions with modified oligonucleotides) is ligated to the 3' or 5' end of a mRNA molecule, respectively (**FIGs. 1C** and **1E**). Additionally, the present disclosure provides compositions, including pharmaceutical compositions, comprising one or more of the modified mRNAs provided herein. Kits containing reagents to produce the modified mRNAs are also described herein. Further provided herein are methods for administering one or more of the described modified mRNAs to a cell or subject, as well as kits for use in administering to a subject any one of the pharmaceutical compositions provided herein.

Conventional mRNAs comprise a poly-A tail with multiple adenosine nucleotides at the 3' end, and a 5' cap at the 5' end. Although each of these components protect the mRNA and help to recruit factors involved in protein translation, mRNAs are subject to degradation by exonucleases. Once exonucleases remove the poly-A tail and/or 5' cap and begin removing nucleotides of the open reading frame, the mRNA is unable to be translated into an encoded protein. One of the primary determinants of mRNA stability in a cell is the time required to degrade the ends of the mRNA. mRNAs that are more resistant to 3' and 5' exonuclease activity are degraded more slowly. The modified mRNAs of the present disclosure typically have longer half-lives, and are thus more stable, in cells. Due to their increased stability (i.e., resistance to exonuclease activity), the modified mRNAs of the present disclosure have greater translation efficiency (i.e., quantity of gene products, e.g., proteins, that are translated from the mRNA per second) than their unmodified counterparts. The modified mRNA may further contain one or more structural changes to the nucleobases, sugars, and/or phosphate linkages of the mRNA that also interfere with exonuclease activity. Moreover, the additional poly-A tails and/or 5' caps added to the mRNAs described herein may also enhance translation efficiency by introducing more sites on the mRNAs for binding by factors involved in eukaryotic protein translation. These effects may be combined, by modifying mRNA molecules to comprise additional poly-A tails and additional 5' caps. Modified mRNAs with increased stability in cells, and thus the ability to produce more of an encoded protein from a given RNA molecule, are useful for a variety of applications, including use in the treatment and prevention of disease, such as, for example, use in vaccines and in other RNA-based therapies, such as the delivery of mRNAs encoding essential enzymes, clotting factors, transcription factors, growth factors, cytokines, chemokines, antibodies, protein hormones, signaling proteins, structural proteins, or cell surface receptors to cells of a subject.

### Definitions

A "messenger RNA" ("mRNA"), as used herein, refers to a nucleic acid comprising an open reading frame encoding a gene produce, such as a protein, and a poly-A region that is 3' to the open reading frame. An mRNA may also comprise a 5' untranslated region (5' UTR) that is 5' to (upstream of) the open reading frame, and a 3' untranslated region that is 3' to (downstream of) the open reading frame. A mRNA may also comprise a 5' cap at the 5' end of the mRNA

An "open reading frame encoding a protein," as used herein, refers to a nucleic acid sequence comprising a coding sequence, that leads to the production of the protein when the open reading frame is translated. The nucleic acid sequence may be an RNA sequence, in which case translation of the RNA sequence produces a polypeptide with the amino acid sequence of the protein. The nucleic acid sequence may be a DNA sequence, in which case the protein is produced when an RNA polymerase uses the DNA sequence to transcribe an RNA molecule comprising an RNA sequence that is complementary to the DNA sequence, and translation of the RNA sequence produces a polypeptide with the amino acid sequence of the protein. An open reading frame typically begins with a START codon, such as AUG in the RNA sequence (ATG in the DNA sequence), and ends with a STOP codon, such as UAG, UAA, or UGA in the RNA sequence (TAG, TAA, or TGA in the DNA sequence), with the number of bases between the G of the START codon and the T or U of the STOP codon being a multiple of 3 (*e.g.*, 3, 6, 9).

An RNA molecule that can be translated is referred to as a messenger RNA, or mRNA. A DNA or RNA sequence encodes a gene through codons. A codon refers to a group of three nucleotides within a nucleic acid, such as DNA or RNA, sequence. An anticodon refers to a group of three nucleotides within a nucleic acid, such as a transfer RNA (tRNA), that are complementary to a codon, such that the codon of a first nucleic acid associates with the anticodon of a second nucleic acid through hydrogen bonding between the bases of the codon and anticodon. For example, the codon 5'-AUG-3' on an mRNA has the corresponding anticodon 3'-UAC-5' on a tRNA. During translation, a tRNA with an anticodon complementary to the codon to be translated associates with the codon on the mRNA, generally to deliver an amino acid that corresponds to the codon to be translated, or to facilitate termination of translation and release of a translated polypeptide from a ribosome.

Translation is the process in which the RNA coding sequence is used to direct the production of a polypeptide. The first step in translation is initiation, in which a ribosome associates with an mRNA, and a first transfer RNA (tRNA) carrying a first amino acid associates with the first codon, or START codon. The next phase of translation, elongation, involves three steps. First, a second tRNA with an anticodon that is complementary to codon following the START codon, or second codon, and carrying a second amino acid, associates with the mRNA. Second, the carbon atom of terminal, non-side chain carboxylic acid moiety of the first amino acid reacts with the nitrogen of the terminal, non-side chain amino moiety of the second amino acid carried, forming a peptide bond between the two amino acids, with the second amino acid being bound to the second tRNA, and the first amino acid bound to the second amino acid, but not the first tRNA. Third, the first tRNA dissociates from the mRNA, and the ribosome advances along the mRNA, such that the position at which the first tRNA associated with the ribosome is now occupied by the second tRNA, and the position previously occupied by the second tRNA is now free for an additional tRNA carrying an additional amino acid to associate with the mRNA. These three steps of 1) association of a tRNA carrying amino acid, 2) formation of a peptide bond, which adds an additional amino acid to a growing polypeptide, and 3) advancement of the ribosome along the mRNA, continue until the ribosome reaches a STOP codon, which results in termination of translation. Generally, tRNAs that associate with STOP codons do not carry an amino acid, so the association of a tRNA that does not carry an amino acid during the elongation step results in cleavage of the bond between the polypeptide and the tRNA carrying the final amino acid in the polypeptide, such that the polypeptide is released from the ribosome. Alternatively, ribosomes may dissociate from the mRNA and release the polypeptide if no tRNA associates with the STOP codon.

A "nucleic acid," or "polynucleotide," as used herein, refers to an organic molecule comprising two or more covalently bonded nucleotides. A "nucleotide," as used herein, refers to an organic molecule comprising a 1) a nucleoside comprising a sugar covalently bonded to a nitrogenous base (nucleobase); and 2) a phosphate group that is covalently bonded to the sugar of the nucleoside. Nucleotides in a polynucleotide are typically joined by a phosphodiester bond, in which the 3' carbon of the sugar of a first nucleotide is linked to the 5' carbon of the sugar of a second nucleic acid by a bridging phosphate group. Typically, the bridging phosphate comprises two non-bridging oxygen atoms, which are bonded only to a phosphorus atom of the phosphate, and two bridging oxygen atoms, each of which connects the phosphorus atom to either the 3' carbon of the first nucleotide or the 5' carbon of the second nucleotide. In a nucleic acid sequence describing the order of nucleotides in a nucleic acid, a first nucleotide is said to be 5' to (upstream of) a second nucleotide if the 3' carbon of first nucleotide is connected to the 5' carbon of the second nucleotide. Similarly, a second nucleotide is said to be 3' to (downstream of) a first nucleotide if the 5' carbon of the second nucleotide is connected to the 3' carbon of the first nucleotide. Nucleic acid sequences are typically read in 5'->3' order, starting with the 5' nucleotide and ending with the 3' nucleotide.

A "modified nucleotide," as used herein, refers to a nucleotide with a structure that is not the canonical structure of an adenosine nucleotide, cytidine nucleotide, guanine nucleotide, or uracil nucleotide. A canonical structure of a molecule refers to a structure that is generally known in the art to be the structure referred to by the name of the molecule. A canonical structure of an adenosine nucleotide, which comprises an adenine base, ribose sugar, and one or more phosphate groups, is shown below, in the form of adenosine monophosphate:

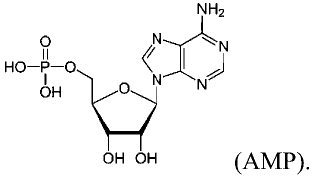

The canonical structure of AMP also refers to structures in which one or more hydroxyl groups of the phosphate and/or one or more hydroxyl groups of the sugar are deprotonated, and structures in which an oxygen atom of the phosphate and/or the 3' oxygen atom of the sugar are bound to an adjacent nucleotide in a nucleic acid sequence.

The canonical structure of a cytosine nucleotide which comprises a cytosine base, ribose sugar, and one or more phosphate groups, is shown below, in the form of cytidine monophosphate: 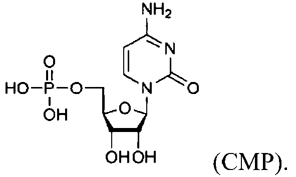 The canonical structure of CMP also refers to structures in which one or more hydroxyl groups of the phosphate and/or one or more hydroxyl groups of the sugar are deprotonated, and structures in which an oxygen atom of the phosphate and/or the 3' oxygen atom of the sugar are bound to an adjacent nucleotide in a nucleic acid sequence.

The canonical structure of a guanine nucleotide which comprises a guanine base, ribose sugar, and one or more phosphate groups, is shown below, in the form of guanosine monophosphate: 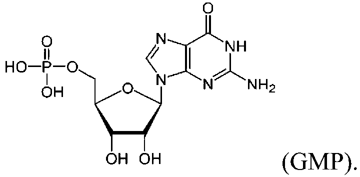 The canonical structure of GMP also refers to structures in which one or more hydroxyl groups of the phosphate and/or one or more hydroxyl groups of the sugar are deprotonated, and structures in which an oxygen atom of the phosphate and/or the 3' oxygen atom of the sugar are bound to an adjacent nucleotide in a nucleic acid sequence.

The canonical structure of a uracil nucleotide which comprises a uracil base, ribose sugar, and one or more phosphate groups, is shown below, in the form of uridine monophosphate: 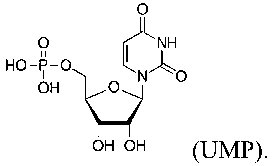 The canonical structure of UMP also refers to structures in which one or more hydroxyl groups of the phosphate and/or one or more hydroxyl groups of the sugar are deprotonated, and structures in which an oxygen atom of the phosphate and/or the 3' oxygen atom of the sugar are bound to an adjacent nucleotide in a nucleic acid sequence.

The structure of a modified nucleotide may differ from the structure of a canonical nucleotide due to one or more modifications in the sugar, nitrogenous base, or phosphate of the nucleotide. In some embodiments, the modified nucleotide comprises a modified nucleoside that is not the canonical structure of an adenine nucleoside, cytosine nucleoside, guanine nucleoside, or uracil nucleoside. As used herein

An example of a canonical structure of adenosine, an adenine nucleoside, is reproduced below: 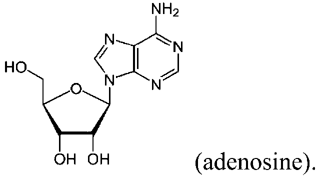 The canonical structure of adenosine also refers to structures in which one or more hydroxyl groups of the phosphate and/or one or more hydroxyl groups of the sugar are deprotonated, structures in which the 5' carbon is bound to a 5' phosphate in a nucleic acid sequence, and structures in which a 3' oxygen atom is bound to a 5' phosphate group of an adjacent nucleotide in a nucleic acid sequence.

An example of a canonical structure of cytidine, a cytosine nucleoside, is reproduced below: 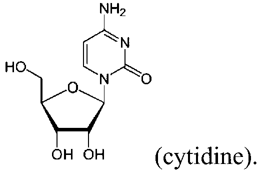 The canonical structure of cytidine also refers to structures in which one or more hydroxyl groups of the phosphate and/or one or more hydroxyl groups of the sugar are deprotonated, structures in which the 5' carbon is bound to a 5' phosphate in a nucleic acid sequence, and structures in which a 3' oxygen atom is bound to a 5' phosphate group of an adjacent nucleotide in a nucleic acid sequence.

An example of a canonical structure of guanosine, a guanine nucleoside, is reproduced below: 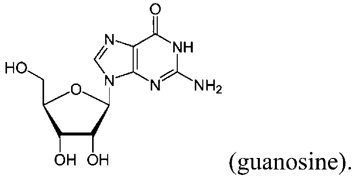 The canonical structure of guanosine also refers to structures in which one or more hydroxyl groups of the phosphate and/or one or more hydroxyl groups of the sugar are deprotonated, structures in which the 5' carbon is bound to a 5' phosphate in a nucleic acid sequence, and structures in which a 3' oxygen atom is bound to a 5' phosphate group of an adjacent nucleotide in a nucleic acid sequence.

An example of a canonical structure of uridine, a uracil nucleoside, is reproduced below: 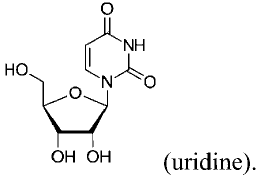 The canonical structure of uridine also refers to structures in which one or more hydroxyl groups of the phosphate and/or one or more hydroxyl groups of the sugar are deprotonated, structures in which the 5' carbon is bound to a 5' phosphate in a nucleic acid sequence, and structures in which a 3' oxygen atom is bound to a 5' phosphate group of an adjacent nucleotide in a nucleic acid sequence.

A "ligase," as used herein, refers to an enzyme that is capable of forming a covalent bond between two nucleotides, and the process of "ligation" refers to the formation of the covalent bond between the two nucleotides.

A "tailing nucleic acid," as used herein, refers to a nucleic acid that is ligated onto the 3' end of another nucleic acid. A tailing nucleic acid may comprise poly-A tails.

A "capping nucleic acid," as used herein, refers to a nucleic acid that is ligated onto the 5' end of another nucleic acid. A capping nucleic acid may comprise 5' caps.

A "poly-A tail," as used herein, refers to a nucleic acid sequence comprising adenosine nucleotides that is attached to the 3' end of a nucleic acid, such as an RNA. A "poly-A region," as used herein, refers to a nucleic acid comprising one or more poly-A tails. A poly-A tail or poly-A region may consist of nucleotides that are 25-100%, 30-100%, 40-100%, 50-100%, 60-100%, 70-100%, 80-100%, 90-100%, 95-100%, 96-100%, 97-100%, 98-100%, or 99-100% adenosine nucleotides. The adenosine nucleotides comprised by a poly-A tail or poly-A region may be canonical adenosine nucleotides or modified (non-canonical) adenosine nucleotides.

A "5' cap," as used herein, refers to one or more nucleotides that are covalently attached to the 5' end of a nucleic acid, such as an RNA. A "5' cap region," as used herein, refers to a nucleic acid comprising one or more 5' caps. A 5' cap may comprise a 5' capping nucleotide that is attached to the 5' end of a mRNA by a 5' to 5' triphosphate internucleotide linkage. In some embodiments, a nucleotide attached to a mRNA by a 5' to 5' triphosphate internucleotide linkage is referred to as a "native" 5' capping nucleotide. In some embodiments, a native 5' capping nucleotide is a 7-methylguanosine (m7G) nucleotide. In some embodiments, a 5' cap is a modified 5' cap, comprising one or more modified nucleotides, such as the 5' capping nucleotide, or one or more modified internucleotide modifications, such as modifications to the 5' to 5' triphosphate internucleotide linkage. In some embodiments, a 5' cap comprises one or more nucleotides with a sugar modification, such as 2'-O-methylation.

An example of a canonical structure of 7-methylguanosine attached to a ribonucleic acid sequence (e.g., a mRNA) by a 5' to 5' triphosphate internucleotide linkage is reproduced below:

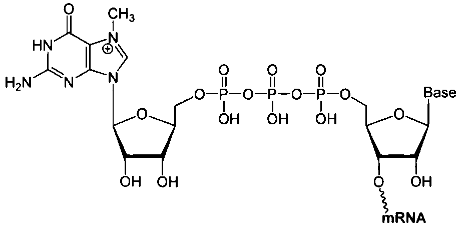

A "brushed oligonucleotide," as used herein, refers to a nucleic acid sequence comprising an internal covalent attachment to either the 3' end or the 5' end of one or more second nucleic acid sequences.

A "dendrimer," as used herein, refers to an organic polymeric compound comprising a core structure to which multiple end groups, referred to as "dendrons", are covalently attached. The dendrons of a dendrimer may be modified for attachment to oligonucleotides (e.g., via a click chemistry reaction).

### Modified mRNAs

In some aspects, the present disclosure provides modified mRNAs comprising i) two or more poly-A tails; and/or ii) two or more 5' caps, wherein each poly-A tail is comprised by a poly-A region at the 3' end of the mRNA, and each 5' cap is comprised by a 5' cap region at the 5' end of the mRNA. In some embodiments, a modified mRNA provided herein comprises one poly-A tail and two or more 5' caps. In some embodiments, a modified mRNA provided herein comprises two or more poly-A tails and one 5' cap. In some embodiments, a modified mRNA provided herein comprises two or more poly-A tails and two or more 5' caps.

As defined herein, the "poly-A region" of an mRNA, also called the "poly(A) region", refers to a region of an mRNA that is 3' to (downstream of) the open reading frame (ORF) and 3' untranslated region (UTR), comprising multiple, consecutive adenosine nucleotides (i.e., any adenosine nucleotide that is covalently linked to at least one other adenosine nucleotide). The poly-A region typically comprises between 50 and 300 consecutive adenosine nucleotides, and may encompass multiple non-adenosine nucleotides that are upstream of, downstream of, or interspersed between the consecutive adenosine nucleotides. The "5' cap region" of an mRNA refers to a region of an mRNA that is 5' to (upstream of) the ORF and 5' UTR. In cells, after transcription of a DNA sequence, which produces a precursor messenger RNA (pre-mRNA), the poly-A tail is added by a polyadenylating enzyme, such as a poly-A polymerase (PAP), resulting in a long sequence of multiple, consecutive adenosine nucleotides, at the 3' end of the RNA, while the 5' cap is added by a 5' capping enzyme, such as mRNA guanylyltransferase. The poly-A region and 5' cap each play multiple roles that are important in the production of proteins encoded by mRNAs. First, the poly-A region provides an attachment site for poly-A binding proteins (PABPs), which associate with the mRNA in the nucleus and promote export into the cytoplasm (see, *e.g.,* Tudek et al. Philos Trans R Soc Lond B Biol Sci. 2018. 373(1762):20180169). Second, the presence of a poly-A tail and a 5' cap in an mRNA facilitates the initiation of translation (see, *e.g.,* Gallie. Genes & Dev. 1991. 5:2108-2116, and Munroe et al. Mol Cell Biol. 1990. 10(7):3441-3455). Finally, the poly-A tail and 5' cap stabilize the mRNA by protecting the ORF from the activity of exonucleases, such as polynucleotide phosphorylase (PNPase), which can remove 3' and 5' nucleotides from an mRNA. As an exonuclease removes nucleotides, the mRNA becomes progressively shorter, and once all of the nucleotides downstream of the open reading frame are removed, the nucleotides removed by the exonuclease will be nucleotides of the ORF. Removal of nucleotides from the ORF prevents translation of the encoded protein. Additionally, the association of an exonuclease with the mRNA near the ORF can inhibit translation by sterically hindering ribosomes and tRNAs from associating with the mRNA. Removal of the poly-A tail particularly is often cited as a rate-limiting step in mRNA degradation, with the life span of an mRNA in a cell being determined by the time required to remove its poly-A tail (see, *e.g.,* Dreyfus et al., Cell. 2002. 111(5):611-613). The composition of a poly-A tail of an mRNA varies, but contains approximately 75 adenosine nucleotides in yeast cells and 250 adenosine nucleotides in mammalian cells. The composition of a 5' cap in a mRNA is less variable, typically comprising a 5' 7-methylguanosine attached to a to the mRNA by a 5' to 5' triphosphate internucleotide linkage, however certain native modifications are known to occur natively in the nucleotides to which the 5' cap is attached, such as 2'-O-methylation in the first nucleotide of the mRNA (cap 1) and 2'-O-methylation in the first and second nucleotide of the mRNA (cap 2). 5' caps in which 2'-O-methylation does not occur are known as "cap 0" caps.

In some embodiments of the modified mRNAs provided herein, the modified mRNA comprises a poly-A region and/or 5' cap region that does not comprise modified nucleotides (i.e., each and every nucleotide in the poly-A region and/or 5' cap region is a canonical nucleotide). In some embodiments, 50% to 60%, 60% to 70%, 70% to 80%, 80% to 90%, 90% to 95%, 95% to 99%, or 100% of the nucleotides of the poly-A region or 5' cap region are canonical nucleotides. In some embodiments, 50% to 60%, 60% to 70%, 70% to 80%, 80% to 90%, 90% to 95%, 95% to 99%, or 100% of the adenosine nucleotides of the poly-A region are canonical adenosine nucleotides.

In some embodiments of the modified mRNAs provided herein, the modified mRNA comprises one or more modified nucleotides in the poly-A region and/or 5' cap region of the mRNA. In some embodiments, the poly-A region and/or 5' cap region includes one or more nucleotides that are not canonical adenosine, cytidine, guanosine, or uridine nucleotides. In some embodiments, the poly-A region comprises one or more nucleotides that are 3' to (downstream of) a nucleic acid sequence comprising multiple, consecutive adenosine nucleotides. In some embodiments, the poly-A region comprises 10-25 consecutive adenosine nucleotides, which may be canonical adenosine nucleotides or modified adenosine nucleotides. In some embodiments, the poly-A region comprises 10-15 consecutive adenosine nucleotides, 15-20 consecutive adenosine nucleotides, or 20-25 consecutive adenosine nucleotides, which may be canonical adenosine nucleotides or modified adenosine nucleotides. In some embodiments, the poly-A region comprises at least 25 consecutive adenosine nucleotides, which may be canonical adenosine nucleotides or modified adenosine nucleotides. In some embodiments, the poly-A region comprises 25-500 consecutive adenosine nucleotides, which may be canonical adenosine nucleotides or modified adenosine nucleotides. In some embodiments, the poly-A region comprises 25-300 consecutive adenosine nucleotides. In some embodiments, the poly-A region comprises at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 consecutive adenosine nucleotides. In some embodiments, the 5' cap region comprises between 1 and 3, between 3 and 5, between 5 and 7, or between 7 and 10 5' caps. In some embodiments, the poly-A region and/or 5' cap region comprises between 10-500 nucleotides. In some embodiments, the poly-A region and/or 5' cap region comprises between 10 and 15, between 15 and 20, between 20 and 25, between 25 and 50, between 50 and 100, between 100 and 150, between 150 and 200, between 200 and 300, between 300 and 400, or between 400 and 500 nucleotides.

In some embodiments, each and every nucleotide of the modified mRNA comprises a canonical phosphate group. In some embodiments, the modified mRNA comprises one or more nucleotides that comprise a modified phosphate group. A modified phosphate group to which two nucleotides of the modified mRNA are attached is referred to as a "modified internucleotide linkage," or a "modified internucleoside linkage." A modified phosphate group is a phosphate group that differs from the canonical structure of phosphate (phosphodiester). An example of a canonical structure of a phosphate (phosphodiester) is shown below: 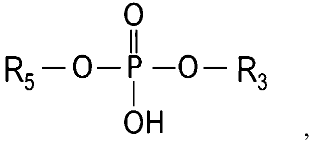 where R₅ and R₃ are atoms or molecules to which the canonical phosphate is bonded. For example, for a phosphate in a nucleic acid sequence, R₅ may refer to the upstream nucleotide of the nucleic acid, and R₃ may refer to the downstream nucleotide of the nucleic acid. The canonical structure of phosphate also refers to structures in which one or more hydroxyl groups of the phosphate are deprotonated, or in which an oxygen atom of the phosphate is bonded to an adjacent nucleotide in a nucleic acid sequence. Non-limiting examples of modified phosphate groups that can be substituted for a canonical phosphate in a nucleic acid include phosphorothioate (PS; R and/or S stereoisomers), thiophosphate, 5'-O-methylphosphonate, 3'-O-methylphosphonate, 5'-hydroxyphosphonate, hydroxyphosphanate, phosphoroselenoate, selenophosphate, phosphoramidate, carbophosphonate, methylphosphonate, phenylphosphonate, ethylphosphonate, H-phosphonate, guanidinium ring, triazole ring, boranophosphate (BP), methylphosphonate, guanidinopropyl phosphoramidate, peptide bonds (e.g., in peptide nucleic acids), thiophosphoramidate.

In some embodiments of the modified mRNAs comprising modified nucleotides provided herein, at least one modified nucleotide comprises a modified nucleobase. In some embodiments, at least one modified nucleotide comprises a modified sugar. In some embodiments, at least one modified nucleotide comprises a modified phosphate. In some embodiments, at least one modified nucleotide comprises a modified nucleobase selected from the group consisting of: xanthine, allyaminouracil, allyaminothymidine, hypoxanthine, digoxigeninated adenine, digoxigeninated cytosine, digoxigeninated guanine, digoxigeninated uracil, 6-chloropurineriboside, N6-methyladenine, methylpseudouracil, 2-thiocytosine, 2-thiouracil, 5-methyluracil, 4-thiothymidine, 4-thiouracil, 5,6-dihydro-5-methyluracil, 5,6-dihydrouracil, 5-[(3-Indolyl)propionamide-N-allyl]uracil, 5-aminoallylcytosine, 5-aminoallyluracil, 5-bromouracil, 5-bromocytosine, 5-carboxycytosine, 5-carboxymethylesteruracil, 5-carboxyuracil, 5-fluorouracil, 5-formylcytosine, 5-formyluracil, 5-hydroxycytosine, 5-hydroxymethylcytosine, 5-hydroxymethyluracil, 5-hydroxyuracil, 5-iodocytosine, 5-iodouracil, 5-methoxycytosine, 5-methoxyuracil, 5-methylcytosine, 5-methyluracil, 5-propargylaminocytosine, 5-propargylaminouracil, 5-propynylcytosine, 5-propynyluracil, 6-azacytosine, 6-azauracil, 6-chloropurine, 6-thioguanine, 7-deazaadenine, 7-deazaguanine, 7-deaza-7-propargylaminoadenine, 7-deaza-7-propargylaminoguanine, 8-azaadenine, 8-azidoadenine, 8-chloroadenine, 8-oxoadenine, 8-oxoguanine, araadenine, aracytosine, araguanine, arauracil, biotin-16-7-deaza-7-propargylaminoguanine, biotin-16-aminoallylcytosine, biotin-16-aminoallyluracil, cyanine 3-5-propargylaminocytosine, cyanine 3-6-propargylaminouracil, cyanine 3-aminoallylcytosine, cyanine 3-aminoallyluracil, cyanine 5-6-propargylaminocytosine, cyanine 5-6-propargylaminouracil, cyanine 5-aminoallylcytosine, cyanine 5-aminoallyluracil, cyanine 7-aminoallyluracil, dabcyl-5-3-aminoallyluracil, desthiobiotin-16-aminoallyl-uracil, desthiobiotin-6-aminoallylcytosine, isoguanine, N1-ethylpseudouracil, N1-methoxymethylpseudouracil, N1-methyladenine, N1-methylpseudouracil, N1-propylpseudouracil, N2-methylguanine, N4-biotin-OBEA-cytosine, N4-methylcytosine, N6-methyladenine, O6-methylguanine, pseudoisocytosine, pseudouracil, thienocytosine, thienoguanine, thienouracil, xanthosine, 3-deazaadenine, 2,6-diaminoadenine, 2,6-daminoguanine, 5-carboxamide-uracil, 5-ethynyluracil, N6-isopentenyladenine (i6A), 2-methyl-thio-N6-isopentenyladenine (ms2i6A), 2-methylthio-N6-methyladenine (ms2m6A), N6-(cis-hydroxyisopentenyl)adenine (io6A), 2-methylthio-N6-(cis-hydroxyisopentenyl)adenine (ms2io6A), N6-glycinylcarbamoyladenine (g6A), N6-threonylcarbamoyladenine (t6A), 2-methylthio-N6-threonyl carbamoyladenine (ms2t6A), N6-methyl-N6-threonylcarbamoyladenine (m6t6A), N6-hydroxynorvalylcarbamoyladenine (hn6A), 2-methylthio-N6-hydroxynorvalyl carbamoyladenine (ms2hn6A), N6,N6-dimethyladenine (m62A), and N6-acetyladenine (ac6A). In some embodiments, at least one modified nucleotide comprises a modified sugar selected from the group consisting of 2'-thioribose, 2',3'-dideoxyribose, 2'-amino-2'-deoxyribose, 2' deoxyribose, 2'-azido-2'-deoxyribose, 2'-fluoro-2'-deoxyribose, 2'-O-methylribose, 2'-O-methyldeoxyribose, 3'-amino-2',3'-dideoxyribose, 3'-azido-2',3'-dideoxyribose, 3'-deoxyribose, 3'-O-(2-nitrobenzyl)-2'-deoxyribose, 3'-O-methylribose, 5'-aminoribose, 5'-thioribose, 5-nitro-1-indolyl-2'-deoxyribose, 5'-biotin-ribose, 2'-O,4'-C-methylene-linked, 2'-O,4'-C-amino-linked ribose, and 2'-O,4'-C-thio-linked ribose. In some embodiments, at least one modified nucleotide comprises a 2' modification, such as a locked-nucleic acid (LNA) modification (*i.e.*, a nucleotide comprising an additional carbon atom bound to the 2' oxygen and 4' carbon of ribose) or 2'-O-methylation. In some embodiments, at least one modified nucleotide comprises a modified phosphate selected from the group consisting of phosphorothioate (PS), thiophosphate, 5'-O-methylphosphonate, 3'-O-methylphosphonate, 5'-hydroxyphosphonate, hydroxyphosphanate, phosphoroselenoate, selenophosphate, phosphoramidate, carbophosphonate, methylphosphonate, phenylphosphonate, ethylphosphonate, H-phosphonate, guanidinium ring, triazole ring, boranophosphate (BP), methylphosphonate, and guanidinopropyl phosphoramidate.

In some embodiments, the modified mRNA comprises more than one type of modified nucleotide. In some embodiments, the modified mRNA comprises at least a first modified nucleotide, and a second modified nucleotide that has a different structure from the first modified nucleotide. Nucleotides may differ in structure due to differences in the nucleobase, sugar, and/or phosphate group. In some embodiments, the modified mRNA comprises at least a first modified phosphate, and a second modified phosphate that has a different structure from the first modified phosphate. In some embodiments, the modified mRNA comprises a first modified nucleoside and a second modified nucleoside.

Aspects of the present disclosure relate to modified mRNAs comprising poly-A regions with 10 or more adenosine nucleotides. An adenosine nucleotide is a nucleotide comprising an adenine nucleoside and a phosphate group. An adenosine nucleoside comprises a sugar and an adenine base. In some embodiments, the poly-A region comprises 10 or more canonical adenosine nucleotides, e.g., 10-15 canonical adenosine nucleotides, 15-20 canonical adenosine nucleotides, or 20-25 canonical adenosine nucleotides. In some embodiments, the poly-A region comprises 25 or more canonical adenosine nucleotides, e.g., 25-500 canonical adenosine nucleotides. A canonical adenosine nucleotide comprises an adenine base, ribose sugar, and phosphate group, as arranged in the structure of adenosine monophosphate (AMP). In some embodiments, the one or more of the hydroxyl groups of the phosphate and/or the 3' hydroxyl group of the ribose are deprotonated, comprising an oxygen ion instead of an -OH group, as shown by the structure: 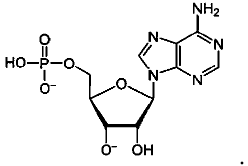 When present in a nucleic acid sequence of an mRNA, a canonical adenosine comprises the following structure and is connected to adjacent nucleotides in the following manner: 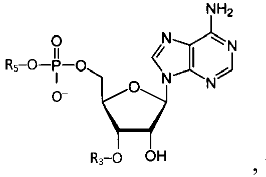 where R₅ is an adjacent nucleotide that is 5' to (upstream of) the adenosine nucleotide in the mRNA, and R₃ is an adjacent nucleotide that is 3' to (downstream of) the adenosine nucleotide in the mRNA. In some embodiments, the canonical adenosine nucleotide is the 3' terminal nucleotide (last nucleotide) of a linear mRNA, R₃ is a hydrogen, and the 3' terminal nucleotide comprises a 3' terminal hydroxyl (-OH) group. In some embodiments, the canonical adenosine nucleotide is the 3' terminal nucleotide (last nucleotide) of a linear mRNA, and R₃ is an electron.

Aspects of the present disclosure also relate to modified mRNAs comprising 5' cap regions with 2 or more 5' caps. A 5' cap may comprise a 5' capping nucleotide, the 5' terminal nucleotide (first nucleotide) of a linear mRNA, that is attached to the mRNA through a 5' to 5' triphosphate linkage. In some embodiments, the cap comprises one or more modified nucleotides, such as, for example, one or more modified nucleotides comprising a 2' modification, such as a locked-nucleic acid (LNA) modification (i.e., a nucleotide comprising an additional carbon atom bound to the 2' oxygen and 4' carbon of ribose) or 2'-O-methylation. In some embodiments, the 5' cap comprises a 5' capping nucleotide that is attached to the mRNA through a noncanonical linkage, such as a 5'-5' triphosphate linkage that is modified by 5'-phosphorothiolate, or a 5'-5' tetraphosphate. In some embodiments, the 5' capping nucleotide is a canonical 5' capping nucleotide, such as a 7-methylguanosine (m7G) nucleotide:

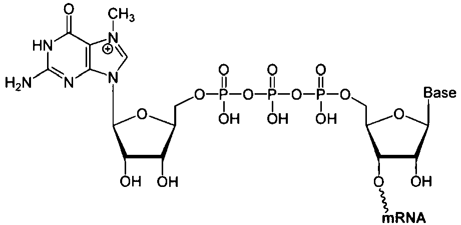

In some embodiments of the modified mRNAs provided herein, the mRNA comprises a 5' untranslated region (5' UTR) and a 3' untranslated region (3' UTR). 5' and 3' UTRs are sequences within an mRNA that do not encode amino acids of the protein encoded by the mRNA, and are thus not part of the open reading frame. The 5' UTR is 5' to (upstream of) the open reading frame. The 3' UTR is 3' to (downstream of) the open reading frame. In some embodiments, the 3' UTR comprises one or more nucleotides that are 3' to the open reading frame and 5' to (upstream of) the poly-A region of the mRNA.

In some embodiments of the mRNAs provided herein, the mRNA comprises, in 5'-to-3' order: 1) a 5' cap region; 2) a 5' UTR; 3) an open reading frame (ORF); 4) a 3' UTR; and 5) a poly-A region. In some embodiments, the first nucleotide of the 5' UTR is 3' to (downstream of) the last nucleotide of the 5' cap region, and the last nucleotide of the 5' UTR is 5' to (upstream of) the first nucleotide of the open reading frame. In some embodiments, the first nucleotide of the open reading frame is 3' to (downstream of) the last nucleotide of the 5' UTR, and the last nucleotide of the open reading frame is 5' to (upstream of) the first base of the 3' UTR. In some embodiments, the open reading frame is between the last nucleotide of the 5' UTR and the first nucleotide of the 3' UTR. In some embodiments, the first nucleotide of the 3' UTR is 3' to (downstream of) the last nucleotide of the open reading frame, and the last nucleotide of the 3' UTR is 5' to (upstream of) the first nucleotide of the poly-A region. In some embodiments, the 5' UTR is between the last nucleotide of the 5' cap region and the first nucleotide of the open reading frame. In some embodiments, the 3' UTR is between the last nucleotide of the open reading frame and the first nucleotide of the poly-A region. In some embodiments, the last nucleotide of the 5' cap region is 5' to (upstream of) the first nucleotide of the 5' UTR. In some embodiments, the first nucleotide of the poly-A region is 3' to (downstream of) the last nucleotide of the 3' UTR.

In some embodiments, the mRNA is a linear mRNA. A linear mRNA is an mRNA with a 5' terminal nucleotide and a 3' terminal nucleotide. The 5' terminal nucleotide of a linear mRNA is covalently bonded to only one adjacent nucleotide of the mRNA, with the adjacent nucleotide occurring 3' to the 5' terminal nucleotide in the nucleic acid sequence of the mRNA. The 3' terminal nucleotide of a linear mRNA is covalently bonded to only one adjacent nucleotide of the mRNA, with the adjacent nucleotide occurring 5' to the 3' terminal nucleotide in the nucleic acid sequence of the mRNA. In a nucleic acid sequence comprising every nucleotide of a linear mRNA in 5'-to-3' order, the 5' terminal nucleotide is the first nucleotide in the sequence, and the 3' terminal nucleotide is the last nucleotide in the sequence.

In some embodiments of the linear mRNAs provided herein, the mRNA comprises a 5' cap. Most mRNAs produced in eukaryotic cells include a 5' cap that is added during processing of the pre-mRNA into a mature mRNA. The 5' cap plays multiple roles in the process of mRNA production, export, and translation. First, assembly of the spliceosome, which mediates removal of introns from the pre-mRNA requires binding of the nuclear cap-binding complex (CBC) to the 5' cap. Furthermore, interactions between the CBC and nuclear pores mediate the export of mRNA from into the cytoplasm, beginning with the 5' end. Finally, CBC bound to the 5' cap mediates the recruitment of multiple factors, such as CBP80, CTIF, eIF3g, eIF4III, Met-tRNAi, and ribosomal subunits, which are required for the initiation of translation (see, e.g., Ramanathan et al. Nucleic Acids Res. 2016. 44(16):7511-7526). In some embodiments, the 5' cap comprises a 7-methylguanosine. In some embodiments, the 7-methylguanosine comprises the structure:

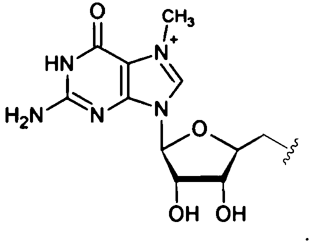

Cap analogs, which herein are also referred to as synthetic cap analogs, chemical caps, chemical cap analogs, or structural or functional cap analogs, differ from natural (i.e., endogenous, wild-type or physiological) 5'-caps in their chemical structure, while retaining cap function. Cap analogs may be chemically (i.e., non-enzymatically) or enzymatically synthesized and/or linked to a nucleic acid molecule. For example, the Anti-Reverse Cap Analog (ARCA) cap contains two guanines linked by a 5'-5'-triphosphate group, wherein one guanine contains an N7 methyl group as well as a 3'-O-methyl group (i.e., N7,3'-O-dimethyl-guanosine-5'-triphosphate-5'-guanosine (m⁷G-3' mppp-G; which may equivalently be designated 3' O-Mem7G(5')ppp(5')G). The 3'-O atom of the other, unmodified, guanine becomes linked to the 5'-terminal nucleotide of the capped nucleic acid molecule (e.g. an mRNA or mmRNA). The N7- and 3'-O-methylated guanine provides the terminal moiety of the capped nucleic acid molecule (e.g., mRNA or mmRNA).

Another exemplary cap is mCAP, which is similar to ARCA but has a 2'-O-methyl group on guanosine (i.e., N7,2'-O-dimethyl-guanosine-5'-triphosphate-5'-guanosine, m7Gm-ppp-G). Non-limiting examples of 5' cap structures include 7-benzylguanosine (Bn7G), 7-(4-chlorophenoxyethyl)-guanosine and its analogs (see, e.g., Kore et al. (2013) Bioorganic & Medicinal Chemistry 21(15):4570-4574), 7-ethyl guanosine (e7G), 7-propyl guanosine (p7G), 7-isopropyl guanosine (ip7G), 7-butyl guanosine (b7G), 7-isobutyl guanosine (ib7G), 7-cyclopentyl guanosine (cp7G), 7-(carboxymethyl) guanosine (cm7G), 7-benzyl guanosine (bn7G), 7-(2-phenylethyl) guanosine [7-(2-PhEt)G], 7-(1-phenylethyl) guanosine [7-(1-PhEt)G], m⁷Gppp_{BH3}G (D1 and D2 stereoisomers), m⁷Gpp_{BH3}G (D1 and D2 stereoisomers), m⁷Gp_{BH3}G (D1 and D2 stereoisomers), m⁷Gpp_{BH3}pm⁷G, m₂^{7,2'-*O*}Gppp_{BH3}G (D1 and D2 stereoisomers), m₂7^{,2'-*O*}Gpp_{BH3}pG (D1 and D2 diastereomers), m2^{7,2'-*O*}GppspG (D1 and D2 diastereomers), N-Arylmethyl analogs (see, e.g., Wojcik et al.(2021) Pharmaceutics 13(11) 1941), glyceryl, inverted deoxy abasic residue (moiety), 4',5'-methylene nucleotide, 1 -(beta-D- erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5-dihydroxypentyl nucleotide, 3'-3 '-inverted nucleotide moiety, 3'-3'-inverted abasic moiety, 3'-2'-inverted nucleotide moiety, 3'-2 '-inverted abasic moiety, 1,4-butanediol phosphate, 3'-phosphoramidate, hexylphosphate, aminohexyl phosphate, 3'-phosphate, 3'-phosphorothioate, phosphorodithioate, or bridging or non-bridging methylphosphonate moiety. Further modified 5'-cap structures which may be used in the context of the present invention are cap1 (additional methylation of the ribose of the adjacent nucleotide of m7GpppN), cap2 (additional methylation of the ribose of the 2nd nucleotide downstream of the m7GpppN), cap3 (additional methylation of the ribose of the 3rd nucleotide downstream of the m7GpppN), cap4 (additional methylation of the ribose of the 4th nucleotide downstream of the m7GpppN), ARCA (anti-reverse cap analogue), modified ARCA (e.g., phosphothioate modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

In some embodiments, the 5' cap comprises one or more phosphates connecting the 7-methylguanosine to an adjacent nucleotide of the modified mRNA. In some embodiments, one or more phosphates of the 5' cap is a modified phosphate selected from the group consisting of phosphorothioate, triazole ring, dihalogenmethylenebisphosphonate, imidodiphosphate, and methylenebis(phosphonate). In some embodiments, the 7-methylguanosine is connected to an adjacent nucleotide of the mRNA by a 5'-to-5' triphosphate bridge. In some embodiments, the 5' cap comprises the structure: 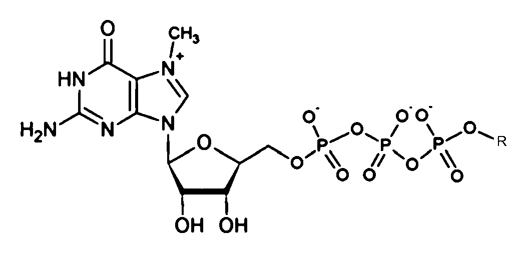 with R being the 5' carbon of the first transcribed nucleotide of the mRNA. In some embodiments, the 5' cap comprises a 3'-O-Me-m7G(5')ppp(5')G.

In some embodiments, the sugar backbone of the mRNA comprises ribose. In some embodiments, the sugar backbone comprises modifications. Non-limiting examples of sugar modifications include a 2' modification in the sugar residue such as 2'-amino 2'-O-methyl, 2'-O-alkyl, 2'-O-alkyl-O-alkyl, morpholino nucleic acids or 2'-fluoro-modified nucleotides. In some embodiments, the mRNA comprises a locked nucleic acid (LNA). A locked nucleic acid is a nucleotide having a modified ribose moiety in which the ribose moiety comprises an extra bridge connecting the 2' and 4' carbons. This structure effectively "locks" the ribose in the 3'-endo structural conformation. In other embodiments, the mRNA backbone comprises deoxyribose. In some embodiments, the mRNA comprises a 2'-fluoro-deoxyribose.

In some embodiments, the mRNA is a circular mRNA. A circular mRNA is an mRNA with no 5' terminal nucleotide or 3' terminal nucleotide. Every nucleotide in a circular mRNA is covalently bonded to both 1) a 5' adjacent nucleotide; and 2) a 3' adjacent nucleotide. In a circular mRNA with a nucleic acid sequence comprising every nucleotide of the circular mRNA in 5'-to-3' order, the last nucleotide of the nucleic acid sequence is covalently bonded to the first nucleotide of the nucleic acid sequence. In some embodiments of circular mRNAs with a 5' cap region, a 5' UTR, a 3' UTR, and a poly-A region, the poly-A region is 3' to (downstream from) the 3' UTR and 5' to (upstream of) the 5' cap region.

In some embodiments of the modified mRNAs provided herein, 1% to 90% of the nucleotides of the poly-A region or 5' cap region are modified nucleotides. In some embodiments, at least 1%, at least 2%, at least 3%, at least 4%, at least 5%, at least 6%, at least 7%, at least 8%, at least 9%, at least 10%, at least 12%, at least 14%, at least 16%, at least 18%, at least 20%, at least 25%, at least 30%, at least 35%, at least 40%, at least 45%, or at least 50% of the nucleotides of the poly-A region or 5' cap region are modified nucleotides.

In some embodiments of the modified mRNAs provided herein, 3 or more of the last 10 nucleotides of the poly-A region are modified nucleotides. In some embodiments of the modified mRNAs provided herein, 3 or more of the last 15 nucleotides of the poly-A region are modified nucleotides. In some embodiments of the modified mRNAs provided herein, 3 or more of the last 20 nucleotides of the poly-A region are modified nucleotides. In some embodiments of the modified mRNAs provided herein, 3 or more of the last 25 nucleotides of the poly-A region are modified nucleotides. In some embodiments, at least 4, at least 5, at least 6, at least 7, at least 8, at least 9, at least 10, at least 11, at least 12, at least 13, at least 14, at least 15, at least 20, or 25 of the last 10-25 nucleotides of the poly-A region are modified nucleotides.

In some embodiments of the modified mRNAs provided herein, at least 25%, at least 30%, at least 40%, at least 50%, at least 60%, at least 70%, at least 80%, at least 90%, at least 91%, at least 92%, at least 93%, at least 94%, at least 95%, at least 96%, at least 97%, at least 98%, or at least 99% of the nucleotides of the poly-A region are adenosine nucleotides. One or more adenosine nucleotides of the poly-A region may be canonical adenosine nucleotides or modified adenosine nucleotides comprising a different structure from the canonical adenosine nucleotide. Non-limiting examples of modified adenosine nucleotides include N6-isopentenyladenosine (i6A), 2-methyl-thio-N6-isopentenyladenosine (ms2i6A), 2-methylthio-N6-methyladenosine (ms2m6A), N6-(cis-hydroxyisopentenyl)adenosine (io6A), 2-methylthio-N6-(cis-hydroxyisopentenyl)adenosine (ms2io6A), N6-glycinylcarbamoyladenosine (g6A), N6-threonylcarbamoyladenosine (t6A), 2-methylthio-N6-threonyl carbamoyladenosine (ms2t6A), N6-methyl-N6-threonylcarbamoyladenosine (m6t6A), N6-hydroxynorvalylcarbamoyladenosine (hn6A), 2-methylthio-N6-hydroxynorvalyl carbamoyladenosine (ms2hn6A), 2'-O-ribosyladenosine (phosphate) (Ar(p)), N6,N6-dimethyladenosine (m62A), N6,2'-O-dimethyladenosine (m6Am), N6,N6,O-2'-trimethyladenosine (m62Am), 1,2'-O-dimethyladenosine (m1Am), N6-acetyladenosine (ac6A), 2'-thioadenosine (2'SA), 5'-thioadenosine (5'SA), 2'-O-(2-azidoethyl)-adenosine, 2'-azido-adenosine, deoxyadenosine (dA), dideoxyadenosine (ddA), and amino-deoxyadenosine (amino-dA).

In some embodiments of the modified mRNAs provided herein, at least 25%, at least 30%, at least 40%, at least 50%, at least 60%, at least 70%, at least 80%, at least 90%, at least 91%, at least 92%, at least 93%, at least 94%, at least 95%, at least 96%, at least 97%, at least 98%, or at least 99% of the nucleotides of the poly-A region are canonical adenosine nucleotides.

In some embodiments of the modified mRNAs provided herein, the poly-A region and/or 5' cap region comprises 10-25 nucleotides. In some embodiments of the modified mRNAs provided herein, the poly-A region and/or 5' cap region comprises 10-15 nucleotides, 15-20 nucleotides, or 20-25 nucleotides. In some embodiments of the modified mRNAs provided herein, the poly-A region and/or 5' cap region comprises 25-500 nucleotides. In some embodiments, the poly-A region and/or 5' cap region comprises at least 25, at least 30, at least 50, at least 100, at least 150, or at least 200 nucleotides. In some embodiments, the poly-A region and/or 5' cap region comprises at least 30, at least 40, at least 50, at least 60, at least 70, at least 80, at least 90, at least 100, at least 110, at least 120, at least 130, at least 140, at least 150, at least 160, at least 170, at least 180, at least 190, at least 200, at least 210, at least 220, at least 230, at least 240, at least 250, at least 260, at least 270, at least 280, at least 290, or at least 300 nucleotides. In some embodiments, the poly-A region and/or 5' cap region comprises about 200 to about 300 nucleotides. In some embodiments, poly-A region and/or 5' cap region comprises about 250 nucleotides.

In some embodiments, the poly-A region and/or 5' cap region comprises at least 3, at least 4, at least 5 phosphorothioates, or least 6 phosphorothioates. In some embodiments, the poly-A region of the mRNA comprises at least 3 phosphorothioates, and does not comprise a 3' terminal hydroxyl. In some embodiments, the poly-A region of the mRNA comprises at least 3 guanine nucleotides and at least 3 phosphorothioates, and does not comprise a 3' terminal hydroxyl. In some embodiments, the poly-A region of the mRNA comprises at least 3 deoxyribose sugars, and does not comprise a 3' terminal hydroxyl. In some embodiments, the poly-A region and/or 5' cap region comprises at least 20 deoxyribose sugars. In some embodiments, the poly-A region of the mRNA comprises at least 20 deoxyribose sugars, and does not comprise a 3' terminal hydroxyl. In some embodiments, the poly-A region of the mRNA comprises at least 3 copies of a G-quadruplex sequence, and does not comprise a 3' terminal hydroxyl. In some embodiments, the poly-A region of the mRNA comprises at least 6 phosphorothioates, and does not comprise a 3' terminal hydroxyl. In some embodiments, the poly-A region of the mRNA comprises at least 6 sequential phosphorothioates, and does not comprise a 3' terminal hydroxyl. In some embodiments, the poly-A region of the mRNA comprises at least 6 phosphorothioates and 3 guanine nucleosides, and does not comprise a 3' terminal hydroxyl. In some embodiments, the poly-A region of the mRNA comprises at least 3 copies of a G-quadruplex sequence and at least 6 phosphorothioates, and does not comprise a 3' terminal hydroxyl. In some embodiments, the poly-A region of the mRNA comprises at least 3 copies of a telomeric repeat sequence, and at least 6 phosphorothioates, and does not comprise a 3' terminal hydroxyl. In some embodiments, the 3' terminal nucleotide that does not comprise a 3' terminal hydroxyl is a dideoxycytidine or an inverted-deoxythymidine.

In some embodiments, a poly-A region comprises two or more poly-A tails and at least two instances of the poly-A tails are covalently linked to a first linker. In some embodiments, the poly-A region comprises the following structure: -(a first poly-A tail)-[(a first linker)-(a second poly-A tail)n1]n2; wherein each instance of n1 is independently an integer between 1 and 20, inclusive; n2 is an integer between 2 and 10, inclusive; and the first poly-A tail is covalently linked to the 3' UTR of the mRNA.

In some embodiments, the poly-A region comprises a brushed oligonucleotide comprising two or more poly-A tails.

In some embodiments, each instance of n1 is 1. In some embodiments, at least one instance of the first linker is covalently linked to an internal nucleotide of the first poly-A tail. In some embodiments, at least one instance of the first linker is covalently linked to a second poly-A tail at a 3' nucleotide of the second poly-A tail. In some embodiments, at least one instance of the first linker comprises at least one moiety formed by reacting two orthogonal click-chemistry handles. Any click chemistry reaction known in the art can be used. Click chemistry is a chemical approach introduced by Sharpless in 2001 and describes chemistry tailored to generate substances quickly and reliably by joining small units together. See, *e.g.,* Kolb, Finn and Sharpless Angewandte Chemie International Edition (2001) 40: 2004-2021; Evans, Australian Journal of Chemistry (2007) 60: 384-395). Exemplary click chemistry reactions include formation of esters, thioesters, amides (*e.g*., such as peptide coupling) from activated acids or acyl halides; nucleophilic displacement reactions (*e.g*., such as nucleophilic displacement of a halide or ring opening of strained ring systems); azide-alkyne Huisgen cycloaddition; thiol-yne addition; imine formation; Michael additions (*e.g*., maleimide addition); and Diels-Alder reactions (*e.g*., tetrazine [4 + 2] cycloaddition). Examples of click chemistry reactions can be found in, *e.g.,* Kolb, H. C.; Finn, M. G. and Sharpless, K. B. Angew. Chem. Int. Ed. 2001, 40, 2004-2021. Kolb, H. C. and Shrapless, K. B. Drug Disc. Today, 2003, 8, 112-1137; Rostovtsev, V. V.; Green L. G.; Fokin, V. V. and Shrapless, K. B. Angew. Chem. Int. Ed. 2002, 41, 2596-2599; Tomoe, C. W.; Christensen, C. and Meldal, M. J. Org. Chem. 2002, 67, 3057-3064. Wang, Q. et al. J. Am. Chem. Soc. 2003, 125, 3192-3193; Lee, L. V. et al. J. Am. Chem. Soc. 2003 125, 9588-9589; Lewis, W. G. et al. Angew. Chem. Int. Ed. 2002, 41, 1053- 41057; Manetsch, R. et al., J. Am. Chem. Soc. 2004, 126, 12809- 12818; Mocharla, V. P. et al. Angew. Chem., Int. Ed. 2005, 44, 116-120; each of which is incorporated by reference in their entirety. In certain embodiments, each instance of the click-chemistry handles is a monovalent moiety. In certain embodiments, two orthogonal click-chemistry handles (a first click-chemistry handle and a second click-chemistry handle) react via a click-chemistry reaction to form a divalent moiety. In certain embodiments, at least one instance of the first click-chemistry handle comprises C≡C or C=C. In certain embodiments, at least one instance of the first click-chemistry handle comprises C≡CH, C=CH, CH=CH, C=CH₂, or CH=CH₂. In certain embodiments, at least one instance of the first click-chemistry handle is -C≡CH, substituted or unsubstituted cyclooctynyl optionally fused independently with one or more instances of substituted or unsubstituted phenyl, substituted or unsubstituted cyclopropenyl, substituted or unsubstituted cyclobutenyl, substituted or unsubstituted trans-cyclooctenyl optionally fused independently with one or more instances of substituted or unsubstituted phenyl, or substituted or unsubstituted 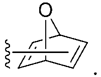 In certain embodiments, at least one instance of the first click-chemistry handle comprises -C≡CH. In certain embodiments, at least one instance of the first click-chemistry handle comprises 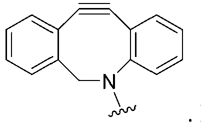 In certain embodiments, at least one instance of the first click-chemistry handle comprises trans-cyclooctenyl, *e.g*., 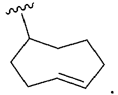 In certain embodiments, at least one instance of the second click-chemistry handle comprises -N₃. In certain embodiments, at least one instance of the second click-chemistry handle comprises 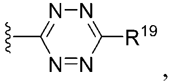 wherein R¹⁹ is H, halogen, unsubstituted C₁₋₆ alkyl, or -O-(unsubstituted C₁₋₆ alkyl). In certain embodiments, R¹⁹ is -CH₃. In some embodiments, at least one moiety formed by reacting two orthogonal click-chemistry handles is of the formula:

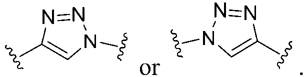

In some embodiments, at least one moiety formed by reacting two orthogonal click-chemistry handles is of the formula: 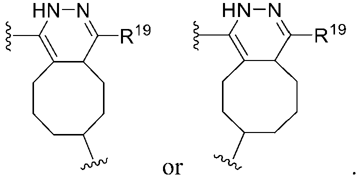 In some embodiments, at least one moiety formed by reacting two orthogonal click-chemistry handles is of the formula: 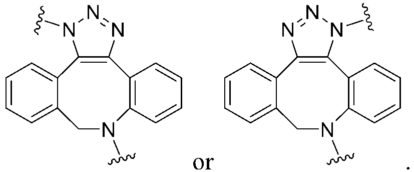 In some embodiments, each instance of the first linker is independently substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, or substituted or unsubstituted, C₁₋₂₀₀ heteroalkynylene. In some embodiments, one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with substituted or unsubstituted carbocyclylene, substituted or unsubstituted heterocyclylene, substituted or unsubstituted arylene, or substituted or unsubstituted heteroarylene. In some embodiments, one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with 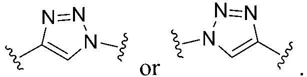 In some embodiments, one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with 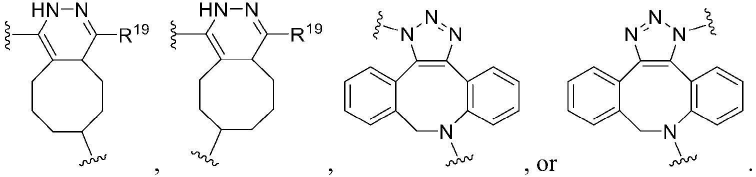 In some embodiments, at least one instance of the first linker is of the formula: 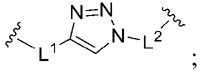 wherein each instance of L¹ is independently substituted or unsubstituted, C₁₋₁₉₉ alkylene or substituted or unsubstituted, C₁₋₁₉₉ heteroalkylene; and each instance of L² is independently substituted or unsubstituted, C₁₋₁₉₉ alkylene or substituted or unsubstituted, C₁₋₁₉₉ heteroalkylene. In some embodiments, at least one instance of the first linker is of the formula:

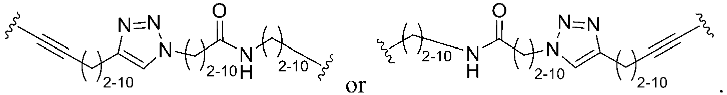

In some embodiments, the first poly-A tail comprises a nucleotide sequence set forth as: 5'-rArArArArArAdU(a1)rArArArArArArArArArArArAdU(a1)rArArArArArArArArArArArA dU(a1)ddC-3' (SEQ ID NO: 1); and at least one instance of the second poly-A tails comprises a nucleotide sequence set forth as: 5'-rArArArArArArArArArArArArArArArArArArArArArA rArArA*rA*rA* rA*rA*rA*(b1)-3' (SEQ ID NO: 2); wherein each instance of the first linker is independently 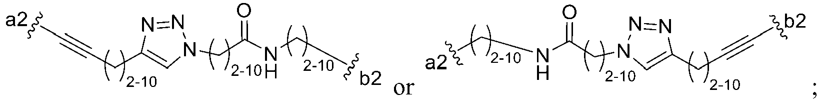 wherein "rA" represents an adenosine ribonucleotide; "dU(a1)" represents a modified uridine deoxyribonucleotide; "ddC" represents a cytosine dideoxyribonucleotide; "*" represents a phosphorothioate linkage; the a1 of each instance of dU(a1) represents an attachment point on the uridine of the dU(a1); the b1 of rA*(b1) represents an attachment point on the * of the rA*(b1); each instance of a1 is attached to an instance of a2; and each instance of b1 is attached to an instance of b2. In some embodiments, the modified uridine deoxyribonucleotide is 5-Octadiynyl deoxyuridine, which is shown in the structure:

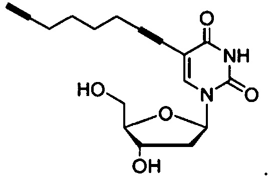

In some embodiments, the poly-A region comprises a dendrimer comprising two or more poly-A tails.

In some embodiments, each instance of n1 is independently an integer between 2 and 20, inclusive. In some embodiments, at least one instance of the first linker comprises a dendrimer. In some embodiments, at least one instance of the dendrimer is a polyamidoamine (PAMAM) dendrimer. In some embodiments, at least one instance of the PAMAM dendrimer is of the formula: 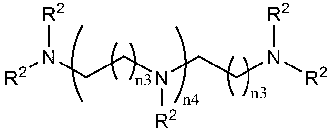 wherein each instance of n3 is independently an integer between 1 and 10, inclusive; each instance of n4 is independently an integer between 0 and 10, inclusive; each instance of R² is independently hydrogen or 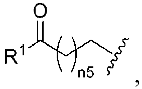 provided that at least three instances of R² is 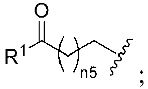 and each instance of R¹ is substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, or substituted or unsubstituted, C₁₋₁₀ heteroalkynylene. In some embodiments, one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with substituted or unsubstituted carbocyclylene, substituted or unsubstituted heterocyclylene, substituted or unsubstituted arylene, or substituted or unsubstituted heteroarylene; and each instance of n5 is independently an integer between 0 and 10, inclusive. In some embodiments, at least one instance of the PAMAM dendrimer is of the following formula:

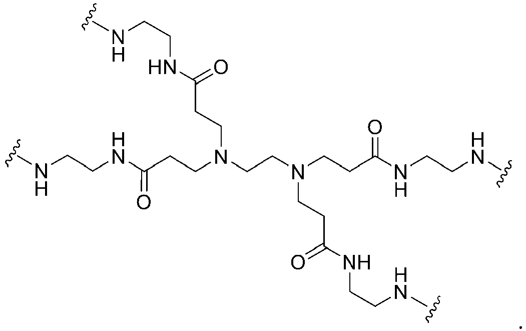

In some embodiments, one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with 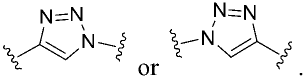 In some embodiments, one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with 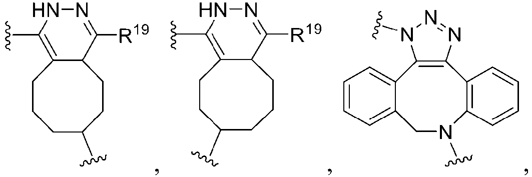 or 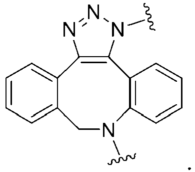 In some embodiments, at least one instance of the first linker is of the following formula:

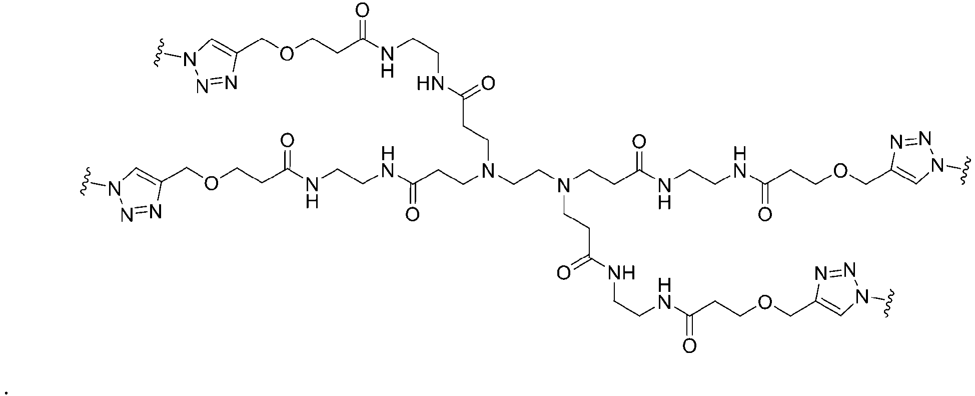

In some embodiments, the first poly-A tail and the second poly-A tails comprise a nucleotide sequence set forth as:
5'-rArArArArArArArArArArArArArArArArArArArArArArArArA*rA*rA* rA*rA*rA* /AzideN/-3' (SEQ ID NO: 3); wherein "AzideN" represents a 3' azide moiety and "*" represents a phosphorothioate linkage.

In some embodiments, at least one instance of the first linker is covalently linked to a 3' nucleotide of the first poly-A tail. In some embodiments, at least one instance of the first linker is covalently linked to a 3' nucleotide of the at least one instance of the second poly-A tails.

In some embodiments, the 5' cap region comprises two or more 5' caps, and at least two instances of the 5' caps are covalently linked by a second linker. In some embodiments, the 5' cap region comprises the following structure: -(a first 5' cap)-[(a second linker)-(a second 5' cap)m1]m2; wherein each instance of m1 is independently an integer between 1 and 20, inclusive; m2 is an integer between 2 and 10, inclusive; the first 5' cap is covalently linked to the 5' UTR; and the second linker is covalently linked to the first 5' cap and to the second 5' cap.

In some embodiments, the 5' cap region comprises a brushed oligonucleotide comprising two or more 5' caps.

In some embodiments, each instance of m1 is 1. In some embodiments, at least one instance of the second linker is covalently linked to an internal nucleotide of the first 5' cap. In some embodiments, at least one instance of the second linker is covalently linked to a second 5' cap at a 3' nucleotide of the second 5' cap. In some embodiments, at least one instance of the second linker comprises at least one moiety formed by reacting two orthogonal click-chemistry handles. In certain embodiments, the click-chemistry handles are as described herein. In some embodiments, each instance of the second linker is independently substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, or substituted or unsubstituted, C₁₋₂₀₀ heteroalkynylene. In some embodiments, one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with substituted or unsubstituted carbocyclylene, substituted or unsubstituted heterocyclylene, substituted or unsubstituted arylene, or substituted or unsubstituted heteroarylene. In some embodiments, one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with 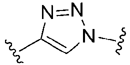 or 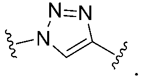 In some embodiments, one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with 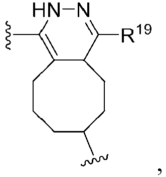 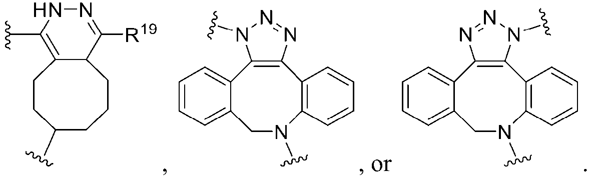 In some embodiments, at least one instance of the second linker is of the formula: 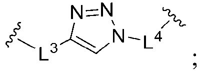 wherein each instance of L³ is independently substituted or unsubstituted, C₁₋₁₉₉ alkylene or substituted or unsubstituted, C₁₋₁₉₉ heteroalkylene; and each instance of L⁴ is independently substituted or unsubstituted, C₁₋₁₉₉ alkylene or substituted or unsubstituted, C₁₋₁₉₉ heteroalkylene. In some embodiments, at least one instance of the second linker is of the formula: 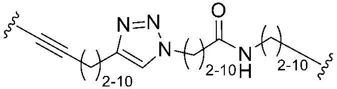 or

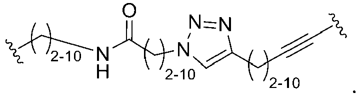

In some embodiments, the first 5' cap comprises a nucleotide sequence set forth as:
5'-/Cap/rGrGrGrArArAdU(c1)rArArGrArGrArGrArArArArGrArArGrArGdU(c1)rArArGrArA rGrArArAdU(cl)rA-3' (SEQ ID NO: 4); and the second 5' cap comprises a nucleotide sequence set forth as:
5'-/Cap/rGrGrGrArGrArCrTrGrCrCrArCrCrA*rA*rA*rA*rA*rA*(dl)-3' (SEQ ID NO: 5);
wherein
each instance of the second linker is independently 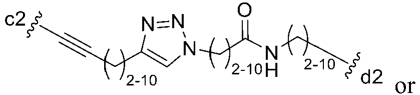 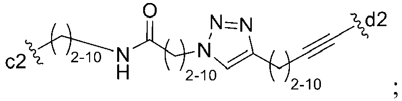 wherein "*" represents a phosphorothioate linkage;
"Cap" represents a 5' cap; the c1 of each instance of dU(c1) represents an attachment point on the U of the dU(c1); the d1 of rA*(d1) represents an attachment point on the * of the rA*(d1); each instance of c1 is attached to an instance of c2; and each instance of d1 is attached to an instance of d2.

In some embodiments, the 5' cap region comprises a dendrimer comprising two or more 5' caps.

In some embodiments, each instance of m1 is independently an integer between 2 and 20, inclusive. In some embodiments, at least one instance of the second linker comprises a dendrimer. In some embodiments, at least one instance of the dendrimer is a polyamidoamine (PAMAM) dendrimer. In some embodiments, at least one instance of the PAMAM dendrimer is of the formula: 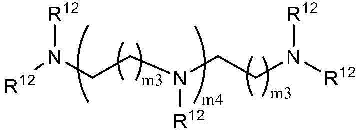 wherein each instance of m3 is independently an integer between 1 and 10, inclusive; each instance of m4 is independently an integer between 0 and 10, inclusive; each instance of R¹² is independently hydrogen or 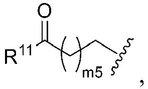 provided that at least three instances of R¹² is 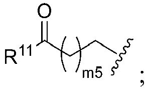 and each instance of R¹¹ is substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, or substituted or unsubstituted, C₁₋₁₀ heteroalkynylene. In some embodiments, one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with substituted or unsubstituted carbocyclylene, substituted or unsubstituted heterocyclylene, substituted or unsubstituted arylene, or substituted or unsubstituted heteroarylene; and each instance of m5 is independently an integer between 0 and 10, inclusive. In some embodiments, at least one instance of the PAMAM dendrimer comprises the following formula:

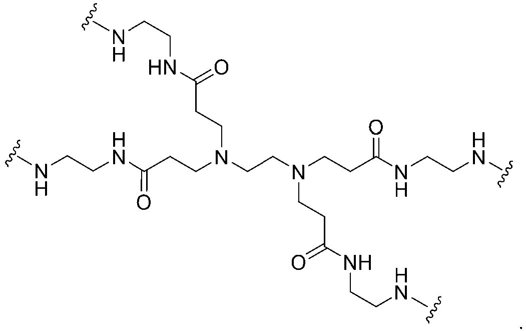

In some embodiments, one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with 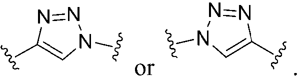 In some embodiments, one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with 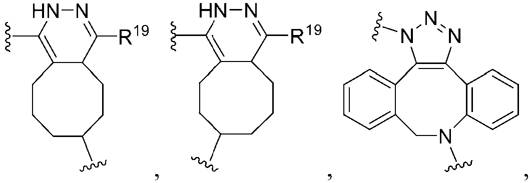 or 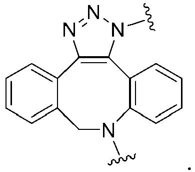 In some embodiments, at least one instance of the first linker is of the following formula:

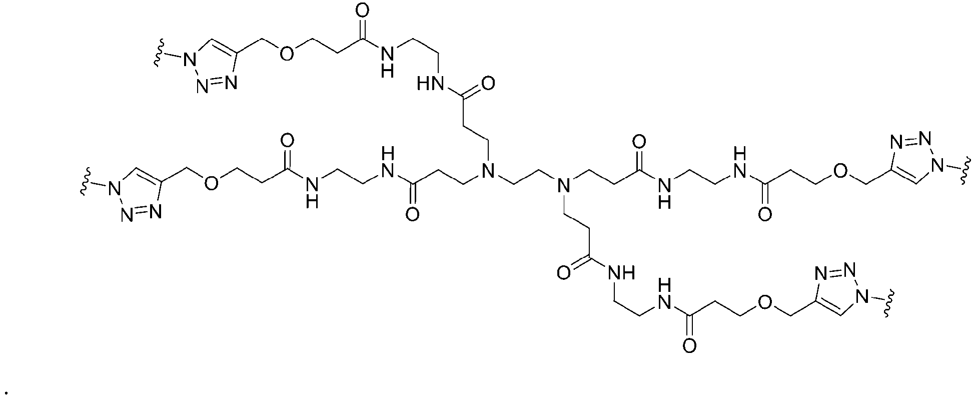

In some embodiments, the first 5' cap comprises a nucleotide sequence set forth as:
/5AzideN/rArArA rArA-3'; and the second 5' cap comprises a nucleotide sequence set forth as: 5'-/Cap/rGrGrGrArGrArCrTrGrCrCrArCrCrA*rA*rA*rA*rA*rA*/AzideN/-3' (SEQ ID NO: 6); wherein "rA" represents an adenosine ribonucleotide; "rT" represents a thymidine ribonucleotide; "rC" represents a cytidine ribonucleotide; "rG" represents a guanosine ribonucleotide; "dU(c1)" represents a modified uridine deoxyribonucleotide; "5AzideN" represents a 5' azide moiety, "3AzideN" represents a 3' azide moiety, "*" represents a phosphorothioate linkage, and "Cap" represents a 5' cap.

In some embodiments, at least one instance of the second linker is covalently linked to a 3' nucleotide of the first 5' cap. In some embodiments, at least one instance of the second linker is covalently linked to a 3' nucleotide of the at least one instance of the second 5' caps.

### Methods of producing modified mRNAs

In some aspects, the present disclosure provides methods of producing modified mRNAs, comprising ligating an RNA comprising an open reading frame encoding a protein to a tailing nucleic acid comprising two or more poly-A tails, and/or to a capping nucleic acid comprising two or more 5' caps, in the presence of a ligase, whereby the ligase forms a covalent bond between the 3' nucleotide of the RNA and the 5' nucleotide of the tailing nucleic acid, or the 3' nucleotide of the capping nucleic acid and the 5' nucleic acid of the RNA, to produce a modified RNA (e.g., a modified mRNA). In some embodiments, a tailing nucleic acid comprises a poly-A region that is described herein, and a capping nucleic acid comprises a 5' cap region that is described herein. When a ligase forms a covalent bond between two linear nucleic acids, a new nucleic acid is produced, with the produced nucleic acid comprising the nucleic acid sequences of both nucleic acids. Ligation of the 3' terminal nucleotide of a first nucleic acid to the 5' terminal nucleotide of a second nucleic acid produces a third nucleic acid, with the third nucleic acid comprising the sequence of the first nucleic acid and the second nucleic acid, and the second nucleic acid sequence being 3' to (downstream of) the first nucleic acid sequence. Ligation by an RNA ligase occurs in several steps. First, an amino (-NH₂) group of an amino acid *(e.g.,* a lysine) of the ligase bonds to a phosphate group of adenosine triphosphate (ATP), such that an adenosine monophosphate (AMP) group is bound to the RNA ligase. Second, a 5' terminal phosphate of the second nucleic acid displaces the phosphate of the RNA ligase-bound AMP. Finally, an oxygen of the 3' terminal hydroxyl group of the first nucleic acid binds to the phosphorus atom of the 5' terminal phosphate of the second nucleic acid. This final step forms a phosphodiester bond between terminal nucleotides of the nucleic acids, thereby forming a single nucleic acid with a continuous sugar-phosphate backbone. In some embodiments, the ligase is an RNA ligase. In some embodiments, the RNA ligase is a T4 RNA ligase.

In some embodiments of the methods of producing modified mRNAs provided herein, the RNA to which a tailing nucleic acid and/or capping nucleic acid are ligated is synthesized by *in vitro* transcription (IVT). IVT is a process in which an RNA, such as a precursor mRNA (pre-mRNA) or mRNA, is generated through transcription of a DNA template by an RNA polymerase. Generally, the DNA template comprises a promoter, such as a bacteriophage promoter, that is upstream of the DNA sequence to be transcribed. The RNA polymerase binds to the promoter, and begins transcription of the DNA sequence, producing an RNA transcript with a nucleic acid sequence that is present in the template, with the exception that thymidine (T) nucleotides in the DNA sequence are replaced with uracil (U) nucleotides in the RNA sequence. The RNA transcript produced by IVT may be modified prior to ligation of a tailing nucleic acid, such as by the addition of a 5' cap, cleavage of one or more nucleotides from the RNA, or polyadenylation to extend the poly-A region. In some embodiments, the DNA template comprises a poly-A region, such that IVT produces an mRNA with a poly-A region. See, *e.g.,* Becker et al. Methods Mol Biol., 2011. 703:29-41.

In some embodiments of the methods of producing modified mRNAs provided herein, the 3' nucleotide of the RNA comprises a 3' terminal hydroxyl group, and the 5' nucleotide of the tailing nucleic acid comprises a 5' terminal phosphate group. In some embodiments of the methods of producing modified mRNAs provided herein, the 3' nucleotide of the capping nucleic acid comprises a 3' terminal hydroxyl group, and the 5' nucleotide of the RNA comprises a 5' terminal phosphate group. The combination of a 3' terminal hydroxyl group and a 5' terminal phosphate group allows for efficient ligation of the two nucleic acids. In some embodiments, the RNA does not comprise a 5' terminal phosphate group. An RNA may lack a 5' terminal phosphate group due to the addition of a 5' cap or another chemical modification. A 5' terminal phosphate may also be removed from an RNA by a phosphatase enzyme to produce an RNA that lacks a 5' terminal phosphate. Lack of a 5' terminal phosphate group on the RNA prevents an RNA ligase from ligating multiple copies of an mRNA together. In some embodiments, the tailing nucleic acid does not comprise a 3' terminal hydroxyl group. An RNA may lack a 3' terminal hydroxyl group if the last nucleotide of the tailing nucleic acid comprises a modified nucleotide that does not contain a 3' hydroxyl group, such as a dideoxyadenosine, dideoxycytidine, dideoxyguanosine, dideoxythymidine, or inverted-deoxythymidine. Lack of a 3' terminal hydroxyl group on the tailing nucleic acid prevents an RNA ligase from ligating multiple tailing nucleic acids together. In some embodiments, the 5' nucleotide of the RNA does not comprise a 5' terminal phosphate group; the 3' nucleotide of the RNA comprises a 3' terminal hydroxyl group; the 5' nucleotide of the tailing nucleic acid comprises a 5' terminal phosphate group; and the 3' nucleotide of the tailing nucleic acid does not comprise a 3' terminal hydroxyl group. In some embodiments, the tailing nucleic acid and/or capping nucleic acid comprises at least 3, at least 4, at least 5, or at least 6 phosphorothioates. In some embodiments, the tailing nucleic acid comprises at least 3, at least 4, at least 5, or at least 6 phosphorothioates, and does not comprise a 3' terminal hydroxyl. In some embodiments, the tailing nucleic acid comprises at least 3 phosphorothioates, and does not comprise a 3' terminal hydroxyl. In some embodiments, the tailing nucleic acid comprises at least 3 guanine nucleotides and at least 3 phosphorothioates, and does not comprise a 3' terminal hydroxyl. In some embodiments, the tailing nucleic acid comprises at least 3 deoxyribose sugars, and does not comprise a 3' terminal hydroxyl. In some embodiments, the tailing nucleic acid and/or capping nucleic acid comprises at least 20 deoxyribose sugars. In some embodiments, the tailing nucleic acid comprises at least 20 deoxyribose sugars, and does not comprise a 3' terminal hydroxyl. In some embodiments, the tailing nucleic acid comprises at least 3 copies of a G-quadruplex sequence, and does not comprise a 3' terminal hydroxyl. In some embodiments, the tailing nucleic acid comprises at least 6 phosphorothioates, and does not comprise a 3' terminal hydroxyl. In some embodiments, the tailing nucleic acid comprises at least 6 sequential phosphorothioates, and does not comprise a 3' terminal hydroxyl. In some embodiments, the tailing nucleic acid comprises at least 6 phosphorothioates and 3 guanine nucleosides, and does not comprise a 3' terminal hydroxyl. In some embodiments, the tailing nucleic acid comprises at least 3 copies of a G-quadruplex sequence and at least 6 phosphorothioates, and does not comprise a 3' terminal hydroxyl. In some embodiments, the tailing nucleic acid comprises at least 3 copies of a telomeric repeat sequence, and at least 6 phosphorothioates, and does not comprise a 3' terminal hydroxyl. In some embodiments, the 3' terminal nucleotide that does not comprise a 3' terminal hydroxyl is a dideoxycytidine or an inverted-deoxythymidine. In some embodiments, the ligase used to ligate the tailing nucleic acid to the RNA is an RNA ligase. In some embodiments, the RNA ligase is a T4 RNA ligase. In some embodiments, the T4 RNA ligase is a T4 RNA ligase 1. In some embodiments, the T4 RNA ligase is a T4 RNA ligase 2.

In some embodiments of the methods of producing modified mRNAs provided herein, the 5' nucleotide of the RNA does not comprise a 5' terminal hydroxyl group, the 3' nucleotide of the RNA comprises a 3' terminal phosphate group, the 5' nucleotide of the tailing nucleic acid comprises a 5' terminal hydroxyl group, the 3' nucleotide of the tailing nucleic acid does not comprise a 3' terminal phosphate group, and the RNA ligase is an RtcB ligase, which ligates a first nucleotide comprising a 3' terminal phosphate group to a second nucleotide comprising a 5' terminal hydroxyl group.

Some embodiments of the methods of making modified mRNAs provided herein further comprise producing a circular mRNA. After a linear modified mRNA is produced by ligating an RNA and a tailing nucleic acid, circularization of the modified mRNA comprises several additional steps. First, a 5' terminal phosphate is introduced onto the first nucleotide of the modified mRNA, a process known as phosphorylation. In some embodiments, the 5' terminal phosphate is introduced by a kinase. A "kinase" refers to an enzyme that introduces a phosphate group to a molecule, forming a covalent bond between the phosphate group and the molecule, in a process referred to as "phosphorylation." Second, the modified mRNA is manipulated to produce a modified mRNA with a 3' terminal hydroxyl group. In some embodiments, the modified mRNA is manipulated by cleaving one or more of the last nucleotides of the modified mRNA, to produce a modified mRNA with a 3' terminal hydroxyl group. In some embodiments, the modified mRNA is cleaved by a restriction enzyme, ribozyme, or endoribonuclease. In some embodiments, cleavage of one or more last nucleotides of the modified mRNA occurs before phosphorylation of the first nucleotide of the modified mRNA. In some embodiments, cleavage occurs after phosphorylation. A modified mRNA comprising a terminal phosphate group at one end and a terminal hydroxyl group at the other end can be circularized by ligation of both terminal nucleotides. An RNA ligase that ligates terminal nucleotides of a linear nucleic acid to produce a circular nucleic acid may be called a "circularizing ligase." In some embodiments, the circularizing ligase is an RNA ligase. In some embodiments, the circularizing ligase is a SplintR ligase. In some embodiments, the circularizing ligase is a T4 RNA ligase. In some embodiments, the circularizing ligase is a T4 RNA ligase 1. In some embodiments, the circularizing ligase is a T4 RNA ligase 2. In some embodiments, the modified mRNA comprises a 5' terminal hydroxyl group and a 3' terminal phosphate group, and the circularizing ligase is RtcB ligase, which is capable of ligating nucleotides with a 3' terminal phosphate and 5' terminal hydroxyl group. For ligation to occur, the 5' and 3' terminal nucleotides of the modified mRNA must be close enough for the RNA ligase to form a bond between both nucleotides. Methods of placing both nucleotides of a linear nucleic acid close enough for ligation to occur, and of circularizing an RNA, are generally known in the art (see, *e.g.,* Petkovic et al., Nucleic Acids Res., 2015. 43(4):2454-2465). In some embodiments, the modified mRNA is incubated with a scaffold nucleic acid, which is capable of hybridizing (hydrogen bonding) to the modified mRNA so that the modified mRNA forms a circular secondary structure when hybridized (bound) to the scaffold nucleic acid.

When an mRNA forms a circular secondary structure, the 5' and 3' terminal nucleotides are in close physical proximity, which is required for an RNA ligase to form a covalent bond between them. In some embodiments of methods of circularizing an mRNA, one or more of the last nucleotides of the mRNA are bound to a first hybridization sequence in the scaffold nucleic acid, and one or more of the first nucleotides of the mRNA are bound to a second hybridization sequence in the scaffold nucleic acid that is 3' to (downstream of) the first hybridization sequence. In some embodiments, the first hybridization sequence comprises 5 or more nucleotides, and the first hybridization sequence is complementary to at least the first five (5) nucleotides of the modified mRNA. In some embodiments, the first hybridization sequence comprises 10 or more, 15 or more, 20 or more, 25 or more, 30 or more, 35 or more, 40 or more, 45 or more, or 50 or more nucleotides, and at least 90%, at least 95%, at least 96%, at least 97%, at least 98%, at least 99%, or up to 100% of the nucleotides of the first hybridization sequence are complementary are complementary to the last *N* nucleotides of the modified mRNA, where *N* is the length of the first hybridization sequence. In some embodiments, the second hybridization sequence comprises 5 or more nucleotides, and the second hybridization sequence is complementary to at least the last five (5) nucleotides of the modified mRNA. In some embodiments, the second hybridization sequence comprises 10 or more, 15 or more, 20 or more, 25 or more, 30 or more, 35 or more, 40 or more, 45 or more, or 50 or more nucleotides, and at least 90%, at least 95%, at least 96%, at least 97%, at least 98%, at least 99%, or up to 100% of the nucleotides of the second hybridization sequence are complementary are complementary to the last *N* nucleotides of the modified mRNA, where *N* is the length of the second hybridization sequence. In some embodiments, at least the first five (5) nucleotides of the modified mRNA hybridize with the first hybridization sequence. In some embodiments, at least the last five (5) nucleotides of the modified mRNA hybridize with the second hybridization sequence. In some embodiments, at least the first five (5) nucleotides of the modified mRNA hybridize with the first hybridization sequence, and at least the last five (5) nucleotides of the modified mRNA hybridize with the second hybridization sequence. In some embodiments, the last nucleotide of the first hybridization sequence and the first nucleotide of the second hybridization sequence are adjacent in the scaffold nucleic acid, and are not separated by any other nucleotides.

In some embodiments of the methods of producing circular mRNAs provided herein, a scaffold nucleic acid is not used to promote the formation of a circular secondary structure by the modified mRNA. Instead, the modified mRNA comprises a first hybridization sequence at the 5' end that is complementary to a second hybridization sequence at the 3' end. In some embodiments, each hybridization sequence comprises at least five (5) nucleotides. In some embodiments, each hybridization sequence comprises at least 10, at least 15, at least 20, at least 25, at least 30, at least 35, at least 40, at least 45, or at least 50 nucleotides.

In some embodiments of the methods of producing circular mRNAs provided herein, the modified mRNA is not circularized through the use of a scaffold nucleic acid and circularizing ligase, but rather is circularized by a ribozyme, a nucleic acid that catalyzes a reaction, such as the formation of a covalent bond between two nucleotides. In some embodiments, prior to circularization, the modified mRNA comprises a 3' intron that is 5' to (upstream of) the 5' UTR of the mRNA, and a 5' intron that is 3' to (downstream of) the poly-A region and/or one or more structural sequences of the mRNA. Ribozymes and other enzymes that catalyze splicing of pre-mRNA to remove introns can catalyze the formation of a covalent bond between the nucleotide that is 5' to the 5' intron and the nucleotide that is 3' to 3' intron, resulting in the formation of a circular mRNA. See, *e.g.,* Wesselhoeft et al., Nat Commun. 2018. 9:2629.

In some embodiments of the methods of producing circular mRNAs provided herein, the modified mRNA is not circularized through the use of a scaffold nucleic, but rather is circularized through the use of complementary sequences that promote the formation of a secondary structure by the mRNA that places the 5' and 3' terminal nucleotides of the mRNA in close proximity. In some embodiments, prior to circularization the modified mRNA comprises (i) a first self-hybridization sequence that is 5' to the open reading frame; (ii) a second self-hybridization sequence that is 3' to the open reading frame; (iii) a first non-hybridization sequence that is 5' to the first self-hybridization sequence; and (iv) a second non-hybridization sequence that is 3' to the second self-hybridization sequence. The first and second self-hybridization sequences are capable of hybridizing with each other, but the first and second self-hybridization sequences are not capable of hybridizing with each other. In some embodiments, hybridization of the first and second self-hybridization sequences forms a secondary structure in which the 5' terminal nucleotide and the 3' terminal nucleotide of the modified mRNA are separated by a distance of less than 100 Å. In some embodiments, the 5' terminal nucleotide and the 3' terminal nucleotide are separated by a distance of less than 90 Å, less than 80 Å, less than 70 Å, less than 60 Å, less than 50 Å, less than 40 Å, less than 30 Å, less than 20 Å, or less than 10 Å See, *e*.*g*., Carmona, Ellese Marie. 2019. Circular RNA: Design Criteria for Optimal Therapeutical Utility. Doctoral dissertation, Harvard University, Graduate School of Arts & Sciences; Petkovic et al. Nucleic Acids Res., 2015. 43(4):2454-2465; and WO 2020/237227.

In some embodiments of the modified mRNAs produced by the methods provided herein, the poly-A region of the modified mRNA comprises at least one modified nucleotide. In some embodiments, the tailing nucleic acid comprises at least one modified nucleotide. In some embodiments, at least one modified nucleotide comprises a modified nucleobase. In some embodiments, at least one modified nucleotide comprises a modified sugar. In some embodiments, at least one modified nucleotide comprises a modified phosphate. In some embodiments, at least one modified nucleotide comprises a modified nucleobase selected from the group consisting of: xanthine, allyaminouracil, allyaminothymidine, hypoxanthine, digoxigeninated adenine, digoxigeninated cytosine, digoxigeninated guanine, digoxigeninated uracil, 6-chloropurineriboside, N6-methyladenine, methylpseudouracil, 2-thiocytosine, 2-thiouracil, 5-methyluracil, 4-thiothymidine, 4-thiouracil, 5,6-dihydro-5-methyluracil, 5,6-dihydrouracil, 5-[(3-Indolyl)propionamide-N-allyl]uracil, 5-aminoallylcytosine, 5-aminoallyluracil, 5-bromouracil, 5-bromocytosine, 5-carboxycytosine, 5-carboxymethylesteruracil, 5-carboxyuracil, 5-fluorouracil, 5-formylcytosine, 5-formyluracil, 5-hydroxycytosine, 5-hydroxymethylcytosine, 5-hydroxymethyluracil, 5-hydroxyuracil, 5-iodocytosine, 5-iodouracil, 5-methoxycytosine, 5-methoxyuracil, 5-methylcytosine, 5-methyluracil, 5-propargylaminocytosine, 5-propargylaminouracil, 5-propynylcytosine, 5-propynyluracil, 6-azacytosine, 6-azauracil, 6-chloropurine, 6-thioguanine, 7-deazaadenine, 7-deazaguanine, 7-deaza-7-propargylaminoadenine, 7-deaza-7-propargylaminoguanine, 8-azaadenine, 8-azidoadenine, 8-chloroadenine, 8-oxoadenine, 8-oxoguanine, araadenine, aracytosine, araguanine, arauracil, biotin-16-7-deaza-7-propargylaminoguanine, biotin-16-aminoallylcytosine, biotin-16-aminoallyluracil, cyanine 3-5-propargylaminocytosine, cyanine 3-6-propargylaminouracil, cyanine 3-aminoallylcytosine, cyanine 3-aminoallyluracil, cyanine 5-6-propargylaminocytosine, cyanine 5-6-propargylaminouracil, cyanine 5-aminoallylcytosine, cyanine 5-aminoallyluracil, cyanine 7-aminoallyluracil, dabcyl-5-3-aminoallyluracil, desthiobiotin-16-aminoallyl-uracil, desthiobiotin-6-aminoallylcytosine, isoguanine, N1-ethylpseudouracil, N1-methoxymethylpseudouracil, N1-methyladenine, N1-methylpseudouracil, N1-propylpseudouracil, N2-methylguanine, N4-biotin-OBEA-cytosine, N4-methylcytosine, N6-methyladenine, O6-methylguanine, pseudoisocytosine, pseudouracil, thienocytosine, thienoguanine, thienouracil, xanthosine, 3-deazaadenine, 2,6-diaminoadenine, 2,6-daminoguanine, 5-carboxamide-uracil, 5-ethynyluracil, N6-isopentenyladenine (i6A), 2-methyl-thio-N6-isopentenyladenine (ms2i6A), 2-methylthio-N6-methyladenine (ms2m6A), N6-(cis-hydroxyisopentenyl)adenine (io6A), 2-methylthio-N6-(cis-hydroxyisopentenyl)adenine (ms2io6A), N6-glycinylcarbamoyladenine (g6A), N6-threonylcarbamoyladenine (t6A), 2-methylthio-N6-threonyl carbamoyladenine (ms2t6A), N6-methyl-N6-threonylcarbamoyladenine (m6t6A), N6-hydroxynorvalylcarbamoyladenine (hn6A), 2-methylthio-N6-hydroxynorvalyl carbamoyladenine (ms2hn6A), N6,N6-dimethyladenine (m62A), and N6-acetyladenine (ac6A). In some embodiments, at least one modified nucleotide comprises a modified sugar selected from the group consisting of 2'-thioribose, 2',3'-dideoxyribose, 2'-amino-2'-deoxyribose, 2' deoxyribose, 2'-azido-2'-deoxyribose, 2'-fluoro-2'-deoxyribose, 2'-O-methylribose, 2'-O-methyldeoxyribose, 3'-amino-2',3'-dideoxyribose, 3'-azido-2',3'-dideoxyribose, 3'-deoxyribose, 3'-O-(2-nitrobenzyl)-2'-deoxyribose, 3'-O-methylribose, 5'-aminoribose, 5'-thioribose, 5-nitro-1-indolyl-2'-deoxyribose, 5'-biotin-ribose, 2'-O,4'-C-methylene-linked, 2'-O,4'-C-amino-linked ribose, and 2'-O,4'-C-thio-linked ribose. In some embodiments, at least one modified nucleotide comprises a modified phosphate selected from the group consisting of phosphorothioate (PS), thiophosphate, 5'-O-methylphosphonate, 3'-O-methylphosphonate, 5'-hydroxyphosphonate, hydroxyphosphanate, phosphoroselenoate, selenophosphate, phosphoramidate, carbophosphonate, methylphosphonate, phenylphosphonate, ethylphosphonate, H-phosphonate, guanidinium ring, triazole ring, boranophosphate (BP), methylphosphonate, and guanidinopropyl phosphoramidate. In some embodiments, the poly-A region of the mRNA comprises at least 3, at least 4, or at least 5 phosphorothioates, and does not comprise a 3' terminal hydroxyl. In some embodiments, the poly-A region of the mRNA comprises at least 3 phosphorothioates, and does not comprise a 3' terminal hydroxyl. In some embodiments, the poly-A region of the mRNA comprises at least 3 guanine nucleotides and at least 3 phosphorothioates, and does not comprise a 3' terminal hydroxyl. In some embodiments, the poly-A region of the mRNA comprises at least 3 deoxyribose sugars, and does not comprise a 3' terminal hydroxyl. In some embodiments, the poly-A region of the mRNA comprises at least 20 deoxyribose sugars, and does not comprise a 3' terminal hydroxyl. In some embodiments, the poly-A region of the mRNA comprises at least 3 copies of a G-quadruplex sequence, and does not comprise a 3' terminal hydroxyl. In some embodiments, the poly-A region of the mRNA comprises at least 6 phosphorothioates, and does not comprise a 3' terminal hydroxyl. In some embodiments, the poly-A region of the mRNA comprises at least 6 sequential phosphorothioates, and does not comprise a 3' terminal hydroxyl. In some embodiments, the poly-A region of the mRNA comprises at least 6 phosphorothioates and 3 guanine nucleosides, and does not comprise a 3' terminal hydroxyl. In some embodiments, the poly-A region of the mRNA comprises at least 3 copies of a G-quadruplex sequence and at least 6 phosphorothioates, and does not comprise a 3' terminal hydroxyl. In some embodiments, the poly-A region of the mRNA comprises at least 3 copies of a telomeric repeat sequence, and at least 6 phosphorothioates, and does not comprise a 3' terminal hydroxyl. In some embodiments, the 3' terminal nucleotide that does not comprise a 3' terminal hydroxyl is a dideoxycytidine or an inverted-deoxythymidine.

In some embodiments of the modified mRNAs produced by the methods provided herein, the modified mRNA comprises more than one type of modified nucleotide. In some embodiments, the modified mRNA comprises at least a first modified nucleoside, and a second modified nucleoside that has a different structure from the first modified nucleoside. In some embodiments, the modified mRNA comprises at least a first modified phosphate, and a second modified phosphate that has a different structure from the first modified phosphate. In some embodiments, the modified mRNA comprises a modified nucleoside and a modified nucleoside.

In some embodiments of the modified mRNAs produced by the methods provided herein, 1% to 90% of the nucleotides of the poly-A region are modified nucleotides. In some embodiments, at least 1%, at least 2%, at least 3%, at least 4%, at least 5%, at least 6%, at least 7%, at least 8%, at least 9%, at least 10%, at least 12%, at least 14%, at least 16%, at least 18%, at least 20%, at least 25%, at least 30%, at least 35%, at least 40%, at least 45%, or at least 50% of the nucleotides of the poly-A region are modified nucleotides.

In some embodiments of the modified mRNAs produced by the methods provided herein, 3 or more of the last 10-25 nucleotides of the poly-A region are modified nucleotides. In some embodiments, at least 4, at least 5, at least 6, at least 7, at least 8, at least 9, at least 10, at least 11, at least 12, at least 13, at least 14, at least 15, at least 20, or 25 of the last 25 nucleotides of the poly-A region are modified nucleotides.

In some embodiments of the modified mRNAs produced by the methods provided herein, at least 25%, at least 30%, at least 40%, at least 50%, at least 60%, at least 70%, at least 80%, at least 90%, at least 91%, at least 92%, at least 93%, at least 94%, at least 95%, at least 96%, at least 97%, at least 98%, or at least 99% of the nucleotides of the poly-A region are adenosine nucleotides. One or more adenosine nucleotides of the poly-A region may be canonical adenosine nucleotides or modified adenosine nucleotides comprising a different structure from the canonical adenosine nucleotide.

In some embodiments of the modified mRNAs produced by the methods provided herein, at least 25%, at least 30%, at least 40%, at least 50%, at least 60%, at least 70%, at least 80%, at least 90%, at least 91%, at least 92%, at least 93%, at least 94%, at least 95%, at least 96%, at least 97%, at least 98%, or at least 99% of the nucleotides of the poly-A region are canonical adenosine nucleotides.

In some embodiments of the modified mRNAs produced by the methods provided herein, the poly-A region comprises 10-25 nucleotides. In some embodiments of the modified mRNAs produced by the methods provided herein, the poly-A region comprises 10-15 nucleotides, 15-20 nucleotides, or 20-25 nucleotides. In some embodiments of the modified mRNAs produced by the methods provided herein, the poly-A region comprises 25-500 nucleotides. In some embodiments, the poly-A region comprises at least 25, at least 30, at least 50, at least 100, at least 150, or at least 200 nucleotides. In some embodiments, the poly-A region comprises at least 30, at least 40, at least 50, at least 60, at least 70, at least 80, at least 90, at least 100, at least 110, at least 120, at least 130, at least 140, at least 150, at least 160, at least 170, at least 180, at least 190, at least 200, at least 210, at least 220, at least 230, at least 240, at least 250, at least 260, at least 270, at least 280, at least 290, or at least 300 nucleotides. In some embodiments, the poly-A region comprises about 200 to about 300 nucleotides. In some embodiments, the poly-A region comprises about 250 nucleotides.

In some embodiments of the methods of producing modified mRNAs provided herein, prior to the ligation of a tailing nucleic acid, the RNA comprises an open reading frame and a poly-A region prior to ligation of a tailing nucleic acid. In some embodiments, prior to ligation of a tailing nucleic acid, the poly-A region of the RNA comprises 10-25 nucleotides. In some embodiments, prior to ligation of a tailing nucleic acid, the poly-A region of the RNA comprises 10-15 nucleotides, 15-20 nucleotides, or 20-25 nucleotides. In some embodiments, prior to ligation of a tailing nucleic acid, the poly-A region of the RNA comprises 25-500 nucleotides. In some embodiments, the poly-A region comprises at least 25, at least 30, at least 50, at least 100, at least 150, or at least 200 nucleotides. In some embodiments, the poly-A region comprises at least 30, at least 40, at least 50, at least 60, at least 70, at least 80, at least 90, at least 100, at least 110, at least 120, at least 130, at least 140, at least 150, at least 160, at least 170, at least 180, at least 190, at least 200, at least 210, at least 220, at least 230, at least 240, at least 250, at least 260, at least 270, at least 280, at least 290, or at least 300 nucleotides. In some embodiments, the poly-A region comprises about 200 to about 300 nucleotides. In some embodiments, the poly-A region comprises about 250 nucleotides.

In some embodiments of the methods of producing modified mRNAs provided herein, prior to ligation of a tailing nucleic acid, the RNA comprises, in 5'-to-3' order, a 5' UTR, an open reading frame, a 3' UTR, and a poly-A region. In some embodiments, the open reading frame is between the 5' UTR and the 3' UTR. In some embodiments, the 3' UTR is between the open reading frame and the poly-A region.

In some embodiments of the methods of producing modified mRNAs provided herein, prior to ligation of a tailing nucleic acid, the RNA comprises a 5' cap. In some embodiments, the 5' cap comprises a 7-methylguanosine. In some embodiments, the 5' cap comprises one or more phosphates that connect the 7-methylguanosine to an adjacent nucleotide of the RNA. In some embodiments, a 5' cap is added after ligation of the tailing nucleic acid.

In some aspects of the methods of producing modified mRNAs provided herein comprising ligating a tailing nucleic acid to an mRNA, the tailing nucleic acid comprises one or more modified nucleotides. In some embodiments, the tailing nucleic acid comprises at least one modified nucleotide comprising a modified nucleoside. In some embodiments, at least one modified nucleotide comprises a modified nucleoside comprising a modified nucleobase and/or a modified sugar. In some embodiments, at least one modified nucleotide comprises a modified nucleoside comprising a modified nucleobase and a modified sugar. In some embodiments, at least one modified nucleotide comprises a modified nucleobase. In some embodiments, at least one modified nucleotide comprises a modified sugar. In some embodiments, at least one modified nucleotide comprises a modified phosphate. In some embodiments, at least one modified nucleotide comprises a modified nucleobase selected from the group consisting of: xanthine, allyaminouracil, allyaminothymidine, hypoxanthine, digoxigeninated adenine, digoxigeninated cytosine, digoxigeninated guanine, digoxigeninated uracil, 6-chloropurineriboside, N6-methyladenine, methylpseudouracil, 2-thiocytosine, 2-thiouracil, 5-methyluracil, 4-thiothymidine, 4-thiouracil, 5,6-dihydro-5-methyluracil, 5,6-dihydrouracil, 5-[(3-Indolyl)propionamide-N-allyl]uracil, 5-aminoallylcytosine, 5-aminoallyluracil, 5-bromouracil, 5-bromocytosine, 5-carboxycytosine, 5-carboxymethylesteruracil, 5-carboxyuracil, 5-fluorouracil, 5-formylcytosine, 5-formyluracil, 5-hydroxycytosine, 5-hydroxymethylcytosine, 5-hydroxymethyluracil, 5-hydroxyuracil, 5-iodocytosine, 5-iodouracil, 5-methoxycytosine, 5-methoxyuracil, 5-methylcytosine, 5-methyluracil, 5-propargylaminocytosine, 5-propargylaminouracil, 5-propynylcytosine, 5-propynyluracil, 6-azacytosine, 6-azauracil, 6-chloropurine, 6-thioguanine, 7-deazaadenine, 7-deazaguanine, 7-deaza-7-propargylaminoadenine, 7-deaza-7-propargylaminoguanine, 8-azaadenine, 8-azidoadenine, 8-chloroadenine, 8-oxoadenine, 8-oxoguanine, araadenine, aracytosine, araguanine, arauracil, biotin-16-7-deaza-7-propargylaminoguanine, biotin-16-aminoallylcytosine, biotin-16-aminoallyluracil, cyanine 3-5-propargylaminocytosine, cyanine 3-6-propargylaminouracil, cyanine 3-aminoallylcytosine, cyanine 3-aminoallyluracil, cyanine 5-6-propargylaminocytosine, cyanine 5-6-propargylaminouracil, cyanine 5-aminoallylcytosine, cyanine 5-aminoallyluracil, cyanine 7-aminoallyluracil, dabcyl-5-3-aminoallyluracil, desthiobiotin-16-aminoallyl-uracil, desthiobiotin-6-aminoallylcytosine, isoguanine, N1-ethylpseudouracil, N1-methoxymethylpseudouracil, N1-methyladenine, N1-methylpseudouracil, N1-propylpseudouracil, N2-methylguanine, N4-biotin-OBEA-cytosine, N4-methylcytosine, N6-methyladenine, O6-methylguanine, pseudoisocytosine, pseudouracil, thienocytosine, thienoguanine, thienouracil, xanthosine, 3-deazaadenine, 2,6-diaminoadenine, 2,6-daminoguanine, 5-carboxamide-uracil, 5-ethynyluracil, N6-isopentenyladenine (i6A), 2-methyl-thio-N6-isopentenyladenine (ms2i6A), 2-methylthio-N6-methyladenine (ms2m6A), N6-(cis-hydroxyisopentenyl)adenine (io6A), 2-methylthio-N6-(cis-hydroxyisopentenyl)adenine (ms2io6A), N6-glycinylcarbamoyladenine (g6A), N6-threonylcarbamoyladenine (t6A), 2-methylthio-N6-threonyl carbamoyladenine (ms2t6A), N6-methyl-N6-threonylcarbamoyladenine (m6t6A), N6-hydroxynorvalylcarbamoyladenine (hn6A), 2-methylthio-N6-hydroxynorvalyl carbamoyladenine (ms2hn6A), N6,N6-dimethyladenine (m62A), and N6-acetyladenine (ac6A). In some embodiments, at least one modified nucleotide comprises a modified sugar selected from the group consisting of 2'-thioribose, 2',3'-dideoxyribose, 2'-amino-2'-deoxyribose, 2' deoxyribose, 2'-azido-2'-deoxyribose, 2'-fluoro-2'-deoxyribose, 2'-O-methylribose, 2'-O-methyldeoxyribose, 3'-amino-2',3'-dideoxyribose, 3'-azido-2',3'-dideoxyribose, 3'-deoxyribose, 3'-O-(2-nitrobenzyl)-2'-deoxyribose, 3'-O-methylribose, 5'-aminoribose, 5'-thioribose, 5-nitro-1-indolyl-2'-deoxyribose, 5'-biotin-ribose, 2'-O,4'-C-methylene-linked, 2'-O,4'-C-amino-linked ribose, and 2'-O,4'-C-thio-linked ribose. In some embodiments, at least one modified nucleotide comprises a modified phosphate selected from the group consisting of phosphorothioate (PS), thiophosphate, 5'-O-methylphosphonate, 3'-O-methylphosphonate, 5'-hydroxyphosphonate, hydroxyphosphanate, phosphoroselenoate, selenophosphate, phosphoramidate, carbophosphonate, methylphosphonate, phenylphosphonate, ethylphosphonate, H-phosphonate, guanidinium ring, triazole ring, boranophosphate (BP), methylphosphonate, and guanidinopropyl phosphoramidate.

In some embodiments of the methods of producing modified mRNAs provided herein, the tailing nucleic acid comprises more than one type of modified nucleotide. In some embodiments, the tailing nucleic acid comprises at least a first modified nucleoside, and a second modified nucleoside that has a different structure from the first modified nucleoside. In some embodiments, the tailing nucleic acid comprises at least a first modified phosphate, and a second modified phosphate that has a different structure from the first modified phosphate. In some embodiments, the tailing nucleic acid comprises a modified nucleoside and a modified nucleoside.

In some embodiments, 1% to 90% of the nucleotides of the tailing nucleic acid are modified nucleotides. In some embodiments, at least 2%, at least 3%, at least 4%, at least 5%, at least 6%, at least 7%, at least 8%, at least 9%, at least 10%, at least 12%, at least 14%, at least 16%, at least 18%, at least 20%, at least 25%, at least 30%, at least 35%, at least 40%, at least 45%, or at least 50% of the nucleotides of the tailing nucleic acid are modified nucleotides. In some embodiments, 3 or more of the 10-25 last nucleotides of the tailing nucleic acid are modified nucleotides. In some embodiments, at least 4, 5, 6, 7, 8, 9, 10, 15, 20, or 25 of the 10-25 last nucleotides of the tailing nucleic acid are modified nucleotides.

In some embodiments, a tailing nucleic acid comprises a poly-A region comprising two or more poly-A tails and at least two instances of the poly-A tails are covalently linked to a first linker. In some embodiments, the tailing nucleic acid comprises a poly-A region comprising the following structure: -(a first poly-A tail)-[(a first linker)-(a second poly-A tail)n1]n2; wherein each instance of n1 is independently an integer between 1 and 20, inclusive; n2 is an integer between 2 and 10, inclusive; and the first poly-A tail is covalently ligated to the 3' UTR of the mRNA.

In some embodiments, the tailing nucleic acid comprises a poly-A region comprising a brushed oligonucleotide comprising two or more poly-A tails.

In some embodiments, each instance of n1 is 1. In some embodiments, at least one instance of the first linker is covalently linked to an internal nucleotide of the first poly-A tail. In some embodiments, at least one instance of the first linker is covalently linked to a second poly-A tail at a 3' nucleotide of the second poly-A tail. In some embodiments, at least one instance of the first linker comprises at least one moiety formed by reacting two orthogonal click-chemistry handles. In certain embodiments, the click-chemistry handles are as described herein. In some embodiments, each instance of the first linker is independently substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, or substituted or unsubstituted, C₁₋₂₀₀ heteroalkynylene. In some embodiments, one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with substituted or unsubstituted carbocyclylene, substituted or unsubstituted heterocyclylene, substituted or unsubstituted arylene, or substituted or unsubstituted heteroarylene. In some embodiments, one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with 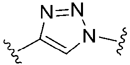 or 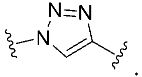 In some embodiments, one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with 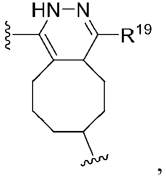 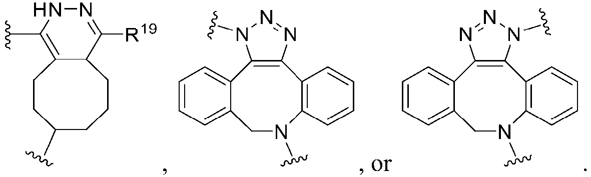 In some embodiments, at least one instance of the first linker is of the formula: 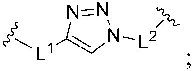 wherein each instance of L¹ is independently substituted or unsubstituted, C₁₋₁₉₉ alkylene or substituted or unsubstituted, C₁₋₁₉₉ heteroalkylene; and each instance of L² is independently substituted or unsubstituted, C₁₋₁₉₉ alkylene or substituted or unsubstituted, C₁₋₁₉₉ heteroalkylene. In some embodiments, at least one instance of the first linker is of the formula: 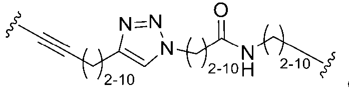 or

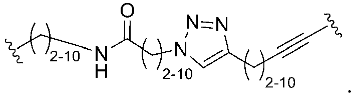

In some embodiments, the first poly-A tail comprises a nucleotide sequence set forth as: 5'-rArArArArArAdU(a1)rArArArArArArArArArArArAdU(a1)rArArArArArArArArArArArAdU( a1)ddC-3' (SEQ ID NO: 1); and at least one instance of the second poly-A tails comprises a nucleotide sequence set forth as: 5'-rArArArArArArArArArArArArArArArArArArArArArArArArA*rA*rA* rA*rA*rA*(b1)-3' (SEQ ID NO: 2); wherein each instance of the first linker is independently 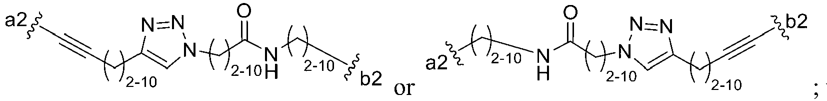 wherein "rA" represents an adenosine ribonucleotide; "dU(a1)" represents a modified uridine deoxyribonucleotide; "ddC" represents a cytosine dideoxyribonucleotide; "*" represents a phosphorothioate linkage; the a1 of each instance of dU(a1) represents an attachment point on the uridine of the dU(a1); the b1 of rA*(b1) represents an attachment point on the * of the rA*(b1); each instance of a1 is attached to an instance of a2; and each instance of b1 is attached to an instance of b2. In some embodiments, the modified uridine deoxyribonucleotide is 5-Octadiynyl deoxyuridine, which is shown in the structure:

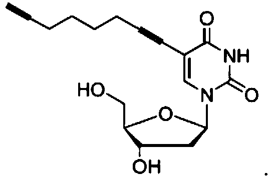

In some embodiments, the tailing nucleic acid comprises a poly-A region comprising a dendrimer comprising two or more poly-A tails.

In some embodiments, each instance of n1 is independently an integer between 2 and 20, inclusive. In some embodiments, at least one instance of the first linker comprises a dendrimer. In some embodiments, at least one instance of the dendrimer is a polyamidoamine (PAMAM) dendrimer. In some embodiments, at least one instance of the PAMAM dendrimer is of the formula: 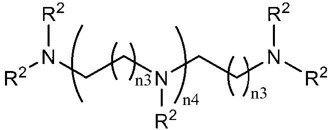 wherein each instance of n3 is independently an integer between 1 and 10, inclusive; each instance of n4 is independently an integer between 0 and 10, inclusive; each instance of R² is independently hydrogen or 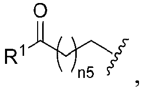 provided that at least three instances of R² is 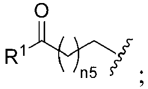 and each instance of R¹ is substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, or substituted or unsubstituted, C₁₋₁₀ heteroalkynylene. In some embodiments, one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with substituted or unsubstituted carbocyclylene, substituted or unsubstituted heterocyclylene, substituted or unsubstituted arylene, or substituted or unsubstituted heteroarylene; and each instance of n5 is independently an integer between 0 and 10, inclusive. In some embodiments, at least one instance of the PAMAM dendrimer is of the following formula:

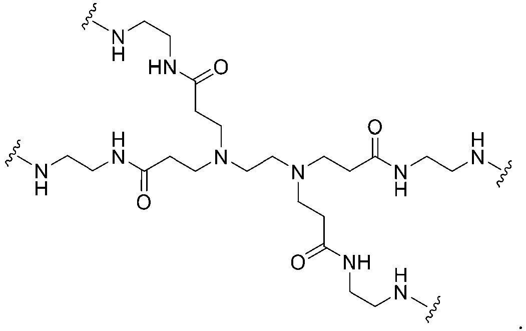

In some embodiments, one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with 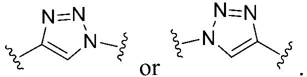 In some embodiments, one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with 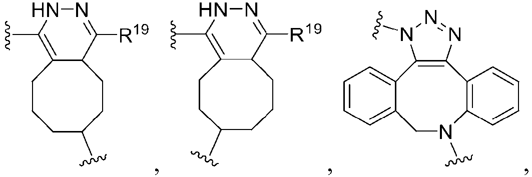 or 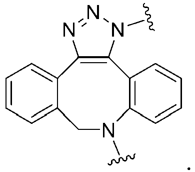 In some embodiments, at least one instance of the first linker is of the following formula:

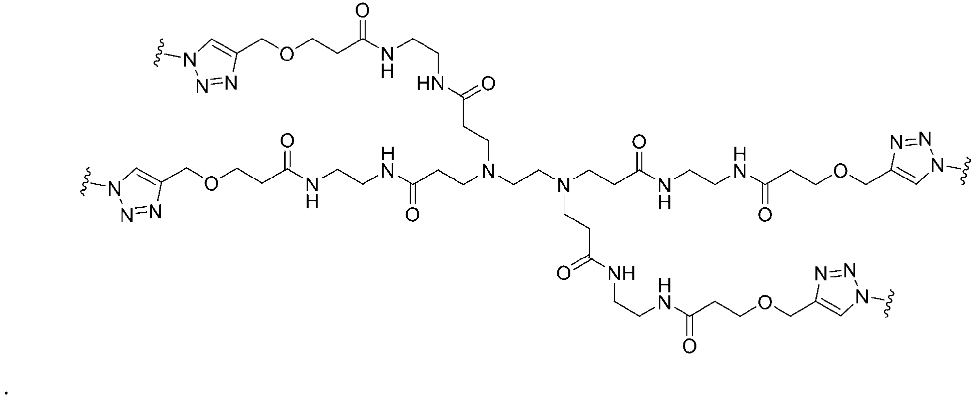

In some embodiments, the first poly-A tail and the second poly-A tails comprise a nucleotide sequence set forth as: 5'-rArArArArArArArArArArArArArArArArArArArArArArArArA*rA*rA* rA*rA*rA*/AzideN/- 3' (SEQ ID NO: 3); wherein "rA" represents an adenosine ribonucleotide; "AzideN" represents a 3' azide moiety and "*" represents a phosphorothioate linkage.

In some embodiments, at least one instance of the first linker is covalently linked to a 3' nucleotide of the first poly-A tail. In some embodiments, at least one instance of the first linker is covalently linked to a 3' nucleotide of the at least one instance of the second poly-A tails.

In some embodiments, the capping nucleic acid comprises a 5' cap region comprising two or more 5' caps, and at least two instances of the 5' caps are covalently linked by a second linker. In some embodiments, the capping nucleic acid comprises a 5' cap region comprising the following structure: -(a first 5' cap)-[(a second linker)-(a second 5' cap)m1]m2; wherein each instance of m1 is independently an integer between 1 and 20, inclusive; m2 is an integer between 2 and 10, inclusive; the first 5' cap is covalently linked to the 5' UTR; and the second linker is covalently linked to the first 5' cap and to the second 5' cap.

In some embodiments, the capping nucleic acid comprises a 5' cap region comprising a brushed oligonucleotide comprising two or more 5' caps.

In some embodiments, each instance of m1 is 1. In some embodiments, at least one instance of the second linker is covalently linked to an internal nucleotide of the first 5' cap. In some embodiments, at least one instance of the second linker is covalently linked to a second 5' cap at a 3' nucleotide of the second 5' cap. In some embodiments, at least one instance of the second linker comprises at least one moiety formed by reacting two orthogonal click-chemistry handles. In certain embodiments, the click-chemistry handles are described herein. In some embodiments, each instance of the second linker is independently substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, or substituted or unsubstituted, C₁₋₂₀₀ heteroalkynylene. In some embodiments, one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with substituted or unsubstituted carbocyclylene, substituted or unsubstituted heterocyclylene, substituted or unsubstituted arylene, or substituted or unsubstituted heteroarylene. In some embodiments, one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with 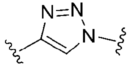 or 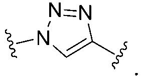 In some embodiments, one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with 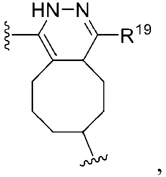 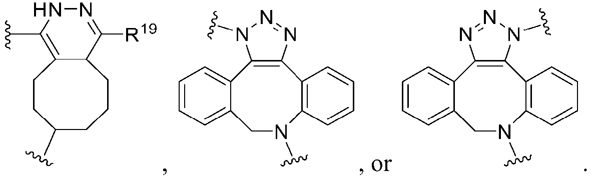 In some embodiments, at least one instance of the second linker is of the formula: 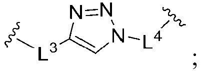 wherein each instance of L³ is independently substituted or unsubstituted, C₁₋₁₉₉ alkylene or substituted or unsubstituted, C₁₋₁₉₉ heteroalkylene; and each instance of L⁴ is independently substituted or unsubstituted, C₁₋₁₉₉ alkylene or substituted or unsubstituted, C₁₋₁₉₉ heteroalkylene. In some embodiments, at least one instance of the second linker is of the formula: 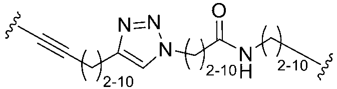 or

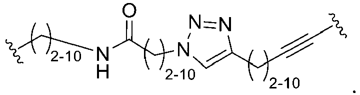

In some embodiments, the first 5' cap comprises a nucleotide sequence set forth as:
5'-/Cap/rGrGrGrArArAdU(c1)rArArGrArGrArGrArArArArGrArArGrArGdU(c1)rArArGrArA rGrArArAdU(cl)rA-3' (SEQ ID NO: 4); and the second 5' cap comprises a nucleotide sequence set forth as: 5'-/Cap/rGrGrGrArGrArCrTrGrCrCrArCrCrA* rA*rA*rA*rA*rA*(d1)-3' (SEQ ID NO: 5); wherein
each instance of the second linker is independently 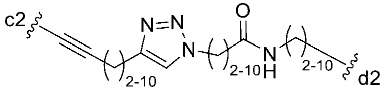 or 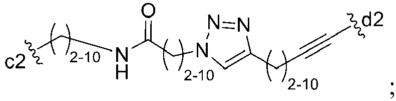 wherein "rA" represents an adenosine ribonucleotide; "rT" represents a thymidine ribonucleotide; "rC" represents a cytidine ribonucleotide; "rG" represents a guanosine ribonucleotide; "dU(c1)" represents a modified uridine deoxyribonucleotide; "*" represents a phosphorothioate linkage; "Cap" represents a 5' cap; the c1 of each instance of dU(cl) represents an attachment point on the U of the dU(c1); the d1 of rA*(d1) represents an attachment point on the * of the rA*(d1); each instance of c1 is attached to an instance of c2; and each instance of d1 is attached to an instance of d2. In some embodiments, the modified uridine deoxynucleotide is 5-Octadiynyl deoxyuridine which is shown in the structure:

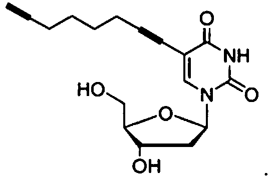

In some embodiments, the capping nucleic acid comprises a 5' cap region comprising a dendrimer comprising two or more 5' caps.

In some embodiments, each instance of m1 is independently an integer between 2 and 20, inclusive. In some embodiments, at least one instance of the second linker comprises a dendrimer. In some embodiments, at least one instance of the dendrimer is a polyamidoamine (PAMAM) dendrimer. In some embodiments, at least one instance of the PAMAM dendrimer is of the formula: 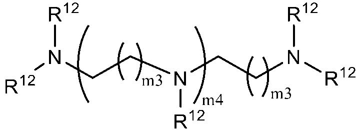 wherein each instance of m3 is independently an integer between 1 and 10, inclusive; each instance of m4 is independently an integer between 0 and 10, inclusive; each instance of R¹² is independently hydrogen or 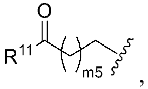 provided that at least three instances of R¹² is 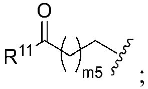 and each instance of R¹¹ is substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, or substituted or unsubstituted, C₁₋₁₀ heteroalkynylene. In some embodiments, one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with substituted or unsubstituted carbocyclylene, substituted or unsubstituted heterocyclylene, substituted or unsubstituted arylene, or substituted or unsubstituted heteroarylene; and each instance of m5 is independently an integer between 0 and 10, inclusive. In some embodiments, at least one instance of the PAMAM dendrimer comprises the following formula:

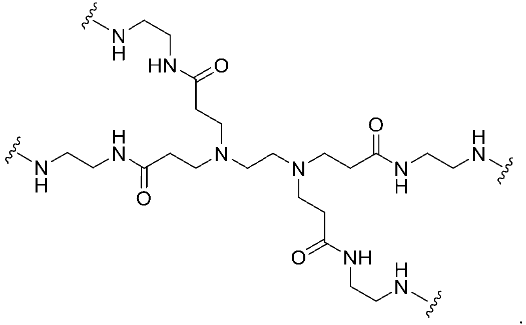

In some embodiments, one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with 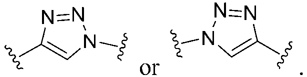 In some embodiments, one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with 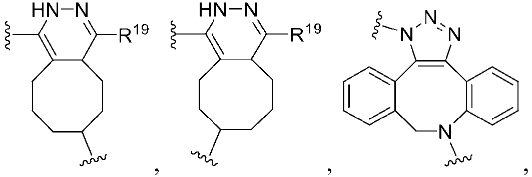 or 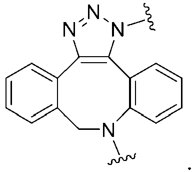 In some embodiments, at least one instance of the first linker is of the following formula:

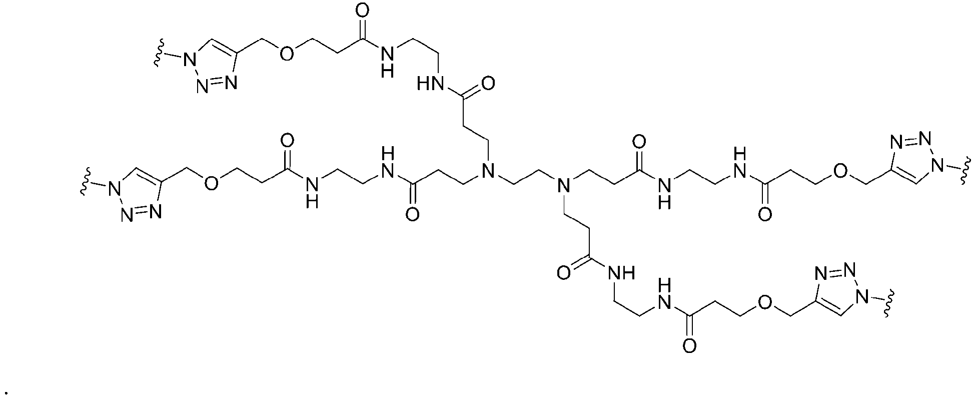

In some embodiments, the first 5' cap comprises a nucleotide sequence set forth as:
/5AzideN/rArArA rArA-3'; and the second 5' cap comprises a nucleotide sequence set forth as: 5'-/Cap/rGrGrGrArGrArCrTrGrCrCrArCrCrA*rA*rA*rA*rA*rA*/3AzideN/-3' (SEQ ID NO: 6); wherein "rA" represents an adenosine ribonucleotide; "rT" represents a thymidine ribonucleotide; "rC" represents a cytidine ribonucleotide; "rG" represents a guanosine ribonucleotide; "5AzideN" represents a 5' azide moiety, "3AzideN" represents a 3' azide moiety, "*" represents a phosphorothioate linkage, and "Cap" represents a 5' cap.

In some embodiments, at least one instance of the second linker is covalently linked to a 3' nucleotide of the first 5' cap. In some embodiments, at least one instance of the second linker is covalently linked to a 3' nucleotide of the at least one instance of the second 5' caps.

In some embodiments, the modified mRNA described herein is purified by any method known in the art to remove undesired components from IVT or associated reactions (e.g., click chemistry reactions) (including unincorporated rNTPs, protein enzyme, salts, metal ions, short RNA oligos, etc). Techniques for the isolation of the mRNA transcripts are well known in the art. Well-known procedures include phenol/chloroform extraction or precipitation with alcohol (ethanol, isopropanol) in the presence of monovalent cations or lithium chloride. Additional, nonlimiting examples of purification procedures which can be used include size exclusion chromatography (Lukavsky, P.J. and Puglisi, J.D., 2004, Large-scale preparation and purification of polyacrylamide-free RNA oligonucleotides, RNA v.10, 889-893), silica-based affinity chromatography and polyacrylamide gel electrophoresis (Bowman, et al. in RNA in vitro transcription and RNA purification by denaturing PAGE in Recombinant and in vitro RNA syntheses Methods v. 941 Conn G.L. (ed), New York, N.Y. Humana Press, 2012). Purification can be performed using a variety of commercially available kits including, but not limited to SV Total Isolation System (Promega) and In Vitro Transcription Cleanup and Concentration Kit (Norgen Biotek). Techniques to remove contaminants, such as dsDNA, have been developed and are known in the art including but not limited to scalable HPLC purification (see, e.g., Kariko, et al., 2011, Nucl Acid Res, v. 39 e142; Weissman, et al., 2012, Synthetic Messenger RNA and Cell Metabolism Modulation v.969 (Rabinovich, P.H. Ed)). In a preferred embodiment, modified mRNA is purified through HPLC, as HPLC-purified mRNA has been reported to be translated at much greater levels compared to other purification methods, particularly in primary cells and in vivo.

### Compositions comprising modified mRNAs and methods of use

In some aspects, the present disclosure provides compositions comprising any one of the modified mRNAs provided herein. In some embodiments, the modified mRNA is made by any of the methods provided herein comprising ligating a tailing nucleic acid and/or capping nucleic acid onto an RNA. Compositions comprising a modified mRNA are useful for delivering the modified mRNA to a cell in order to vaccinate the subject against a foreign antigen, or express a therapeutic protein to treat a condition or disorder. Compositions comprising modified mRNAs are also useful for exerting a desired effect in a subject in the absence of disease, such as for agricultural uses. For example, an mRNA encoding a biological pesticide or growth-augmenting factor may be used to increase the tolerance of a plant to pests, or modulate growth in a manner that increases crop yield, respectively.

In some embodiments, the open reading frame (ORF) of the mRNA is codon-optimized for expression in a cell of a subject. As used herein, "codon-optimized" refers to the preferential use of codons that are more efficiently translated in a cell. Multiple codons can encode the same amino acid, with the translation rate and efficiency of each codon being determined by multiple factors, such as the intracellular concentration of aminoacyl-tRNAs comprising a complementary anticodon. Codon optimization of a nucleic acid sequence may include replacing one or more codons with codons that encode the same amino acid as, but are more efficiently translated than, the replaced codons. For example, the amino acid threonine (Thr) may be encoded by ACA, ACC, ACG, or ACT (ACU in RNA), but in mammalian host cells ACC is the most commonly used codon; in other species, different Thr codons may be preferred for codon-optimized. An mRNA with a codon-optimized open reading frame is thus expected to be translated more efficiently, and produce more polypeptides in a given amount of time, than an mRNA with an open reading frame that is not codon-optimized. In some embodiments, the open reading frame is codon-optimized for expression in a human cell.

In some embodiments of the modified mRNAs provided herein, the open reading frame encodes an antigen or a therapeutic protein. As used herein, a "therapeutic protein" refers to a protein that prevents, reduces, or alleviates one or more signs or symptoms of a disease when expressed in a subject, such as a human subject that has, or is at risk of developing, a disease or disorder. A therapeutic protein may be, for example, an essential enzyme, clotting factor, transcription factor, growth factor, cytokine, chemokine, antibody (or antibody fragment thereof), protein hormone, signaling protein, structural protein, or cell surface receptor encoded by a gene that is mutated in a subject. A mutation in a gene encoding such a protein may cause diminished levels of the protein to be expressed in one or more cells of the subject. For example, IPEX syndrome in humans is caused by a mutation in the *FOXP3* gene, which hinders development of FOXP3+ regulatory T cells and results in increased susceptibility to autoimmune and inflammatory disorders. Expression of an essential enzyme, clotting factor, transcription factor, growth factor, cytokine, chemokine, antibody (or antibody fragment thereof), protein hormone, signaling protein, structural protein, or cell surface receptor from an mRNA may therefore compensate for a mutation in the gene encoding such a protein in a subject. In some embodiments, the therapeutic protein is a protein that is expressed in one or more cells of a subject a level that is less than (e.g., significantly less than) that of a reference value, such as the level of expression of the protein that is typical in cells of one or more healthy subjects (i.e., subjects who do not have and are not at risk for developing the disease or disorder). As used herein, "antigen" refers to a molecule (e.g., a protein) that, when expressed in a subject, elicits the generation of antibodies in the subject that bind to the antigen. In some embodiments, the antigen is a protein derived from a pathogen, such as a pathogenic virus, bacterium, protozoan, or fungus. In some embodiments, the antigen is a protein derived from a virus (viral antigen) or a fragment thereof. In some embodiments, the antigen is a protein derived from a bacterium (bacterial antigen) or a fragment thereof. In some embodiments, the antigen is a protein derived from a protozoan (protozoal antigen) or a fragment thereof. In some embodiments, the antigen is a protein derived from a fungus (fungal antigen) or a fragment thereof. A fragment of a full-length protein refers to a protein with an amino acid sequence that is present in, but shorter than, the amino acid sequence of the full-length protein.

In some embodiments, a composition provided herein (*e.g.,* a pharmaceutical composition) further comprises one or more additional agents. In some embodiments, the additional agent is a nucleotide, a nucleic acid, an amino acid, a peptide, a protein, a small molecule, an aptamer, a lipid, or a carbohydrate. In some embodiments, the additional agent is an agent which has a therapeutic effect when administered to a subject. In some embodiments, the additional agent is an agent that is capable of modulating expression of a gene and/or protein is a subject, such as a short hairpin RNA (shRNA), a small interfering RNA (siRNA), or an antisense oligonucleotide (ASO). In some embodiments, the additional agent is a small molecular inhibitor. In some embodiments, the additional agent is an agent that is capable of eliciting or enhancing an immune response in a subject. In some embodiments, the additional agent is an antigen (*e.g.,* a viral antigen, a bacterial antigen). In some embodiments, the additional agent is an adjuvant, which is defined as an agent that is sufficient for enhancing an immune response in a subject when administered at an effective amount, but does not elicit an immune response in a subject when administered alone. In some embodiments, the additional agent is an enzyme, such as an enzyme that is capable of catalyzing one or more chemical reactions in a subject or in cells of a subject.

In some aspects, the present disclosure provides a delivery reagent comprising any of the modified mRNAs provided herein. In some embodiments, any of the modified mRNAs provided herein are conjugated to a delivery agent. Any of the modified mRNAs provided herein may be conjugated to a delivery agent that includes, for example, to a lipid, a peptide, a protein, an antibody, or a carbohydrate. Lipids used in the conjugation and delivery of modified mRNAs are generally known in the art, and include, for example, cholesterol. Peptides, proteins, antibodies, and carbohydrates used in the conjugation and delivery of modified mRNAs are generally known in the art and include, for example, any peptide, protein, antibody, or carbohydrate known to bind specifically to a moiety (e.g., a protein) on the surface of a target cell type. Methods for conjugating a lipid, peptide, protein, antibody, or carbohydrate to a modified mRNA include, for example, methods of conjugating a lipid, peptide, protein, antibody, or carbohydrate to a modified mRNA at a 5' or 3' terminus, and are generally known in the art.

In some embodiments, any of the modified mRNAs provided herein are conjugated to or encapsulated by a delivery agent that includes, for example, a nanoparticle, a microparticle, or an exosome. A nanoparticle refers to a particle having a diameter between approximately 10 nm and 1000 nm. A microparticle is defines as a particle having a diameter greater than 1000 nm (1 µm), such as a particle having a diameter between approximately 1 µm and 100 µm. In some embodiments, a nanoparticle or microparticle is approximately spherical. In some embodiments, a nanoparticle or microparticle is hollow, comprising an internal core. In some embodiments, a nanoparticle or microparticle is a lipid nanoparticle or lipid microparticle, respectively. A lipid nanoparticle or lipid microparticle refers to a composition comprising one or more lipids that form an aggregate of lipids, or an enclosed structure with an interior surface and an exterior surface. In some embodiments, a lipid nanoparticle or lipid microparticle comprises a lipid bilayer that encloses an aqueous core. Lipids used in the formulation of lipid nanoparticles and lipid microparticles for delivering mRNA are generally known in the art, and include, but are not limited to, ionizable amino lipids, non-cationic lipids, sterols, and polyethylene glycol-modified lipids. See, *e.g.,* Buschmann et al. Vaccines. 2021. 9(1):65. In some embodiments, the modified mRNA is surrounded by the lipids of the lipid nanoparticle or the lipid microparticle and are present in the interior of the lipid nanoparticle or lipid microparticle. In some embodiments, the mRNA is dispersed throughout the lipids of the lipid nanoparticle or lipid microparticle. In some embodiments, the lipid nanoparticle or lipid microparticle comprises an ionizable amino lipid, a non-cationic lipid, a sterol, and/or a polyethylene glycol (PEG)-modified lipid. Lipid nanoparticles and lipid microparticles comprising modified mRNAs may be prepared by any means generally known in the art, such as, for example, detergent dialysis, emulsion, centrifugation, evaporation, thin film hydration, or ethanol dilution. See, *e*.*g*., Barba et al. Pharmaceutics. 2019. 11(8):360. An exosome refers to a type of lipid nanoparticle produced by eukaryotic cells as a result of the inward budding of vesicles within multivesicular bodies and are generally between 30 nm and 150 nm in diameter. Exosomes comprise a heterogenous mixture of endogenous lipids, such as phospholipids, membrane-anchored proteins, and carbohydrates present in eukaryotic cells, and enclose an aqueous core. Exosomes may have beneficial features that are difficult to achieve with synthetically produced lipid nanoparticles, such as, for example, the ability to pass through the blood brain barrier and deliver modified mRNAs to tissues within the brain. Exosomes comprising modified mRNAs may be produced by any means generally known in the art, such as, for example, by sonicating or electroporating isolated exosomes in the presence of modified mRNA, or mixing exosomes with a lipid-conjugated modified mRNA, such as, for example, modified mRNA that has been conjugated to cholesterol. See, *e.g.,* Roberts et al. Nat Rev Drug Discov. 2020. 19(10):673-694.

In some embodiments, a nanoparticle or microparticle is a polymeric nanoparticle or polymeric microparticle, respectively. A polymeric nanoparticle or polymeric microparticle refers to a nanoparticle or microparticle composition, respectively, comprising one or more polymers that form an aggregate of polymers, or an enclosed structure with an interior surface and an exterior surface. In some embodiments, a polymeric nanoparticle or polymeric microparticle comprises a polymeric layer that encloses an aqueous core. Polymers used in the formulation of polymeric nanoparticles and polymeric microparticles for delivering mRNA are generally known in the art, and include cationic polymers such as, but are not limited to, polyethylenimine (PEI), poly-amido-amine (PAA), poly-beta amino-esters (PBAEs), polylysine (PLL), spermine, chitosan, polyurethane, and derivatives thereof (*e.g*., PEI stearic acid (PSA) copolymer). See, *e.g.,* Liu et al. Front Bioeng Biotechnol. 2021. 9:718753. In some embodiments, the modified mRNA is surrounded by the polymers of the polymeric nanoparticle or the polymeric microparticle and are present in the interior of the polymeric nanoparticle or polymeric microparticle. In some embodiments, the mRNA is dispersed throughout the polymers of the polymeric nanoparticle or polymeric microparticle.

In some embodiments, a nanoparticle or microparticle is a protein nanoparticle or protein microparticle, respectively. A protein nanoparticle or protein microparticle refers to a nanoparticle or microparticle composition, respectively, comprising one or more proteins that form an aggregate of proteins, or an enclosed structure with an interior surface and an exterior surface. In some embodiments, a protein nanoparticle or protein microparticle comprises a protein layer that encloses an aqueous core. Proteins used in the formulation of protein nanoparticles and protein microparticles for delivering mRNA are generally known in the art, and include but are not limited to, viral coat proteins and ferritin. See, *e.g.,* Wang et al. Nat Nanotechnol. 2020. 15(5):406-416. In some embodiments, the modified mRNA is surrounded by the proteins of the protein nanoparticle or the protein microparticle and are present in the interior of the protein nanoparticle or protein microparticle. In some embodiments, the modified mRNA is external to the proteins of the protein nanoparticle or the protein microparticle and are attached to the exterior surface of the protein nanoparticle or protein microparticle. In some embodiments, the modified mRNA is conjugated to proteins of the protein nanoparticle or protein microparticle through a covalent linkage, such as, for example, that formed by a click chemistry reaction, or by fusing the modified mRNA and protein each to a protein or peptide of a protein/peptide pair known to react to form a covalent linkage (*e.g.,* a SpyCatcher/SpyTag protein/peptide pair).

In some embodiments, a nanoparticle or microparticle is a solid nanoparticle or solid microparticle. A solid nanoparticle or solid microparticle refers to a nanoparticle or microparticle composition, respectively, comprising one or more materials that form a solid structure, which has an external surface and may or may not comprise an internal surface. A solid nanoparticle or solid microparticle may comprise any suitable material that is generally known in the art, such as, for example, gold, silver, or silicon dioxide (silica). In some embodiments, a modified mRNA is conjugated to the external surface of a solid nanoparticle or solid microparticle. Solid nanoparticles and solid microparticles comprising modified mRNAs may be produced by any means generally known in the art, such as, for example, by linking the modified mRNAs to the surface of the solid nanoparticle or solid microparticle through thiol linkages (*e.g*., modifying the DNA to comprise cyclic disulfide-anchoring groups), or by modifying the external surface of the solid nanoparticle or solid microparticle with one or more cationic materials (e.g., PEI) within which modified mRNAs are present. See, *e.g.,* Roberts et al. Nat Rev Drug Discov. 2020. 19(10):673-694, Lee et al. Nano Lett. 2007, 7(7):2112-2115, and Paris and Vallet-Regi. Pharmaceutics. 2020, 12(6):526.

In some aspects, the present disclosure provides cells comprising any of the modified mRNAs provided herein. In some embodiments, the cell is a human cell comprising any one of the modified mRNAs provided herein. A "cell" is the basic structural and functional unit of all known independently living organisms. It is the smallest unit of life that is classified as a living thing. Some organisms, such as most bacteria, are unicellular (consist of a single cell). Other organisms, such as plants, fungi, and animals, including cattle, horses, chickens, turkeys, sheep, swine, dogs, cats, and humans, are multicellular. In some embodiments, the half-life of the modified mRNA in the cell is 15-900 minutes. In some embodiments, the half-life of the modified mRNA in the cell is 30-600 minutes. In some embodiments, the half-life of the modified mRNA in the cell is 60-300 minutes. In some embodiments, the half-life of the modified mRNA is at least 15, at least 20, at least 25, at least 30, at least 35, at least 40, at least 45, at least 50, at least 55, at least 60 minutes. In some embodiments, the half-life of the modified mRNA in the cell is at least 30, at least 60, at least 90, at least 120, at least 150, at least 180, at least 210, at least 240, at least 270, at least 300, at least 330, at least 360, at least 390, at least 420, at least 450, at least 480, at least 510, at least 540, at least 570, at least 600, at least 630, at least 660, at least 690, at least 720, at least 750, at least 780, at least 810, at least 840, or at least 870 minutes.

In some aspects, the present disclosure provides compositions comprising any of the modified mRNAs, delivery agents, or cells provided herein. In some embodiments, the composition further comprises one or more additional agents, such as a nucleotide, a nucleic acid, an amino acid, a peptide, a protein, a small molecule, an aptamer, a lipid, or a carbohydrate. In some embodiments, the additional agent has a therapeutic effect when administered to a subject. In some embodiments, the additional agent is an agent for use in modulating the expression and/or activity of one or more gene products (*e.g*., proteins) in a subject. In some embodiments, the additional agent is a nucleic acid for use in decreasing the expression and/or activity of one or more gene products (*e.g*., proteins), such as a short hairpin RNA (shRNA), small interfering RNA (siRNA), or an antisense oligonucleotide (ASO). In some embodiments, the additional agent is an inhibitor for decreasing the activity of one or more gene products (e.g., proteins). In some embodiments, the agent is a small molecular inhibitor. In some embodiments, the additional agent is an agent for enhancing an immune response in a subject. In some embodiments, the additional agent is an antigen, such as a nucleic acid antigen, a protein antigen, or a phospholipid antigen. In some embodiments, the additional agent is an adjuvant, such as, for example, aluminum hydroxide or potassium aluminum sulfate (alum), monophosphoryl lipid A (MPL), an oil-in-water emulsion (e.g., a squalene emulsion), a cytosine phosphoguanine (CpG) oligodeoxynucleotide, or another adjuvant that is known in the art. See, *e.g.,* Di Pasquale, A et al. Vaccines. 2015. 3(2):320-343. In some embodiments, the composition is a pharmaceutical composition comprising any one of the modified mRNAs, delivery agents, or cells provided herein, and a pharmaceutically acceptable excipient. Pharmaceutically acceptable excipients, carriers, buffers, stabilisers, isotonicising agents, preservatives or antioxidants, or other materials well known to those skilled in the art. Such materials should be non-toxic and should not interfere with the efficacy of the active ingredient. The precise nature of the carrier or other material may depend on the route of administration, e.g., parenteral, intramuscular, intradermal, sublingual, buccal, ocular, intranasal, subcutaneous, intrathecal, intratumoral, oral, vaginal, or rectal.

In some aspects, the present disclosure provides a method of administering to a subject any of the modified mRNAs, delivery agents, cells, compositions, or pharmaceutical compositions provided herein. In some embodiments, the subject is a human. In some embodiments, the administration is parenteral, intramuscular, intradermal, sublingual, buccal, ocular, intranasal, subcutaneous, intrathecal, intratumoral, oral, vaginal, or rectal. In some embodiments, the composition is to be stored below 50°C, below 40 °C, below 30 °C, below 20 °C, below 10 °C, below 0 °C, below -10 °C, below -20 °C, below -30 °C, below -40 °C, below - 50 °C, below -60°C, below -70 °C, or below -80 °C, such that the nucleic acids are relatively stable over time. In some embodiments, the modified mRNA is introduced into a cell in a subject by *in vivo* electroporation. *In vivo* electroporation is the process of introducing nucleic acids or other molecules into a cell of a subject using a pulse of electricity, which promote passage of the nucleic acids or other molecules through the cell membrane and/or cell wall. See, *e*.*g*., Somiari et al. Molecular Therapy., 2000. 2(3):178-187. The modified mRNA to be delivered is administered to the subject, such as by injection, and a pulse of electricity is applied to the injection site, whereby the electricity promotes entry of the nucleic acid into cells at the site of administration. In some embodiments, the modified mRNA is delivered to and taken up by cells of the subject (e.g., cells local to the site of administration or throughout the subject) via a delivery agent that is associated with (e.g., conjugated to) the modified mRNA. In some embodiments, the modified mRNA is administered with other elements, such as buffers and/or excipients, that increase the efficiency of electroporation.

In some aspects, the present disclosure provides a kit comprising any of the RNAs and any of the tailing nucleic acids and/or capping nucleic acids provided herein. The RNA and tailing nucleic acid and/or capping nucleic acid can be combined in the presence of an RNA ligase to produce a modified mRNA, such as one of the modified mRNAs provided herein. In some embodiments, the kit comprises a ligase. In some embodiments, the kit comprises an RNA ligase. In some embodiments, the kit comprises a T4 RNA ligase. In some embodiments, a kit comprises a T4 RNA ligase 1. In some embodiments, a kit comprises a T4 RNA ligase 2. In some embodiments, the kit comprises an RtcB RNA ligase. In some embodiments, the kit further comprises a buffer for carrying out the ligation. In some embodiments, the kit further comprises a nucleotide triphosphate, such as ATP, to provide energy required by the ligase. In some embodiments, the kit is to be stored below 50 °C, below 40 °C, below 30 °C, below 20 °C, below 10 °C, below 0 °C, below -10 °C, below -20 °C, below -30 °C, below -40 °C, below -50 °C, below -60°C, below -70 °C, or below -80 °C, such that the nucleic acids are relatively stable over time.

In some aspects, the present disclosure provides a kit comprising any of the pharmaceutical compositions provided herein and a delivery device. A delivery device refers to machine or apparatus suitable for administering a composition to a subject, such as a syringe or needle. In some embodiments, the kit is to be stored below 50 °C, below 40 °C, below 30 °C, below 20 °C, below 10 °C, below 0 °C, below -10 °C, below -20 °C, below -30 °C, below -40 °C, below -50 °C, below -60°C, below -70 °C, or below -80 °C, such that the nucleic acids of the pharmaceutical composition are relatively stable over time. In some embodiments, the kit further comprises instructions for administering any of the pharmaceutical compositions provided herein to a subject.

### EXAMPLES

In order that the disclosure may be more fully understood, the following examples are set forth. The examples are offered to illustrate the modified mRNAs, pharmaceutical compositions, kits, and methods provided herein and are not to be construed in any way as limiting their scope.

### Materials and Methods

### Plasmid Cloning, Characterization, and Purification

The DNA coding sequences (CDS) of proteins of interest were inserted into an optimized backbone containing (in order) an SP6 or T7 promoter sequence, a 5' human alpha globin UTR, a CDS, a 3' human alpha globin UTR, a 100×A template-encoded poly(A) tail, and an Esp3I linearization site. The CDS-containing plasmid/gene blocks were PCR amplified, gel-purified, and assembled into the optimized backbone using NEBuilder HiFi DNA Assembly Master Mix (NEB, E2621S), transformed into stable cells, and sequence-verified with Sanger sequencing.

The firefly luciferase construct was obtained from pmirGLO Dual-Luciferase miRNA Target Expression Vector (Promega, E1330). The destabilized Firefly luciferase construct (i.e., Firefly-PEST) was obtained by inserting a GeneBlock (IDT, human codon-optimized) encoding the degron derived from mouse ornithine decarboxylase. The Cas9 encoding constructs were obtained from Addgene (Plasmid #52962) deposited by Feng Zhang lab. The nano luciferase constructs were obtained by gene synthesis from Genewiz. Renilla luciferase constructs were obtained from pmirGLO without cloning into the optimized vector.

### Linear mRNA Synthesis and Characterization

DNA plasmids were linearized by Esp3I (NEB, R0734S). Linearized plasmids were purified with the DNA Clean & Concentrator-25 kit from Zymo Research (D4033) and characterized with agarose gel electrophoresis. mRNA constructs were synthesized by in vitro transcription (IVT) using mMESSAGE mMACHINE SP6 Transcription Kit [ThermoFisher Scientific, AM1340] (for SP6 promoter constructs) or HiScribe T7 High Yield RNA Synthesis Kit [NEB, E2040S] (for T7 promoter constructs) per manufacturer's protocol except 100% replacement of UTP with N1-methyl pseudouridine-5'-triphosphate (Trilink, N-1081-1) and addition of 1:50 SUPERase-In RNase inhibitor [ThermoFisher Scientific, AM2694]. All linear mRNA constructs were co-transcriptionally capped by replacing GTP to a final concentration of 2 mM GTP and 8 mM 3'-O-Me-m7G(5')ppp(5')G RNA anti-reversed cap analogue (ARCA) [NEB, S1411S] for SP6 promoter constructs. T7 constructs were cotranscriptionally capped with CleanCap Reagent AG [Trilink, N-7113]. Following IVT reaction, DNA templates were digested by TURBO DNase and purified using MEGAclear Transcription Clean-Up Kit [ThermoFisher Scientific, AM1908]. mRNA concentrations were quantified using the Qubit RNA HS Assay [ThermoFisher Scientific, Q32852] immediately prior to use. Unless otherwise specified, mRNA products are suspended in 1:50 (v/v) RNase inhibitor-containing RNase-free water (subsequently referred to as RNase-free water) and stored at -80 °C.

### Chemical Conjugation of Modified Synthetic poly(A) Oligonucleotides

Chemically modified poly(A) oligonucleotides were obtained from IDT and suspended in RNase-free water to a final concentration of 100 µM.

For thiol-ene/yne conjugation, disulfide-protected thiol-oligos were deprotected with 100 molar access of TCEP (tris(2-carboxyethyl)phosphine) and immediately mixed with equimolar alkene/alkyne modified oligos and incubated at 37 °C for 30 minutes to 1 hour. In cases where radical conditions were used, substoichiometric amounts of 2,2-dimethoxy-2-phenylacetophenone were added and incubated at room temperature under 370 nm radiation [Kessil, KSPR160L370].

For amine-phosphate conjugation, oligos were mixed in excess amounts of imidazole with 1~2 equivalents of EDC as an additive. The reaction mixture was incubated at 37 °C for 30 minutes to 1 hour.

For IEDDA (inverse demand Diels-Alder reaction) conjugation, methyl tetrazine (Me-Tz) and transcycle-octene (TCO) labeled oligos were obtained from corresponding amine-modified oligos upon labeling with Tetrazine-PEG5-NHS Ester (Click Chemistry Tools, 1143) or TCO-PEG4-TFP Ester [Click Chemistry Tools, 1198] overnight at 4 °C in 100 mM NaHCO3 with a molar ratio of 500:1 (small molecule:oligonucleotides). NHS-labeling products were purified using ethanol precipitation. Me-Tz/TCO-labeled oligos were suspended in RNase-free water and incubated at 55 °C for 30 minutes.

For CuAAC (copper-catalyzed azide-alkyne conjugation) conjugation, azide/alkyne-containing oligonucleotides were mixed with indicated molar ratio (1:1 for one-branched oligo, 2:1 for di-branched oligo, and 3:1 for tri-branched oligo). The oligonucleotide mix was diluted in a modified 1.5× click chemistry buffer (Lumiprobe, 61150, with 5% SUPERase Inhibitor, 5% DMSO, and 5% 10 mM dNTP mix [ThermoFisher Scientific, 18427089]) that was briefly degassed by argon purging for 20 mins prior to the reaction. For a typical 100 µL reaction, 33 µL of oligonucleotide solution was mixed with 66 µL of click chemistry buffer and 2 µL of 100 mM L-ascorbic acid solution [Sigma Aldrich, A5960] was added immediately prior to the reaction. The mixture was incubated at 37 °C for 1 hr and the reaction was stopped by addition of 1 µL of 500 mM EDTA (pH 8.0). The reaction was first purified using Monarch RNA Cleanup Kit [NEB, T2040] and the crude products were repurified using RNase-free HPLC on an Agilent 1260 Infinity II HPLC with acetonitrile [Sigma Aldrich, 34851] and 100 mM hexylamine/acetic acid (pH 7.0, with 10% urea w/v) as mobile phase. HPLC fractions were analyzed by Novex TBE Urea gels, stained by 1× SYBR Gold [ThermoFisher Scientific, S11494], and visualized using BioRad ChemiDoc MP Imaging System [12003154]. Desired fractions were then pooled, desalted, and concentrated using Monarch RNA Cleanup Kit for small-scale preparations or ethanol precipitation for large-scale preparations.

### Enzymatic Ligation of Modified Oligonucleotides to mRNAs

Synthetic oligos and mRNA were mixed at a molar ratio of 100:1, and diluted in 2.5× 50% PEG-8000, 10× T4 RNA ligase buffer, 8× T4 RNA ligase [Promega, M1051], and RNase-free water. The reaction was incubated at 37 °C for 45 mins and inactivated by the addition of 50× 500 mM EDTA (pH 8.0). Products were purified by RNA Clean XP [Beckman Coulter] per manufacturer's protocols, with the exception of using 1 volume of beads. For large-scale reactions, ligation products were HPLC purified and residual modified oligos were recovered. Ligation products were characterized by RNase H assays as described before. In cases of residual oligonucleotides detected, a second purification was performed. Eluted mRNAs were quantified with Qubit, suspended in RNase-free water, and stored at -80 °C. For each batch of mRNA ligation, a "mock ligation" condition was included where all reaction set-ups were the same except the addition of the synthetic oligo, and the recovered product was used as the linear mRNA control.

### mRNA Modification Screening with Time-course Dual Luciferase Assay

HeLa cells (CCL-2, ATCC) were maintained in DMEM culture media (ThermoFisher Scientific, 119951) containing 10% FBS and 1% penicillin-streptomycin (ThermoFisher Scientific, 15070063) in a 37 °C incubator with 5% CO2 and passaged at a ratio of 1:10 every 3 days. On the day before mRNA transfection, HeLa cells were seeded at 75% confluence in individual wells on three white clear-bottom 96-well plates [Corning, 3610]. The following day, 30 ng of Renilla luciferase (internal control) mRNA and 30 ng of modified Firefly luciferase mRNA were transfected using Lipofectamine MessengerMAX Transfection Reagent [ThermoFisher Scientific, LMRNA003] per manufacturer's protocols. Additional controls that contain only Renilla luciferase mRNA or lipofection reagent only were included. Three individual transfections were conducted for each condition. At 24/48/72 hours post-transfection, cell culture media was removed and cells were rinsed with 1xDPBS. Cells were lysed and luciferase activity was measured using the Promega Dual Glo Luciferase Assay System [Promega, E2920]. Briefly, 50 µL of PBS and 50 µL of Firefly luciferase working solution (prepared following manufacturer's protocols) were added to each well using multichannel pipette and mixed by pipetting. After 10 mins incubation with gentle shaking and protection from light at room temperature, Firefly luciferase luminescence was measured using a microplate reader. 50 µL of freshly prepared Renilla luciferase Stop&Glow working solution (prepared following manufacturer's protocols) was added. Renilla luciferase luminescence was measured similarly after 10 minutes incubation. For both Firefly and Renilla luminescence, background was measured by cells treated with only lipofectamine reagent and subtracted. Firefly luminescence/Renilla luminescence for each well was used as mRNA activity readout.

### Firefly Luciferase Degron Assay

HeLa cells were seeded at 75% confluency in individual wells on a 24-well plate the day before mRNA transfection. The following day, 200 ng of unmodified Renilla luciferase (internal control) mRNA and 200 ng of Firefly-degron mRNA bearing designed modifications were transfected into each well using Lipofectamine MessengerMAX per manufacturer's protocols. After a 6-hour incubation, cell culture media containing the transfection mixture was removed, and cells were rinsed with 1xDPBS and trypsinized to reseed into four white clear bottomed 96-well plates at a ratio of 8:6:4:3, respectively, for luciferase quantification at 8, 24, 48, 72 hours. Luciferase was quantified at each time point using the aforementioned protocols. For each time point, Firefly luciferase activity was normalized by cell number (reseeding ratio) and then by luminescence activity using Firefly luminescence / Renilla luminescence * Average Renilla luminescence; calculated firefly luciferase luminescence was normalized against 8-hour readouts.

### mRNA Quantification in Transfected Cell Culture Using STARmap/RIBOmap

HeLa cells were seeded at 75% confluency in individual wells on a 24-well plate the day before mRNA transfection. The following day, 500 ng of unmodified Renilla luciferase (internal control) mRNA and 500 ng of Firefly-degron mRNA bearing designed modifications were transfected into each well using Lipofectamine MessengerMAX per manufacturer's protocols. After a 6-hour incubation, the transfection mixture was removed, and cells were rinsed with 1xDPBS and trypsinized to reseed into six (two for each time point) glass-bottom 96-well plates (MatTek, PBK96G-1.5-5-F, poly-D-lysine [Sigma-Aldrich, A-003-M] coated) at a ratio of 6:4:3, respectively, for in situ sequencing quantification at 24, 48, and 72 hours after transfection. Each well of transfected cells were reseeded equally onto two 96-well plates for each time point with one measured by STARmap (Firefly) + STARmap (Renilla) and the other measured by RIBOmap (Firefly) + STARmap (Renilla).

For quantification of mRNA quantities, we followed the STARmap/RIBOmap procedure for cell cultures as published (Wang et al. "Three-dimensional intact-tissue sequencing of single-cell transcriptional states" (2018) Science 361(6400), eaat5691; Hu et al., "Spatially Resolved Single-cell Translatomics at Molecular Resolution" (2022) bioRxiv)*.* Briefly, at 24/48/72 hours post transfection, culture media was removed, and cells were washed with 150 µL PBS solution. Cells were then fixed with 1.6% paraformaldehyde (Electron Microscope Sciences, 15710-S)/1XPBS (Gibco, 10010-023) at room temperature for 15 minutes before further fixation and permeabilization with prechilled methanol at -20 °C for one hour. Subsequently, the methanol was removed, and the cells were rehydrated with PBSTR/Glycine/YtRNA (PBS with 0.1%Tween-20 [TEKNOVA INC, 100216-360], 0.5% SUPERaseIn [Invitrogen, AM2696], 100 mM glycine, 1% yeast tRNA) at RT for 5 minutes followed by one PBSTR wash. Samples were then hybridized with SNAIL probes targeting Firefly and Renilla luciferase mRNA sequences in the hybridization buffer ((2XSSC [Sigma-Aldrich, S6639], 10% formamide [Calbiochem, 655206], 1% Tween-20, 20 mM RVC [ribonucleoside vanadyl complex, New England Biolabs, S1402S], 0.5% SUPERaseIn, and 1% yeast tRNA, 100 nM each probe) in a 40 °C humidified oven with shaking and parafilm wrapping overnight (see Supplementary Table 2 for probe sequences). For RIBOmap quantification, SNAIL probes targeting Firefly luciferase were replaced with RIBOmap primer/padlock probes and splint probes targeting 18S rRNA, with the rest of the protocols remaining the same. After hybridization, the cells were then washed with PBSTR twice at 37 °C (20 minutes each wash) and high salt wash buffer (PBSTR with 4XSSC) once at 37 °C before rinsing once with PBSTR at RT. A ligation reaction was performed for 2 hours at room temperature to circularize padlock probes that were adjacent to the primer in ligation buffer (1XT4 DNA Ligase buffer, T4 DNA ligase, 0.1 Weiss U/µL [ThermoFisher Scientific, EL0012], 0.5 mg/mL Ultrapure BSA [ThermoFisher Scientific, AM2618], 0.5% SUPERaseIn). Cells were washed twice with PBSTR and rolling circle amplification (RCA) was carried out in RCA buffer (1XPhi29 buffer, 0.2 Weiss U/µL Phi29 DNA polymerase [ThermoFisher Scientific, EP0094], 250 uM dNTP mix [ThermoFisher Scientific, 18427089], 0.5 mg/mL BSA, 0.5% SUPERaseIn) at 30 °C for 2 hours, followed by two washes with PBST. Samples were then stained with fluorescent detection oligo in the wash and imaging buffer (2XSSC, 10% formamide, 100 nM per detection probe, 1xDAPI [Molecular Probes, D1306]) at room temperature for 1 hour (see Supplementary Table 2 for fluorescent detection probe sequences). Confocal imaging stacks were taken with a Leica Stellaris 8 with a 60× oil objective at a pixel size of 283 × 283 nm. A 14-µm stack is imaged with 2 µm/step × 7 steps. Three independent transfections were conducted for each condition, of which at least four representative FOVs were taken. The same imaging setting was used for all the samples to be compared. Excitation/detection wavelengths are as follows: DAPI, Diode 405 nm/~[420-489] nm; Alexa546, WLL 557 nm/~[569-612] nm; Alexa647, WLL 653 nm/~[668-738] nm.

MATLAB 2021a and CellProfiler 4.0.7 were used for the amplicon count-based fluorescence image analysis. First, the centroids of amplicons in each fluorescent channel (Firefly, Renilla) were identified by finding extended maxima on images. Then a 3*3*3 voxel volume centering the centroid of each fluorescent dot was defined. Within each voxel volume, the integrated intensities in the Firefly and Renilla channels were calculated, and the ratio between Firefly intensity and Renilla intensity was used for amplicon classification. For STARmap quantification, all the measurements were pooled together and the distribution of log(Firefly/Renilla) values were plotted. The corresponding ratio values at the nadirs (local minimum) on the distribution plot were identified as cutoff values. The first cutoff value smaller than 0 was noted as cutoff1, and the first cutoff value greater than 0 was noted as cutoff2. For RIBOmap quantification, cutoff1 was assigned as -0.3 and cutoff2 was assigned as 0.3. Any amplicon with a log(Firefly/Renilla) value smaller than cutoff1 was identified as a Renilla amplicon. Any amplicon with a log(Firefly/Renilla) value larger than cutoff2 was identified as a Firefly amplicon. Any amplicon with a log(Firefly/Renilla) value between cutoff1 and cutoff2 was identified as a granule. Amplicon classification information, as well as the location of every amplicon, was saved in a file. In each figure, the ratio between the number of Firefly amplicons and the number of Renilla amplicons were calculated and used to reflect the amount of Firefly luciferase mRNAs. In single-cell STARmap quantification, cell segmentation was performed using the same method as cell segmentation in single-cell protein quantification (see GFP Ablation Using Cas9 mRNA), and the segmentation masks were saved as uint16 images. We then assigned amplicons to cells according to where they were located on the masks. The ratio between the number of Firefly amplicons and the number of Renilla amplicons in each cell was calculated and used to reflect the amount of Firefly luciferase mRNAs in a single cell. Cells with no Firefly amplicons or no Renilla amplicons were considered unsuccessfully transfected and thus excluded from our analyses.

### EMSA (electrophoretic mobility shift assay/gel shift assay)

A codon-optimized DNA sequence encoding full length human PABPC1 (residues 1-636) (GST tagged) was cloned into pBluescript II KS(-) vector by GenScript. Transformed Escherichia coli cells [BL21 Competent E. coli, New England Biolabs] were grown in 1 L Luria Bertani medium in the presence of 50 mg ampicillin to an absorbance at 600 nm of 0.6-0.8 at 37 °C. The expressions of glutathione S-transferase (GST)-fusion proteins were induced by addition of 0.5 or 1 mM isopropyl β-D-1-thiogalactopyranoside and cells were grown overnight at 18 °C. The cells were harvested by centrifuge and disrupted by sonication in 30 mL lysis buffer (10 mM Na2HPO4 (pH 7.4), 1.8 mM KH2PO4, 637 mM NaCl, 2.7 mM KCl, 10% glycerol, 1 mM phenylmethylsulfonyl fluoride, 0.5 mM 4-(2-aminoethyl) benzenesulfonyl fluoride hydrochloride, 0.15 µM aprotinin, 1 µM E-64 and 1 µM leupeptin) with 0.15% polyethylenimine for removal of nucleic acids in lysate. Cell debris was removed by centrifuge. The supernatants were applied to GlutathioneSepharose 4B column [GE Healthcare] equilibrated with PBS at 4 °C, and washed using PBS at 4 °C. The GST-fusion proteins were eluted with the elution buffer (100 mM Tris-HCl (pH 7.8), 300 mM NaCl and 20 mM glutathione 3 (reduced form)). Eluted solution of isolated PABPC1-GST was concentrated using spin filters and concentration of the protein was quantified using Qubit Protein Broad Range (BR) Assay Kits [Invitrogen, Q33211]. Branched/Linear poly(A) oligonucleotides labeled at 5'-end with AlexaFluor 546 were synthesized as described in the earlier sections. Poly(A) oligos were incubated in the binding buffer (10 mM Na2HPO4, 1.8 mM KH2PO4, 137 mM NaCl, 2.7 mM KCl, 5 mM MgSO4, 1 mM DTT, Novex Hi-Density TBE Sample Buffer [Invitrogen, LC6678]) with varying PABPC1-GST concentrations prepared by sequential dilutions from the stock solution. For the competitive binding assay, the concentration of Alexa 546-labeled branched oligo and PABPC1-GST were kept constant while varying concentrations of unlabeled linear poly(A) oligos were added. The oligos and PABP were incubated at 4 °C for 30 mins and characterized by EMSA with Novex TBE Gels, 4-20% [Invitrogen, EC6225BOX] at 4 °C. Alexa 546 labeled oligos were imaged with BioRad ChemiDoc MP Imaging System [12003154] and band intensities were quantified using ImageJ. For Kd calculation, the percentage of poly(A) oligos bonded to x mers of PABP were calculated as the total percentages of oligos bonded to >x mers of PABP and Kd was calculated by plotting percentage of PABP-bonded poly (A) oligos against PABP concentration using GraphPad Prism (Specific binding with Hill slope).

### GFP Ablation Using Cas9 mRNA

Linear/branched Cas9 mRNAs were synthesized using the aforementioned protocols. sgRNAs were designed and synthesized using the IDT sgRNA design tool [IDT, Alt-R CRISPR-Cas9 sgRNA]. GFP expressing HEK293 cells [GenTarget Inc, SC058] were seeded at 50% confluency in individual wells on a 12-well plate the day before mRNA transfection. The following day, cells were co-transfected with 100 ng sgRNA and varying amounts of linear/branched Cas9 mRNA using Lipofectamine MessengerMAX. After a 48-hour incubation, cell culture media was removed, and cells were rinsed with 1xDPBS, trypsinized, and reseeded onto glass-bottom 12-well plates [MatTek, P12G-1.5-14-F, poly-D-lysine coated]. 72 hours post-transfection, the culture media was removed, and the cells were rinsed with 1xDPBS once before being incubated in the nuclei staining media (FluoroBrite DMEM [ThermoFisher, A1896701] with 1:2000 dilution of Hoechst 33342 [ThermoFisher, 62249]) at 37 °C for 10 min. Confocal images of the nuclei (Hoechst) and GFP were taken by Leica Stellaris 8 with a 10× air objective at a pixel size of 1135 × 1135 nm. A 14-µm stack was imaged with 2 µm/step × 7 steps. At least six representative FOVs were taken for each condition tested and the same imaging setting was used for all samples to be compared. Excitation/detection wavelengths were as follows: Hoechst, Diode 405 nm/~[430-480] nm; GFP, WLL 489 nm/~[500-576] nm.

Analyses were performed on the maximum projection images of the raw image stacks. CellProfiler 4.0.7 was used for single-cell protein quantification. For single-cell analyses, first, Hoechst-stained nuclei were identified as primary objects. Then, the Hoechst channel and GFP channel were merged and subsequently converted to a grayscale image. Cells were identified as secondary objects in this grayscale image. Following cell segmentation, GFP intensity in each cell was measured.

### Circular RNA Synthesis

Sequences of circular RNA encoding secretive NanoLuc with translation-enhancing UTRs (iHRVB3 + PABPv3) were adopted from previous studies (Chen et al., "Engineering circular RNA for enhanced protein production" Nature biotechnology (2022) 1-11). DNA templates were synthesized from Genewiz, PCR amplified and gel purified to be used as IVT templates. CircRNA was synthesized by using the HiScribe T7 High Yield RNA Synthesis Kit [New England Biolabs, E2040S]. Post IVT, DNA templates were digested with Turbo DNase [ThermoFisher, AM2238]. The reaction mixture was heated to 70 °C for 5 minutes and then immediately placed on ice for 3 minutes, after which, GTP was added to a final concentration of 2 mM, and the reaction mixture was incubated at 55 °C for 15 minutes. CircRNA was enriched by treatment with RNase R [Lucigen Corporation, RNR07250] for 1.5 hours, and the products were column purified. CircRNA products were characterized by gel electrophoresis, and in cases of remaining linear RNA precursors, another round of RNase R digestion was performed. Precursor and product patterns were in agreement with previous reports.

### In Vitro Secretive NanoLuc Assay

HeLa cells were seeded at 75% confluency in individual wells on a 96-well plate the day before mRNA transfection. To minimize the influence of phenol red on the assay, FluoroBrite DMEM supplemented with 10% fetal bovine serum and 4 mM L-glutamine was used for culturing. The following day, equal molars of linear/branched/circular RNA (corresponding to 50 ng, 59 ng, and 90 ng of mRNA, respectively) were transfected into the cells using lipofectamine. Media was exchanged 6 hours post-transfection to 150 µL of aforementioned FluoroBrite media. On each day, old media was all replaced with a new aliquot of 150 µL FluoroBrite media for each well, and 50 µL of the old media was used for bioluminescence measurement using Nano-Glo Luciferase Assay System [Promega, N1110]. At least three independent transfections were performed for each condition tested and background luminescence was subtracted from each well. The assay was run for a total of 14 days.

### Mice

All animal procedures followed animal care guidelines approved by the Institutional Animal Care and Use Committee (IACUC) of the Broad Institute of MIT and Harvard under animal protocol # 0255-08-19. Animal experiments were conducted in compliance with IACUC policies and NIH guidelines.

### In Vivo Delivery of mRNA

The BALB/c (male and female, 6-8 weeks old) mice used for in vivo NanoLuc assays in this study were purchased from The Jackson Laboratory (JAX). Branched and linear mRNAs encoding NanoLuc were formulated into LNPs using NanoAssemblr Spark Kit - Hepato9 mRNA [Precision Nanosystems, NWS0016] on a NanoAssemblr Spark instrument [Precision Nanosystems] per manufacturer's protocol. Upon formulation, mRNA-LNPs were diluted in 10 mL 1xPBS solution and the buffer was exchanged by concentrating with a 30 kDa spin filter [MilliporeSigma, UFC901008] to remove residue ethanol. Concentrations of mRNA were determined using Quant-it RiboGreen RNA Assay Kit [ThermoFisher, R11490] and encapsulation efficiencies were higher than 95%. 390 pg per nucleotide of linear/circular mRNA-LNP (corresponds to 350 ng of linear mRNA) was injected immediately through retro orbital injection into each mice at an injection volume of 100 µL. Three male mice and three female mice were used for each condition.

### In Vivo Imaging of NanoLuc

In vivo activity of NanoLuc in mice was measured using a Competent IVIS-Perkin Elmer IVIS Spectrum CT System [Perkin Elmer]. At each time point, mice were anesthetized with isoflurane. The fluorofurimazine substrate [Promega] was freshly reconstituted in 1.05 ml of PBS per vial and was injected into the mice at a dose of 44 nmol per gram body weight. Mice were imaged 5 minutes post injection using default settings. The luminescent activity was quantified using Aura 4.0 imaging software. The background was determined by including mice injected with poly C-LNP negative control and subtracted from each measurement.

### In Vivo Toxicity Assay

To evaluate in vivo toxicity and immune response, blood was collected and proceeded to serum from mice on day 15 post mRNA-LNP administration. AST, ALT, and TNF-alpha were measured using ELISA kits for AST [G-Biosciences, IT5530], ALT [G-Biosciences, IT5508], and TNF-alpha [R&D Systems, MTA00B] per manufacturer's protocols.

### EXAMPLE 1. Production of poly-tailed and poly-capped mRNAs.

3' poly-A tails and 5' caps are both required for efficient translation of eukaryotic mRNA molecules (FIG. 1A). However, both poly-A tails and 5' caps are vulnerable to degradation by exonucleases that remove nucleotides from the mRNA molecule (FIG. 1B). Once exonucleases degrade the coding portion of an mRNA (e.g., a protein coding sequence), the mRNA is unable to be effectively translated. Strategies to enhance mRNA stability include methods of modifying mRNA molecules to become resistant to exonuclease activity. Exonuclease resistance may be conferred to mRNA molecules by modifying mRNA to include additional poly-A tails and/or 5' caps. Additional poly-A tails and 5' caps may be introduced to mRNA molecules through any number of techniques.

In one technique, for example, additional poly-A tails may be introduced to an mRNA molecule (e.g., a mRNA isolated from a cell or organism, or an mRNA molecule produced by in vitro transcription (IVT)) by ligating the 3' end of the mRNA to a "brushed" poly-A tail oligonucleotide comprising an internal covalent attachment to either the 3' end or the 5' end of one or more second nucleic acid sequences (FIG. 1C). A brushed poly-A tail oligonucleotide can be produced by starting with a poly-A oligonucleotide modified with several internal nucleotides capable of a click chemistry reaction (e.g., 5-Octadiynyl dU), and attaching to these nucleotides additional poly-A oligonucleotides comprising a 3' azide moiety (FIG. 1D). For additional exonuclease resistance, any of the oligonucleotides may be modified with one or more modified nucleotides or internucleotide linkages, such as a phosphorothioate linkage. For example, the first poly-A oligonucleotide may comprise a sequence of /5Phos/rArArA rArArA /i5OctdU/rArA rArArA rArArA rArArA rA/i5OctdU/rA rArArA rArArA rArArA rArA/i5OctdU//3ddC/ (SEQ ID NO: 7), to which oligonucleotides are attached comprising a sequence of /5Phos/ rArArA rArArA rArArA rArArA rArArA rArArA rArArA rArArA rA*rA*rA* rA*rA*rA* /3AzideN/ (SEQ ID NO: 8), where "*" indicates a phosphorothioate linkage. After assembly through the click chemistry reaction (e.g., Cu(I)-catalyzed azide-alkyne cycloaddition), the 5' end of the brushed poly-A oligonucleotide can then be ligated to the 3' end of an mRNA molecule, which may or may not already comprise a poly-A tail, using an RNA ligase (e.g., T4 RNA ligase).

Alternately, additional poly-A tails may be introduced to an mRNA molecule by ligating the 3' end of the mRNA to a dendrimer that has been modified with poly-A tails (FIG. 1E). A dendrimer comprising poly-A tails may be produced by first functionalizing a dendrimer (e.g., a PAMAM dendrimer; CAS number: 1174157-65-3) with alkyne groups. This functionalization can be achieved by nucleophilic substitution between propargyl-N-hydroxysuccinimidyl ester (CAS number: 1174157-65-3) and the amino groups on the dendrimer is efficient under mild conditions. The functionalized dendrimer can then be attached to poly-A tail oligonucleotides which comprise a 3' azide moiety through a click chemistry reaction (FIG. 1F). For additional exonuclease resistance, any of the oligonucleotides may be modified with one or more modified nucleotides or internucleotide linkages, such as a phosphorothioate linkage. For example, poly-A oligonucleotides may comprise a sequence of /5Phos/ rArArA rArArA rArArA rArArA rArArA rArArA rArArA rArArA rA*rA*rA* rA*rA*rA* /3AzideN/ (SEQ ID NO: 8), where "*" indicates a phosphorothioate linkage. The 5' end of one of the poly-A oligonucleotides attached to the dendrimer through a click chemistry reaction (e.g., Cu(I)-catalyzed azide-alkyne cycloaddition) can then be ligated to the 3' end of an mRNA molecule, which may or may not already comprise a poly-A tail, using an RNA ligase (e.g., T4 RNA ligase).

Orthologous pathways may also be used to produce a poly-capped mRNAs. For example, a poly-capped mRNA may be produced by ligating the 5' end of a mRNA to a "brushed" 5' capping oligonucleotide (FIG. 1G). Similar to the way in which a brushed poly-A tail oligonucleotide is produced, a brushed poly-capped oligonucleotide can be produced by starting with a poly-capped oligonucleotide modified with several internal nucleotides capable of a click chemistry reaction (e.g., 5-Octadiynyl dU), and attaching to these nucleotides additional poly-capped oligonucleotides comprising a 3' azide moiety (FIG. 1H). For additional exonuclease resistance, any of the oligonucleotides may be modified with one or more modified nucleotides or internucleotide linkages, such as a phosphorothioate linkage. The 5' caps may be 7-methylguansine (m7G) caps, or modified caps, e.g., caps comprising modified nucleotides or internucleotide linkages. For example, the first poly-capped oligonucleotide may comprise a sequence of 5'-/Cap/rGrGrG rArArA /i5OctdU/rArA rGrArG rArGrA rArArA rGrArA rGrArG /i5OctdU/rArA rGrArA rGrArA rA/i5OctdU/rA (SEQ ID NO: 9), to which oligonucleotides are attached comprising a sequence of 5'-/Cap/rGrGrG rArGrA rCrTrG rCrCrA rCrCrA* rA*rA*rA* rA*rA* /AzideN/-3' (SEQ ID NO: 6), where "*" indicates a phosphorothioate linkage. After assembly through the click chemistry reaction (e.g., Cu(I)-catalyzed azide-alkyne cycloaddition), the 3' end of the brushed poly-A oligonucleotide can then be ligated to the uncapped 5' end of an mRNA molecule using an RNA ligase (e.g., T4 RNA ligase).

Alternately, additional 5' caps may be introduced to an mRNA molecule by ligating the 5' end of the mRNA to a dendrimer that has been modified with 5' caps (FIG. 1I). Similar to the way in which a dendrimer that has been modified with poly-A tails is produced, a dendrimer comprising 5' caps may be produced by first functionalizing a dendrimer (e.g., a PAMAM dendrimer; CAS number: 1174157-65-3) with alkyne groups. This functionalization can be achieved by nucleophilic substitution between propargyl-N-hydroxysuccinimidyl ester (CAS number: 1174157-65-3) and the amino groups on the dendrimer is efficient under mild conditions. The functionalized dendrimer can then be attached to 5' capped oligonucleotides which comprise a 3' azide moiety through a click chemistry reaction (FIG. 1J). An uncapped oligonucleotide comprising a 5' azide is also attached to the functionalized dendrimer. The rate of attachment to the dendrimer between capped oligonucleotides comprising a 3' azide moiety and uncapped oligonucleotides comprising a 5' azide moiety may be controlled by adjusting the stoichiometry between the oligonucleotides. For additional exonuclease resistance, any of the oligonucleotides may be modified with one or more modified nucleotides or internucleotide linkages, such as a phosphorothioate linkage. The 5' caps may be 7-methylguansine (m7G) caps, or modified caps, e.g., caps comprising modified nucleotides or internucleotide linkages. For example, 5' capped oligonucleotides may comprise a sequence of 5'-/Cap/ rGrGrG rArGrA rCrTrG rCrCrA rCrCrA* rA*rA*rA* rA*rA* /AzideN/-3' (SEQ ID NO: 6), while the uncapped oligonucleotide may comprise a sequence of /5AzideN/rArArA rArA, where "*" indicates a phosphorothioate linkage. The 3' end of an uncapped oligonucleotides attached to the dendrimer through a click chemistry reaction (e.g., Cu(I)-catalyzed azide-alkyne cycloaddition) can then be ligated to the 5' end of an uncapped mRNA molecule using an RNA ligase (e.g., T4 RNA ligase).

Modified mRNA molecules comprising additional poly-A tails may also be modified to comprise additional 5' caps, and vice versa. In addition to having enhanced resistance to exonucleases, mRNA molecules that are modified with additional poly-A tails and/or 5' caps may also have enhanced translation efficiency relative to unmodified mRNA molecules. For example, the addition of one or more additional poly-A tails or 5' caps to a mRNA molecule may enhance binding between the mRNA molecule and one or more components of eukaryotic translation machinery, such as poly-A binding protein (PABP) and the eukaryotic initiation factor 4E (eIF4E) cap-binding protein of the eukaryotic initiation factor 4 complex (eIF4F) (FIGs. 1C, E, G, and I).

### EXAMPLE 2. Effect of multiple poly(A) tails on mRNA translation in vitro.

To preliminarily test the translation efficiency of mRNA molecules modified with additional poly(A) tails, mRNAs encoding fluorescent or luminescent proteins were modified with brushed poly-A tail oligonucleotides as described in Example 1. Briefly, mRNA molecules encoding either a constitutively fluorescent variant of green fluorescent protein (hMGFP) or firefly luciferase were ligated to a brushed poly-A oligonucleotide precursor, a brushed poly-A oligonucleotide precursor to which poly-A oligonucleotides with a 5' azide were attached, or a brushed poly-A oligonucleotide precursor to which poly-A oligonucleotides with a 3' azide where attached (Table 1). Mock ligations conducted with mRNA and no oligo were also tested, as well as mRNA without treatment. Each mRNA was transfected into human HeLa cell culture, along with a constant amount of a control mRNA. Transfections with mRNA encoding hMGFP were co-transfected with mRNA encoding mCherry. Transfections with mRNA encoding firefly luciferase, were co-transfected with Renilla luciferase. Ratios of hMGFP/mCherry fluorescence and firefly luciferase/Renilla luciferase were measured 24 hours, 48 hours, and 72 hours after transfection and used to assess translation efficiency from the transfected mRNA.

Similar ratios of fluorescence and luminescence were measured in cells transfected with untreated mRNA or mock ligated mRNA, which were generally unchanged over the 72 hour time course (FIGs. 1K, L). Cells transfected with mRNAs ligated only to the brush oligo precursor demonstrated an increase in fluorescence/luminescence after 48 hours and 72 hours, indicating that ligation to the brushed poly-A tail precursor enhanced mRNA stability and translation efficiency. This effect was further enhanced by addition of poly(A) oligonucleotides comprising a 5' azide moiety to the brushed poly(A) tail precursor, indicating that the number of poly-A tails attached to an mRNA is positively correlated with stability and translation efficiency of the mRNA. However, attachment of poly-A oligonucleotides comprising a 3' azide moiety to the brushed poly-A tail precursor diminished the fluorescence/luminescence observed. This result was not surprising, as attachment of the poly-A oligonucleotides to the brushed poly-A tail precursor via a 3' azide would cause the brushed oligonucleotide to have uncapped 5' phosphates at its ends, rather than a consecutive set of phosphorothioate-modified adenosine ribonucleotides, which is located at the 3' end. Therefore, orienting the azide-modified poly-A oligonucleotide in this way would be expected to cause the poly-A oligonucleotides to be vulnerable to exonucleases.

It should also be noted that the ratio of hMGFP to mCherry fluorescence was observed to be stable after 72 hours post-transfection, while the ratio of firefly luciferase to Renilla luciferase luminescence was observed to still be increasing after 72 hours (FIGs. 1K, L). These results indicate that translation from mRNAs modified with additional poly-A tails is long lasting and could therefore be of use in in vivo applications (e.g., prophylactic and therapeutic uses).

**Table 1: Oligonucleotides for synthesis of brushed poly-A tail oligonucleotides**

| Oligonucleotide name | SEQ ID NO. | Sequence |
|---|---|---|
| Brushed oligo precursor | 10 | /5Phos/rArArA rArArA /i5OctdU/rArA rArArA rArArA rArArA rA/i5OctdU/rA rArArA rArArA* rA*rA*rA* rA*rA*/i5OctdU/ /3ddC/ |
| 5' azide poly-A oligo | 11 | /5AzideN/rArArA rArArA rArArA rArArA rArArA rArArA rArArA rArArA rA*rA*rA* rA*rA*rA* /3ddC/ |
| 3' azide poly-A oligo | 8 | rArArA rArArA rArArA rArArA rArArA rArArA rArArA rArArA rA*rA*rA* rA*rA*rA* /3AzideN/ |

wherein "/5Phos/" refers to a 5' phosphate group, "rA" refers to an adenosine ribonucleotide, "/i5OctdU/" refers to an internal 5-Octadiynyl uracil deoxyribonucleotide, "3ddC" refers to a 3' cytosine dideoxyribonucleotide, "/5AzideN/" refers to a 5' azide moiety, "/3AzideN/" refers to a 3' azide moiety, and "*" refers to a phosphorothioate internucleotide linkage. Internucleotide linkages that are not specifically indicated are phosphodiester internucleotide linkages.

### EXAMPLE 3. Development of chemically modified and topologically novel capped, branched mRNA-oligo conjugates bearing multivalent poly(A) tails.

In the most widely accepted model of cap-dependent translation mechanism, the rate-limiting step is the assembly of the translation initiation complex (TIC) centered around the m7G cap by eukaryotic translation initiation factors (eIFs) including eIF4E and eIF4G (FIG. 1A, left). The TIC is further stabilized through the interaction of eIF4G with cytoplasmic poly(A)-binding proteins (PABPCs), which recognizes the 3'poly(A) tail, forming a closed-loop structure (FIG. 1A, right). PABPCs therefore serve a dual role of initiating translation through engagement with eIFs and extending mRNA stability by cloaking the mRNA from deadenylation. It has been shown that site-specific introduction of exonuclease-resistant modifications at the end of poly(A) tails efficiently increases mRNA stability and protein production in cellulo, presumably via stabilizing the nascent poly(A) tail and maintaining poly(A)-PABPCs interaction. Additionally, structural studies have revealed that PABPCs function as a multimeric protein complex where individual PABPC1 proteins binding the poly(A) tail form a stabilized complex. This structural insight prompted the investigation of achieving enhanced, multivalent interactions of the mRNA transcripts with PABPC1 through multimerization of the poly(A) tail via a branched topology, with each individual poly(A) tail bearing extensive nuclease resistant modifications (FIG. 2A).

Branched mocRNAs were developed based on ligating chemically synthesized oligonucleotides with dense modifications to the 3' end of in vitro transcribed (IVT) mRNA using T4 RNA ligase. Various synthetic strategies for generating the branched poly(A) oligonucleotides were tested. Branched RNA topology can occur naturally in the form of lariat intron RNA, a byproduct of pre-mRNA splicing with a "branched" linkage between 2'-hydroxyl group of an internal nucleotide and the 5'-end of the spliced intron, which is rapidly cleared by debranching enzymes. The screening was thus focused on unnatural branching linkages that cannot be hydrolyzed inside cells, and that have high kinetics at micromolar concentrations for the ease of large-scale synthesis. Several oligonucleotide crosslinking chemistries were tested, including thiol-ene and thiol-yne reactions, a phosphate-amine based reaction, as well as click chemistry methods like tetrazine-transcyclooctene (Tz-TCO) and copper-catalyzed azide-alkyne cycloaddition (CuAAC) (FIG. 2B). While the thiol-ene and thiol-yne reactions had low conversion under non-radical conditions and led to oligomerization when radical initiators were used, the phosphate-amine reaction produced trace amounts of product even with catalysis by EDC/imidazole. The Tz-TCO (IEDDA) and CuAAC reactions both worked well with high conversion, indicating CuAAC to be the preferred choice due to the ability to introduce alkyne handles on 5-octadiynyl deoxyU (EU) during solid-phase synthesis, whereas Tz-TCO (IEDDA) needs an extra step of functionalization (FIGs. 2C-D).

To further enable poly(A) oligo with multiple branches, a 30-nucleotide (nt) "stem" oligo was designed containing a 5'-phosphate for subsequent enzymatic ligation and multiple internal EU modification branching sites gapped by 12 A's for PABPC1 binding, paired with a 30 nt "branch" poly (A) oligo containing a 5' azide handle. Both the stem and the branch poly(A) contained a chain terminating dideoxycytidine (ddC) at the end for preventing self-ligation and phosphorothioate (PS) modifications on the last 6 nt for blocking deadenylation. CuAAC conjugation resulted in a mixture of 7 products with 1~3 branches in different topology as shown on gel electrophoresis (FIG. 2D). A preliminary test was done by ligating the crude branched product to a firefly luciferase reporter, showing a 4.6-5.3-fold increase in bioluminescence in the CuAAC-treated construct compared to the unligated control (p < 0.0001, ANOVA). However, reversing the direction of the branching poly(A)s completely eliminated the increase of luminescence, indicating a normal 5' to 3' directionality is still required on the branched tails (FIG. 2E).

### EXAMPLE 4. Establishment of a pipeline for branched mocRNA synthesis and purification.

A branched mocRNA synthesis and purification pipeline was established by incorporating HPLC purification for systematical screening of various chemical modifications moieties (FIGs. 3A-D). In this pipeline, 8 synthetic poly(A) oligos (shown in FIG. 3F) with different numbers of branches (zero to three) and different types of chemical modifications (PS (*/rA/), DNA (/dA/), and 2'-O-methoxyethyl (*/i2MOErA/)) were chemically assembled, HPLC purified, and enzymatically ligated onto a firefly luciferase reporter mRNA. After quality control of ligation yield using RNase H assay (FIG. 3E), the ligation products were transfected into HeLa cells with a Renilla luciferase mRNA as an internal transfection control, where the effects of modified, branched poly(A) tails on protein production were quantified by the ratio of Firefly/Renilla bioluminescence. Among all the constructs, Construct 8 shown in FIG. 3F, with 3 branches and synergistic incorporation of 2'-O-methoxyethyl (2MOE) and PS on both the stem and branch poly(A) oligos had the highest enhancements, yielding 4, 11, and 19-fold higher luminescent signals than linear mRNA at 24, 48, and 72 hours post transfection, respectively (p < 0.0001, ANOVA, FIG. 3F).

**Table 2: Branched poly(A) tail variants**

| Construct No. (FIG. 3F) | SEQ ID NO. | Sequence | Features |
|---|---|---|---|
| 1 | 12 | 60×rA (template encoded) | Ribose backbone |
| 2 | 13 | /5Phos/dAdAdAdAdAdA/i5OctdU/dAdAdAdAd AdAdAdAdAdAdAdA/i5OctdU/dAdAdAdAdAd AdAdAdAdAdAdA/i5OctdU//3ddC/ | 2-Deoxyribose backbone; 3x EU handles |
| 3 | 10 | /5Phos/rArArArArArA/i5OctdU/rArArArArArAr ArArArArArA/i5OctdU/rArArArArArArA*rA*r A*rA*rA*rA*/i5OctdU//3ddC/ | Ribose backbone; PS tail; 3x EU handles |
| 4 (Backbone) | 14 | /5Phos/rArArArArArArArArArArArArArArA rArArA/i5OctdU/rArArArArArArA*rA*rA*rA* rA*rA*rA/3ddC/ | Ribose backbone; PS tail; 1 triazole linkage; 1 branch with 2MOE tail |
| 4 (Branch) | 11 | /5AzideN/rArArArArArArArArArArArArA rArArArArArArArArArA*rA*rA*rA*rA*rA*/3 ddC/ | |
| 5 (Backbone) | 15 | /5Phos/rArArArArArA/i5OctdU/rArArArArArAr ArArArArArA/i5OctdU/rArArArArArArA*rA*r A*rA*rA*rA*rA/3ddC/ | Ribose backbone; PS tail; 2 triazole linkages; 2 branches with PS tails |
| 5 (Branches) | 11 | 2×/5AzideN/rArArArArArArArArArArArAr ArArArArArArArArArArArA*rA*rA*rA*rA*r A*/3ddC/ | |
| 6 (Backbone) | 15 | /5Phos/rArArArArArA/i5OctdU/rArArArArArAr ArArArArArA/i5OctdU/rArArArArArArA*rA*r A*rA*rA*rA*rA/3ddC/ | Ribose backbone; PS tail; 2 triazole linkages; 2 branches with 2MOE tails |
| 6 (Branches) | 16 | 2×/5AzideN/rArArArArArArArArArArArAr ArArArArArArArArArArA/i2MOErA/*/i2MOEr A/*/i2MOErA/*/i2MOErA/*/i2MOErA/*/i2MO ErA/*/3ddC/ | |
| 7 (Backbone) | 10 | /5Phos/rArArArArArA/i5OctdU/rArArArArArAr ArArArArArA/i5OctdU/rArArArArArArA*rA*r A*rA*rA*rA*/i5OctdU//3ddC/ | Ribose backbone; PS tail; 3 triazole linkages; 3 branches with PS tails |
| 7 (Branches) | 11 | 3×/5AzideN/rArArArArArArArArArArArAr ArArArArArArArArArArArA*rA*rA*rA*rA*r A*/3ddC/ | |
| 8 (Backbone) | 17 | /5Phos/rArArArArArA/i5OctdU/rArArArArArAr ArArArArArA/i5OctdU/rArArArArArA/i2MOEr A/*/i2MOErA/*/i2MOErA/*/i2MOErA/*/i2MO ErA/*/i2MOErA/*/i5OctdU//3ddC/ | Ribose backbone; 2MOE tail; 3 triazole linkages; 3 branches with 2MOE tails |
| 8 (Branches) | 16 | 3×/5AzideN/rArArArArArArArArArArArAr ArArArArArArArArArArA/i2MOErA/*/i2MOEr A/*/i2MOErA/*/i2MOErA/*/i2MOErA/*/i2MO ErA/*/3ddC/ | |
| 9 (Backbone) | 13 | /5Phos/dAdAdAdAdAdA/i5OctdU/dAdAdAdAd AdAdAdAdAdAdAdA/i5OctdU/dAdAdAdAdAd AdAdAdAdAdAdA/i5OctdU//3ddC/ | 2-Deoxyribose backbone; 3 triazole linkages; 3 branches with 2MOE tails |
| 9 (Branches) | 16 | 3×/5AzideN/rArArArArArArArArArArArAr ArArArArArArArArArArA/i2MOErA/*/i2MOEr A/*/i2MOErA/*/i2MOErA/*/i2MOErA/*/i2MO ErA/*/3ddC/ | |

Wherein the "backbone" portion of the sequence is the non-branched segment of the poly-A tail (see, e.g., FIG. 2A, horizontal highlighted region poly-A tail).

### EXAMPLE 5. Mechanistic characterization of mRNA stabilization by multimerized poly(A) tails.

To gain mechanistic insights into how the branched poly(A) tails stabilized the mRNA transcript, the overall effects of protein translation capacity were dissected into two layers, mRNA stability and translational efficiency. To minimize errors introduced by protein half-life in the luciferase assay, branched mRNA transcripts were synthesized encoding a degron-tagged Firefly luciferase (Firefly-PEST), which has been shown to effectively reduce luciferase half-life in HeLa cells from 20.4 hours to around 0.92 hours. Luminescence decay kinetics revealed that branched mRNA increased luminescence half-life from an estimated 7.2 hours to 17.1 hours, confirming increased mRNA stoichiometry at each time point (FIGS. 4A-B). Such enhancement was not due to differences in transfection efficiency, as decay kinetics of the internal Renilla control appeared similar among all conditions (FIG. 4C).

To dissect translational efficiency from mRNA stabilization at molecular level, in situ profiling was performed to quantify RNA copy numbers at subcellular resolution, with STARmap detecting all transcripts of the target sequence and RIBOmap detecting only the ribosome-bound fractions of mRNA copies (FIG. 4D, see materials and methods above). 6 hours post lipofection, cells treated under the same conditions were reseeded into two replicates for three time points, with one replicate profiled by STARmap and the other profiled by RIBOmap for the modified Firefly luciferase construct. Unmodified Renilla luciferase was profiled by STARmap in both replicates to normalize transfection efficiency. In the STARmap/RIBOmap images, the magenta and yellow puncta correspond to amplicons generated from cytosolic Firefly and Renilla mRNAs. The bright RNA granules in STARmap correspond to lipid vesicles formed by lipofectamine reagents, where a large number of non-translating mRNAs were located (FIG. 4E). Such granules were not observed in RIBOmap, further confirming that these combinatorial in situ sequencing methods successfully captured ribosome loads on single transcript and their spatial resolution allowed filtering of non-translating aggregates, leading to more accurate results than traditional bulk ribosome profiling and RT-qPCR methods. Both STARmap and RIBOmap recapitulated the trends observed luciferase assay, as cells transfected with branched Firefly luciferase mRNA yielded observably higher numbers of cytosolic amplicons than the linear counterparts throughout the whole cell population at all time points (FIGs. 4F-G). To approximate translation efficiency (TE), single-cell mRNA stoichiometry was measured by log10(Firefly STARmap / Renilla STARmap) and ribosome load was measured by log10(Firefly RIBOmap / Renilla STARmap). TE was calculated by taking the ratio of the two values from cells reseeded from the same biological conditions. (FIG. 4H) Interestingly, the difference in TE was insignificant between branched and linear constructs at 24 hours, indicating that multimerized poly(A) tails did not hamper nor enhance translation efficacy at that time point. However, at 48 hours and 72 hours, the branched mRNA exhibited higher TE than the linear mRNA, indicating that the branched construct preserved a more functional poly(A) tail at later time points to sustain the cap-dependent translation initiation.

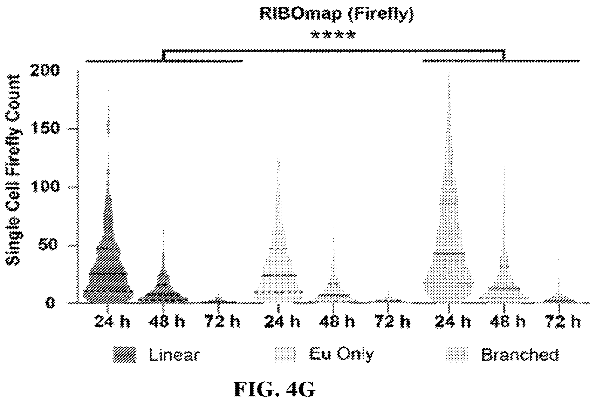

To test whether the stabilization effect of the branched poly(A) tail is due to its enhanced engagement with PABP, the dissociation constant (Kd) of linear versus branched poly(A) oligos with full length recombinant PABPC1 protein was quantified using EMSA (electrophoretic mobility shift assay). Linear/Branched modified poly(A) oligos were labeled with Alexa Fluor 546 at the 5'-end and incubated with varying concentrations of PABPC1-GST protein (FIG. 4I). The linear and branched poly(A) bound the first PABPC1 protein with the Kd of 10.7 and 6.4 nM, respectively, which agree with previously reported nanomolar range Kd of PABPC1 (32, 45) and suggest PABPC1 has higher affinity to the branched oligo (FIG. 4J). Additionally, branched poly(A) bound to multiple molecules of PABPC1 were clearly resolved in EMSA, with apparent Kd values of 6.4/10.9/15.3/35 nM against the first/second/third/fourth equivalents of PABPC1 (FIG. 4G, top). Conversely, the linear poly(A) molecules were only able to bind two equivalents of PABP in the same concentration range tested (FIG. 4G, middle). In a competitive binding assay, the branched poly(A) again demonstrated superior binding to PABP, with a significant portion being bound when 250 folds more of the linear counterparts were added (FIG. 4G, bottom). These results indicate that branching in the poly(A) region facilitated the synergy of PABP multimerization.

### EXAMPLE 6. Comparison of branched mRNA with circular RNA technology.

Numerous efforts have been made to broaden and improve the pharmacokinetic window of mRNA drugs. The use of circular RNA (circRNA), which is resistant to exonuclease cleavage, the main degradation pathway in their linear counterparts, has been purported to be a promising approach to avoid mRNA degradation. However, circRNA technology is limited from being a general mRNA medicine: translation initiation in circRNAs relies on internal ribosome entry sites (IRES), which is significantly less efficient than the 7-methylguanylate (m7G) cap-dependent translation initiation mechanism; additionally, circRNAs are incompatible with most chemical modifications, including N1-methylpseudouridine (m1ψ), as they block RNA splicing required for circRNA synthesis and eliminate IRES-based translation initiation. Therefore, alternative strategies are needed to protect the mRNA transcript from rapid exonuclease-dependent decay, particularly the deadenylation of the 3' poly(A) tail, while preserving the efficient cap-dependent translation initiation machinery.

To compare branched mRNA with the state-of-art circular RNA (circRNA) technology, linear/branched/circular mRNA vectors were synthesized encoding a secretive Nano Luciferase (NLuc) which allowed the repeated harvesting of media and measuring of protein expression kinetics over a longer time course. The circular mRNA vectors were to include an engineered iHRV IRES bearing an eIF4G binding motif 5'-upstream the CDS and a poly(A)-binding motif at the 3'-downstream, which have been reported to strongly enhance translation of circRNAs. CircRNAs were synthesized using IVT, circularized by backsplicing, and enriched by RNase R treatment (FIG. 5A). Both linear and branched capped mRNA bearing the translation-enhancing human alpha globin UTRs outperformed the circRNA construct by more than three orders of magnitude, even at higher dosage of circRNA (FIG. 5B). While linear and branched mRNAs produced similar amounts of in the first 24 hours, branched mRNAs maintained high level of the luciferase protein secretion 5 days post-mRNA lipofection, whereas protein secretion of linear mRNA treated cells quickly decayed after day 1 (FIG. 5C).

### EXAMPLE 7. Branched mRNA as a platform in therapeutic applications.

Compared to DNA carriers such as Adeno-associated viral (AAV), mRNA-LNP delivery of CRISPR-based therapeutic constructs have been shown to have better performance and less safety risks. However, high dosage of mRNA-LNP is still generally required to observe significant genome editing efficiency. To test whether branched mocRNA could further enhanced the efficacy of mRNA-based CRISPR-Cas9, a reporter HEK cell line expressing unstable green fluorescent proteins (uGFP) was treated with branched/linear Cas9 mRNA which were co-transfected with guide RNAs (sgRNA) targeting the GFP coding sequence (FIG. 6A). Both linear and branched Cas9 mRNA resulted in observable level of GFP ablation at 72 hours post-transfection, and the branched RNA knocked out the GFP gene more efficiently (FIG. 6B); the average level of GFP intensity in branched RNA treated cells was 7.1% of the untreated entries while the same dosing of linear mRNA leads to 56.5% of original average GFP intensity (FIG. 6C). Such differences in ablation efficacy could not be overturned by simple dose increment. Increasing the dosage of linear mRNA by 2- or 4-fold decreased GFP intensity to 27.2% and 25.1%, respectively, still not comparable with branched mRNA (FIGs. 6D-E).

To then test the translation efficiency and immunotoxicity of mocRNAs in vivo, branched and linear mRNAs encoding NanoLuc were studied in mice. Synthetic mRNAs were encapsulated in LNPs and administered through intravenous (i.v.) injection (FIG. 6F). At a single, minimal dose of ng, branched mRNA exhibited enhanced and prolonged protein expression in vivo (FIG. 6G-H). Observable level of luminescence was still observed in male mice treated with mRNA at 6 to 10 days post LNP administration, at which point expression of linear mRNA already dropped to background level. mRNA update kinetics appeared slightly different in female mice, but a similar trend was observed (FIG. 6I). Immunogenicity was evaluated by plasma level of tumor necrosis factor alpha (TNF-α) and toxicity was measured by aspartate transaminase (AST) and alanine transaminase (ALT), as i.v. delivery readily targets the liver. Levels of all biomarkers were indistinguishable among male and female mice treated with linear and branched mRNA, as well as male mice treated with poly(C) negative control, all of which were significantly lower than that of male mice treated with poly(I:C), a known RNA immuno-sensitizer recognized by toll-like receptor 3 (TLR3) and induce interferon (IFN)-β (FIG. 6I). The branched poly(A) topology and unnatural triazole chemical linkage therefore did not increase immunotoxicity in vivo, making them suitable for therapeutic RNAs.

### EXAMPLE 8. Production of poly-capped mRNAs.

As noted above, the rate-limiting step in translation initiation is the assembly of the TIC centered around the 7-methylguansine (m7G) cap by eIFs. FIG. 7A depicts the chemical structure of m7G caps denoted by the chemical shape in the following examples. FIG. 7B shows a schematic of the closed-loop structure of mRNA bound by the TIC, leading to the recruitment of ribosomes for translation. Thus, beyond modulating TE through altered poly(A) tails, increasing the affinity of the 5'-cap to eIFs could potentially result in higher TE of mRNA transcripts as well. Alternatively, mRNA bearing multiple 5'-caps may increase TE by forming multiple translation initiation complexes or increasing localized eIFs concentrations, therefore recruiting more ribosomes (FIG. 7C). Such multi-capped mRNAs are generalized into two types of structures (Type 1 and Type 2). Type 1 multi-capped mRNA is featured by a continuous phosphodiester backbone throughout the transcript with chemical conjugation handles in the 5' untranslated regions (UTRs) attached via modified nucleotides. Those handles are cross-linked to multiple capped oligonucleotides through click chemistries, exemplified by copper catalyzed azide-alkyne cycloaddition (CuAAC) or tetrazine-trans cyclooctene inverse-demand Diels-Alder reaction (IEDDA) (FIG. 7D). Alternatively, multiple capped oligonucleotides are first conjugated to a small molecule handle bearing multiple click chemistry sites. This multi-capped oligonucleotide is then ligated onto the 5'-end of an mRNA to yield type 2 multi-capped mRNA (FIG. 7E).

### EXAMPLE 9. Synthesis workflow of Type 1 multi-capped mRNA.

For mRNA with relatively short coding sequences (CDS), the mRNA transcript is completely chemically synthesized such that a terminal phosphate is introduced on the 5'-end with click chemistry handles in the 5'UTR. This modified mRNA is chemically capped and conjugated to capped oligonucleotides (FIG. 8A). Alternatively, the order of capping and conjugation is reversed (FIG. 8B). For longer mRNA constructs, a multi-capped, branched oligo is synthesized similarly through capping and conjugation. This multi-capped oligo is subsequently ligated onto a 5'-monophosphorylated mRNA, synthesized by in vitro transcription (IVT) and triphosphate hydrolysis, yielding a multi-capped mRNA-oligonucleotide conjugate (mocRNA) (FIGs. 8C-D). Such methods are generalizable across multiple transgenes.

### EXAMPLE 10. Multi-capped mRNA confers increased translation efficiency.

A proof-of-concept experiment was performed by synthesizing a doubly-capped mRNA encoding HiBit, a split luciferase tag that could be monitored by bioluminescence assay. HiBit mRNA was chemically synthesized such that it contains a 5'-phosphate and an internal 5-octadiynyl deoxyU (EU) modification in the 5'-UTR. Another short oligo was designed and synthesized similarly that it contained a 5'-phosphate and a terminal azide handle on the 3'-end (FIG. 9A). Both oligos were generated by solid phase phosphoramidite chemistry with ammonia as conterion. The oligo and HiBit mRNA were both chemically capped by treatment of m7G-imidazolide (FIG. 9B) in DMSO at 55 °C, catalyzed by 1-methyl imidazole. The oligos were subsequently purified using reverse-phase HPLC such that uncapped and capped transcripts were separated to ensure 100% capping (FIG. 9C). Capped oligos were conjugated by CuAAC using copper (II) sulfate and ascorbic acid at room temperature for 1 hour and then column purified (FIG. 9D). Synthesized HiBit-encoding constructs (0~4) were transfected into HeLa cells and bioluminescence was evaluated 12 hours after transfection (FIG. 9E). Branching in the 5'UTR without incorporating an additional cap resulted in minimal decrease in protein expression (construct 1 vs construct 2). However, capping of the branched oligo led to additional increase in protein expression, with the 15-nt capped oligo resulting in around 2-fold increase in protein expression (construct 1 vs construct 3). Multiple m7G cap therefore leads to increase in TE.

**Table 3: Branched 5' cap variants**

| Construct No. (FIG. 9E) | SEQ ID NO. | Sequence | Features |
|---|---|---|---|
| 0 | 18 | /5Phos/rArGrArUrArArArCrUrArG/i5OctdU/rArUrUrCr UrUrCrUrGrGrUrCrCrCrCrArCrArGrArCrUrCrArGrAr GrArGrArArCrCrCrGrCrCrArCrCrArUrGrGrUrGrArGr CrGrGrCrUrGrGrCrGrGrCrUrGrUrUrCrArArGrArArGr ArUrUrArGrCrUrGrArArArArArArArArArArArArArAr ArArArArArA | HiBit-mRNA; 1x EU handle; 5'-Phosphate |
| 1 | 19 | /5’m7GCap/rArGrArUrArArArCrUrArG/i5OctdU/rArUr UrCrUrUrCrUrGrGrUrCrCrCrArCrArGrArCrUrCrAr GrArGrArGrArArCrCrCrGrCrCrArCrCrArUrGrGrUrGr ArGrCrGrGrCrUrGrGrCrGrGrCrUrGrUrUrCrArArGrAr ArGrArUrUrArGrCrUrGrArArArArArArArArArArArAr ArArArArArArArA | HiBit-mRNA; 1x EU handle; 1x m7G cap |
| 2 (Backbone) | 19 | /5’m7GCap/rArGrArUrArArArCrUrArG/i5OctdU/rArUr UrCrUrUrCrUrGrGrUrCrCrCrArCrArGrArCrUrCrAr GrArGrArGrArArCrCrCrGrCrCrArCrCrArUrGrGrUrGr ArGrCrGrGrCrUrGrGrCrGrGrCrUrGrUrUrCrArArGrAr ArGrArUrUrArGrCrUrGrArArArArArArArArArArArAr ArArArArArArArA | HiBit-mRNA; 1x triazole linkage; 1x m7G cap; 15 nt branch |
| 2 (Branch) | 20 | /5sPhos/rArGrArGrArUrArArArCrUrArGrUrA/3AzideN/ | |
| 3 (Backbone) | 19 | /5’m7GCap/rArGrArUrArArArCrUrArG/i5OctdU/rArUr UrCrUrUrCrUrGrGrUrCrCrCrArCrArGrArCrUrCrAr GrArGrArGrArArCrCrGrCrCrArCrCrArUrGrGrUrGr ArGrCrGrGrCrUrGrGrCrGrGrCrUrGrUrUrCrArArGrAr ArGrArUrUrArGrCrUrGrArArArArArArArArArArAr ArArArArArArArA | HiBit-mRNA; 1x triazole linkage; 2x m7G cap; 15 nt branch |
| 3 (Branch) | 21 | /5’m7GCap/rArGrArGrArUrArArArCrUrArGrUrA/3Azi deN/ | |
| 4 (Backbone) | 19 | /5’m7GCap/rArGrArUrArArArCrUrArG/i5OctdU/rArUr UrCrUrUrCrUrGrGrUrCrCrCrCrArCrArGrArCrUrCrAr GrArGrArGrArArCrCrCrGrCrCrArCrCrArUrGrGrUrGr | HiBit-mRNA; 1x triazole linkage; 2x |
| | | ArGrCrGrGrCrUrGrGrCrGrGrCrUrGrUrUrCrArArGrAr ArGrArUrUrArGrCrUrGrArArArArArArArArArArArAr ArArArArArArArA | m7G cap; 8 nt branch |
| 4 (Branch) | | /5'm7GCap/rArGrArGrArUrArA/3AzideN/ | |

Further aspects and embodiments of the invention are provided in the following numbered clauses.
1. A modified mRNA comprising:
   (i) an open reading frame (ORF) encoding a protein;
   (ii) a poly-A region, and
   (iii) a 5' cap region;

   wherein the poly-A region is 3' to the ORF and comprises 10 or more nucleotides and the 5' cap region is 5' to the ORF; and
   wherein the poly-A region comprises two or more poly-A tails and/or the 5' cap region comprises two or more 5' caps.
2. The modified mRNA of clause 1, wherein the modified mRNA comprises a poly-A region comprising two or more poly-A tails and a 5' cap region comprising one 5' cap.
3. The modified mRNA of clause 1, wherein the modified mRNA comprises a poly-A region comprising one poly-A tail and a 5' cap region comprising two or more 5' caps.
4. The modified mRNA of clause 1, wherein the modified mRNA comprises a poly-A region comprising two or more poly-A tails and a 5' cap region comprising two or more 5' caps.
5. The modified mRNA of any one of clauses 1-4, wherein the modified mRNA comprises a 5' untranslated region (5' UTR) and a 3' untranslated region (3' UTR), wherein the ORF is between the 5' UTR and the 3' UTR, wherein the 3' UTR is between the ORF and the poly-A region, and wherein the 5' UTR is between the 5' cap region and the ORF.
6. The modified mRNA of any one of clauses 1-5, wherein the modified mRNA is a linear mRNA, wherein the 5' cap region is at the 5' end of the modified mRNA and the poly-A region is at the 3' end of the modified mRNA
7. The modified mRNA of clause 5 or 6, wherein the modified mRNA is a circular mRNA, wherein the poly-A region is between the 3' UTR and the 5' cap region.
8. The modified mRNA of any one of clauses 1-7, wherein the poly-A region comprises two or more poly-A tails, and at least two instances of the poly-A tails are covalently linked by a first linker.
9. The modified mRNA of clause 8, wherein:
   the poly-A region comprises -(a first poly-A tail)-[(a first linker)-(a second poly-A tail)ₙ₁]ₙ2;
   each instance of n1 is independently an integer between 1 and 20, inclusive;
   n2 is an integer between 2 and 10, inclusive;
   the first poly-A tail is covalently linked to the 3' UTR; and
   the first linker is covalently linked to the first poly-A tail and to the second poly-A tail.
10. The modified mRNA of clause 9, wherein each instance of n1 is 1.
11. The modified mRNA of clause 10, wherein at least one instance of the first linker is covalently linked to an internal nucleotide of the first poly-A tail.
12. The modified mRNA of clause 10 or 11, wherein at least one instance of the first linker is covalently linked to a second poly-A tail at the 3' nucleotide of the second poly-A tail.
13. The modified mRNA of any one of clauses 10-12, wherein at least one instance of the first linker comprises at least one moiety formed by reacting two orthogonal click-chemistry handles.
14. The modified mRNA of clause 13, wherein at least one moiety formed by reacting two orthogonal click-chemistry handles is of the formula: 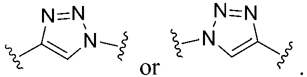
15. The modified mRNA of any one of clauses 10-14, wherein:
   each instance of the first linker is independently substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, substituted or unsubstituted, C₁₋₂₀₀ heteroalkynylene, substituted or unsubstituted carbocyclylene, substituted or unsubstituted heterocyclylene, substituted or unsubstituted arylene, or substituted or unsubstituted heteroarylene; and
   optionally one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with substituted or unsubstituted carbocyclylene, substituted or unsubstituted heterocyclylene, substituted or unsubstituted arylene, or substituted or unsubstituted heteroarylene.
16. The modified mRNA of any one of clauses 10-15, wherein one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with 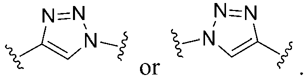
17. The modified mRNA of any one of clauses 10-16, wherein:
   at least one instance of the first linker is of the formula: 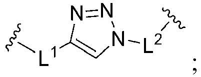
   each instance of L¹ is independently substituted or unsubstituted, C₁₋₁₉₉ alkylene or substituted or unsubstituted, C₁₋₁₉₉ heteroalkylene; and
   each instance of L² is independently substituted or unsubstituted, C₁₋₁₉₉ alkylene or substituted or unsubstituted, C₁₋₁₉₉ heteroalkylene.
18. The modified mRNA of any one of clauses 10-17, wherein at least one instance of the first linker is of the formula: 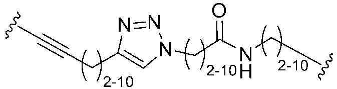 or 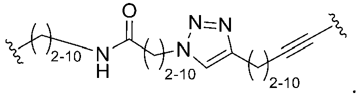
19. The modified mRNA of clause 9, wherein each instance of n1 is independently an integer between 2 and 20, inclusive.
20. The modified mRNA of clause 19, wherein at least one instance of the first linker comprises a dendrimer.
21. The modified mRNA of clause 20, wherein at least one instance of the dendrimer is a polyamidoamine (PAMAM) dendrimer.
22. The modified mRNA of clause 21, wherein at least one instance of the PAMAM dendrimer is of the formula: 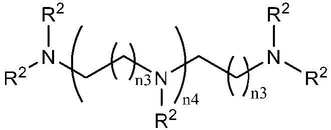 wherein
   each instance of n3 is independently an integer between 1 and 10, inclusive;
   each instance of n4 is independently an integer between 0 and 10, inclusive;
   each instance of R² is independently hydrogen or 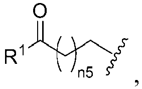 provided that at least three instances of R² is 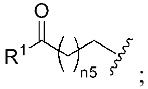
   each instance of R¹ is substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, or substituted or unsubstituted, C₁₋₁₀ heteroalkynylene;
   optionally one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with substituted or unsubstituted carbocyclylene, substituted or unsubstituted heterocyclylene, substituted or unsubstituted arylene, or substituted or unsubstituted heteroarylene; and
   each instance of n5 is independently an integer between 0 and 10, inclusive.
23. The modified mRNA of clause 22, wherein at least one instance of the PAMAM dendrimer comprises: 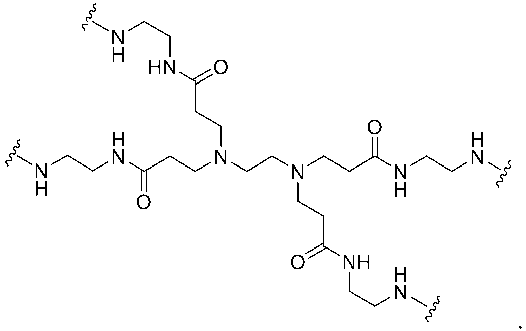
24. The modified mRNA of clause 21, wherein one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with 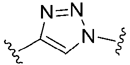 or 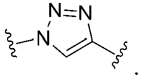
25. The modified mRNA of clause 24, wherein at least one instance of the first linker is of the formula: 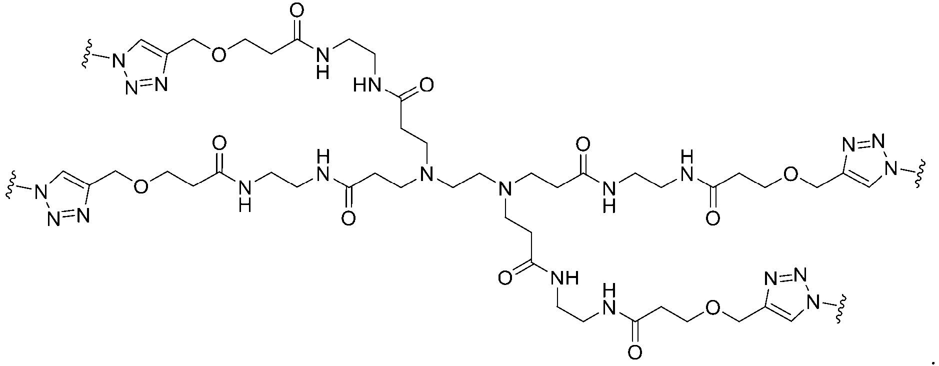
26. The modified mRNA of any one of clauses 8-25, wherein at least one instance of the first linker is covalently linked to a 3' nucleotide of the first poly-A tail.
27. The modified mRNA of any one of clauses 8-26, wherein at least one instance of the first linker is covalently linked to a 3' nucleotide of the second poly-A tail.
28. The modified mRNA of any one of clauses 1-27, wherein the poly-A region of the modified mRNA comprises one or more modified nucleotides.
29. The modified mRNA of clause 28, wherein 3 or more of the last 10 nucleotides of at least one of the two or more poly-A tails are modified nucleotides and/or non-adenosine nucleotides.
30. The modified mRNA of clause 28 or 29, wherein one or more modified nucleotides of the poly-A region are modified adenosine nucleotides.
31. The modified mRNA of any one of clauses 28-30, wherein one or more modified nucleotides of the poly-A region are modified non-adenosine nucleotides.
32. The modified mRNA of any one of clauses 28-31, wherein one or more modified nucleotides of the poly-A region comprise a modified nucleobase.
33. The modified mRNA of clause 32, wherein the modified nucleobase is selected from the group consisting of xanthine, allyaminouracil, allyaminothymidine, hypoxanthine, digoxigeninated adenine, digoxigeninated cytosine, digoxigeninated guanine, digoxigeninated uracil, 6-chloropurineriboside, N6-methyladenine, methylpseudouracil, 2-thiocytosine, 2-thiouracil, 5-methyluracil, 4-thiothymidine, 4-thiouracil, 5,6-dihydro-5-methyluracil, 5,6-dihydrouracil, 5-[(3-Indolyl)propionamide-N-allyl]uracil, 5-aminoallylcytosine, 5-aminoallyluracil, 5-bromouracil, 5-bromocytosine, 5-carboxycytosine, 5-carboxymethylesteruracil, 5-carboxyuracil, 5-fluorouracil, 5-formylcytosine, 5-formyluracil, 5-hydroxycytosine, 5-hydroxymethylcytosine, 5-hydroxymethyluracil, 5-hydroxyuracil, 5-iodocytosine, 5-iodouracil, 5-methoxycytosine, 5-methoxyuracil, 5-methylcytosine, 5-methyluracil, 5-propargylaminocytosine, 5-propargylaminouracil, 5-propynylcytosine, 5-propynyluracil, 6-azacytosine, 6-azauracil, 6-chloropurine, 6-thioguanine, 7-deazaadenine, 7-deazaguanine, 7-deaza-7-propargylaminoadenine, 7-deaza-7-propargylaminoguanine, 8-azaadenine, 8-azidoadenine, 8-chloroadenine, 8-oxoadenine, 8-oxoguanine, araadenine, aracytosine, araguanine, arauracil, biotin-16-7-deaza-7-propargylaminoguanine, biotin-16-aminoallylcytosine, biotin-16-aminoallyluracil, cyanine 3-5-propargylaminocytosine, cyanine 3-6-propargylaminouracil, cyanine 3-aminoallylcytosine, cyanine 3-aminoallyluracil, cyanine 5-6-propargylaminocytosine, cyanine 5-6-propargylaminouracil, cyanine 5-aminoallylcytosine, cyanine 5-aminoallyluracil, cyanine 7-aminoallyluracil, dabcyl-5-3-aminoallyluracil, desthiobiotin-16-aminoallyl-uracil, desthiobiotin-6-aminoallylcytosine, isoguanine, N1-ethylpseudouracil, N1-methoxymethylpseudouracil, Nl-methyladenine, N1-methylpseudouracil, N1-propylpseudouracil, N2-methylguanine, N4-biotin-OBEA-cytosine, N4-methylcytosine, N6-methyladenine, O6-methylguanine, pseudoisocytosine, pseudouracil, thienocytosine, thienoguanine, thienouracil, xanthosine, 3-deazaadenine, 2,6-diaminoadenine, 2,6-daminoguanine, 5-carboxamide-uracil, 5-ethynyluracil, N6-isopentenyladenine (i6A), 2-methyl-thio-N6-isopentenyladenine (ms2i6A), 2-methylthio-N6-methyladenine (ms2m6A), N6-(cis-hydroxyisopentenyl)adenine (io6A), 2-methylthio-N6-(cis-hydroxyisopentenyl)adenine (ms2io6A), N6-glycinylcarbamoyladenine (g6A), N6-threonylcarbamoyladenine (t6A), 2-methylthio-N6-threonyl carbamoyladenine (ms2t6A), N6-methyl-N6-threonylcarbamoyladenine (m6t6A), N6-hydroxynorvalylcarbamoyladenine (hn6A), 2-methylthio-N6-hydroxynorvalyl carbamoyladenine (ms2hn6A), N6,N6-dimethyladenine (m62A), and N6-acetyladenine (ac6A).
34. The modified mRNA of any one of clauses 28-33, wherein one or more modified nucleotides comprise a modified sugar.
35. The modified mRNA of clause 34, wherein the modified sugar is selected from the group consisting of 2'-thioribose, 2',3'-dideoxyribose, 2'-amino-2'-deoxyribose, 2' deoxyribose, 2'-azido-2'-deoxyribose, 2'-fluoro-2'-deoxyribose, 2'-O-methylribose, 2'-O-methyldeoxyribose, 3'-amino-2',3'-dideoxyribose, 3'-azido-2',3'-dideoxyribose, 3'-deoxyribose, 3'-O-(2-nitrobenzyl)-2'-deoxyribose, 3'-O-methylribose, 5'-aminoribose, 5'-thioribose, 5-nitro-1-indolyl-2'-deoxyribose, 5'-biotin-ribose, 2'-O,4'-C-methylene-linked, 2'-O,4'-C-amino-linked ribose, and 2'-O,4'-C-thio-linked ribose.
36. The modified mRNA of any one of clauses 28-35, wherein one or more modified nucleotides comprise a modified phosphate.
37. The modified mRNA of clause 36, wherein the modified phosphate is selected from the group consisting of phosphorothioate (PS), thiophosphate, 5'-O-methylphosphonate, 3'-O-methylphosphonate, 5'-hydroxyphosphonate, hydroxyphosphanate, phosphoroselenoate, selenophosphate, phosphoramidate, carbophosphonate, methylphosphonate, phenylphosphonate, ethylphosphonate, H-phosphonate, guanidinium ring, triazole ring, boranophosphate (BP), methylphosphonate, and guanidinopropyl phosphoramidate.
38. The modified mRNA of clause 36 or 37, wherein the poly-A region comprises between 1 and 3, between 3 and 5, between 5 and 10, between 10 and 15, between 15 and 30, between 30 and 50, between 50 and 100, or between 100 and 200 phosphorothioates.
39. The modified mRNA of clause 38, wherein the poly-A region comprises between 5 and 30 phosphorothioates.
40. The modified mRNA of any one of clauses 28-39, wherein the poly-A region comprises between 3 and 5, between 5 and 10, between 10 and 15, between 15 and 30, between 30 and 50, between 50 and 100, or between 100 and 200 deoxyribose sugars.
41. The modified mRNA of clause 40, wherein the poly-A region comprises between 5 and 30 deoxyribose sugars.
42. The modified mRNA of any one of clauses 28-41, wherein the mRNA comprises a 3' terminal nucleotide wherein the 3' terminal nucleotide is dideoxyadenosine, dideoxycytidine, dideoxyguanosine, dideoxythymidine, dideoxyuridine, or inverted-deoxythymidine.
43. The modified mRNA of any one of clauses 1-42, wherein the poly-A region comprises between 25 and 500 nucleotides.
44. The modified mRNA of clause 43, wherein the poly-A region comprises between 50 and 100, between 100 and 150, between 150 and 200, between 200 and 300, between 300 and 400, or between 400 and 500 nucleotides.
45. The modified mRNA of any one of clauses 1-44, wherein the poly-A region comprises the poly-A region comprises 10 or more adenosine nucleotides.
46. The modified mRNA of any one of clauses 1-45, wherein 25-100%, 30-100%, 40-100%, 50-100%, 60-100%, 70-100%, 80-100%, 90-100%, 95-100%, 96-100%, 97-100%, 98-100%, or 99-100% of nucleotides of the poly-A region are adenosine nucleotides.
47. The modified mRNA of any one of clauses 8-46, wherein the first poly-A tail comprises between 10 and 50 adenosine ribonucleotides, between 1 and 10 modified uridine deoxyribonucleotides comprising an attachment point, and a 3' terminal dideoxyribonucleotide or inverted deoxyribonucleotide; and the second poly-A tails comprises between 10 and 50 adenosine ribonucleotides.
48. The modified mRNA of clause 47, wherein:
   the first poly-A tail comprises a nucleotide sequence set forth as: 5'-rArArArArArAdU(a1) rArArArArArArArArArArArAdU(a1)rArArArArArArArArArArArAdU(a1)ddC-3' (SEQ ID NO: 1);
   at least one instance of the second poly-A tails comprises a nucleotide sequence set forth as: 5'-rArArArArArArArArArArArArArArArArArArArArArArArArA*rA*rA* rA*rA*rA*(bl)-3' (SEQ ID NO: 2);
   each instance of the first linker is independently 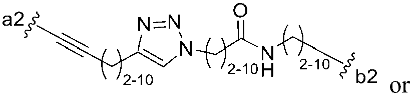 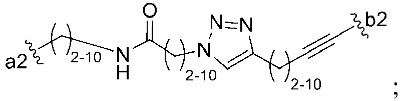
   "rA" represents an adenosine ribonucleotide;
   "dU(a1)" represents a modified uridine deoxyribonucleotide;
   "ddC" represents a cytosine dideoxyribonucleotide;
   "*" represents a phosphorothioate linkage;
   the a1 of each instance of dU(a1) represents an attachment point on the uridine of the dU(a1);
   the b1 of rA*(b1) represents an attachment point on the * of the rA*(b1);
   each instance of a1 is attached to an instance of a2; and
   each instance of b1 is attached to an instance of b2.
49. The modified mRNA of clause 47 or 48, wherein the modified uridine deoxynucleotide is 5-octadiynyl deoxyuridine.
50. The modified mRNA of any one of clauses 8-46, wherein the first poly-A tail and the second poly-A tails comprise between 10 and 50 adenosine ribonucleotides and a 3' terminal azide moiety.
51. The modified mRNA of clause 50, wherein the first poly-A tail and the second poly-A tails comprise a nucleotide sequence set forth as: 
5′-rArArArArArArArArArArArArArArArArArArArArArArA*rA*rA*
rA*rA*rA*/AzideN/-3′ (SEQ ID NO: 3);
 wherein "rA" represents an adenosine ribonucleotide; "AzideN" represents a 3' azide moiety and "*" represents a phosphorothioate linkage.
52. The modified mRNA of any one of clauses 8-46, wherein the first poly-A tail and the second poly-A tails comprise between 10 and 50 adenosine ribonucleotides and a 5' terminal azide moiety.
53. The modified mRNA of clause 52, wherein the first poly-A tail and the second poly-A tails comprise a nucleotide sequence set forth as: 
5′-/AzideN/rArArArArArArArArArArArArArArArArArArArArArArArArA*rA*rA*
rA*rA*rA-3′ (SEQ ID NO: 27);
 wherein "rA" represents an adenosine ribonucleotide; "AzideN" represents a 3' azide moiety and "*" represents a phosphorothioate linkage.
54. The modified mRNA of any one of clauses 1-53, wherein the 5' cap region comprises two or more 5' caps, and at least two instances of the 5' caps are covalently linked by a second linker
55. The modified mRNA of clause 54, wherein:
   the 5' cap region comprises -(a first 5' cap)-[(a second linker)-(a second 5' cap)ₘ₁]ₘ₂;
   each instance of m1 is independently an integer between 1 and 20, inclusive;
   m2 is an integer between 2 and 10, inclusive;
   the first 5' cap is covalently linked to the 5' UTR; and
   the second linker is covalently linked to the first 5' cap and to the second 5' cap.
56. The modified mRNA of clause 55, wherein each instance of m1 is 1.
57. The modified mRNA of clause 56, wherein at least one instance of the second linker is covalently linked to an internal nucleotide of the first 5' cap.
58. The modified mRNA of clause 56 or 57, wherein at least one instance of the second linker is covalently linked to a second 5' cap at a 3' nucleotide of the second 5' cap.
59. The modified mRNA of any one of clauses 56-58, wherein at least one instance of the second linker comprises at least one moiety formed by reacting two orthogonal click-chemistry handles.
60. The modified mRNA of clause 59, wherein at least one moiety formed by reacting two orthogonal click-chemistry handles is of the formula: 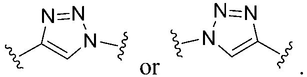
61. The modified mRNA of any one of clauses 56-60, wherein:
   each instance of the second linker is independently substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, substituted or unsubstituted, C₁₋₂₀₀ heteroalkynylene, substituted or unsubstituted carbocyclylene, substituted or unsubstituted heterocyclylene, substituted or unsubstituted arylene, or substituted or unsubstituted heteroarylene; and
   optionally one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with substituted or unsubstituted carbocyclylene, substituted or unsubstituted heterocyclylene, substituted or unsubstituted arylene, or substituted or unsubstituted heteroarylene.
62. The modified mRNA of any one of clauses 49-54, wherein one or more carbon atoms in the parent chain of each instance of the substituted or unsubstituted, C₁₋₂₀₀ alkylene, substituted or unsubstituted, C₁₋₂₀₀ alkenylene, substituted or unsubstituted, C₁₋₂₀₀ alkynylene, substituted or unsubstituted, C₁₋₂₀₀ heteroalkylene, substituted or unsubstituted, C₁₋₂₀₀ heteroalkenylene, and substituted or unsubstituted, C₁₋₂₀₀ heteroalkynylene are independently replaced with 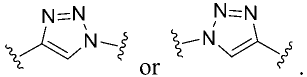
63. The modified mRNA of any one of clauses 56-62, wherein:
   at least one instance of the second linker is of the formula: 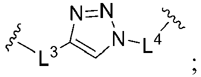
   each instance of L³ is independently substituted or unsubstituted, C₁₋₁₉₉ alkylene or substituted or unsubstituted, C₁₋₁₉₉ heteroalkylene; and
   each instance of L⁴ is independently substituted or unsubstituted, C₁₋₁₉₉ alkylene or substituted or unsubstituted, C₁₋₁₉₉ heteroalkylene.
64. The modified mRNA of any one of clauses 56-62, wherein at least one instance of the second linker is of the formula: 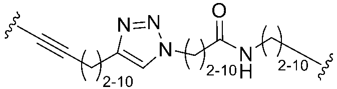 or 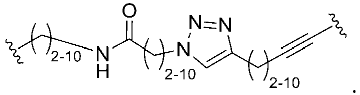
65. The modified mRNA of clause 56, wherein each instance of m1 is independently an integer between 2 and 20, inclusive.
66. The modified mRNA of clause 65, wherein at least one instance of the second linker comprises a dendrimer.
67. The modified mRNA of clause 66, wherein at least one instance of the dendrimer is a polyamidoamine (PAMAM) dendrimer.
68. The modified mRNA of clause 67, wherein at least one instance of the PAMAM dendrimer is of the formula: 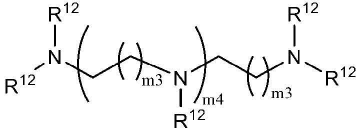 wherein
   each instance of m3 is independently an integer between 1 and 10, inclusive;
   each instance of m4 is independently an integer between 0 and 10, inclusive;
   each instance of R¹² is independently hydrogen or 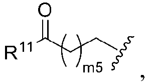 provided that at least three instances of R¹² is 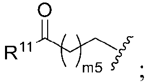
   each instance of R¹¹ is substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, or substituted or unsubstituted, C₁₋₁₀ heteroalkynylene;
   optionally one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with substituted or unsubstituted carbocyclylene, substituted or unsubstituted heterocyclylene, substituted or unsubstituted arylene, or substituted or unsubstituted heteroarylene; and
   each instance of m5 is independently an integer between 0 and 10, inclusive.
69. The modified mRNA of clause 67 or 68, wherein at least one instance of the PAMAM dendrimer comprises: 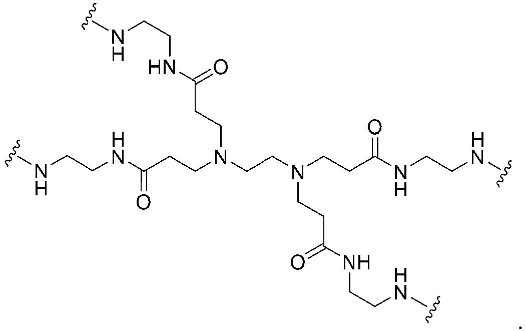
70. The modified mRNA of clause 68, wherein one or more carbon atoms in the parent chain of each instance of the substituted or unsubstituted, C₁₋₁₀ alkylene, substituted or unsubstituted, C₁₋₁₀ alkenylene, substituted or unsubstituted, C₁₋₁₀ alkynylene, substituted or unsubstituted, C₁₋₁₀ heteroalkylene, substituted or unsubstituted, C₁₋₁₀ heteroalkenylene, and substituted or unsubstituted, C₁₋₁₀ heteroalkynylene are independently replaced with 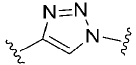 or 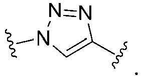
71. The modified mRNA of clause 68, wherein at least one instance of the first linker is of the formula: 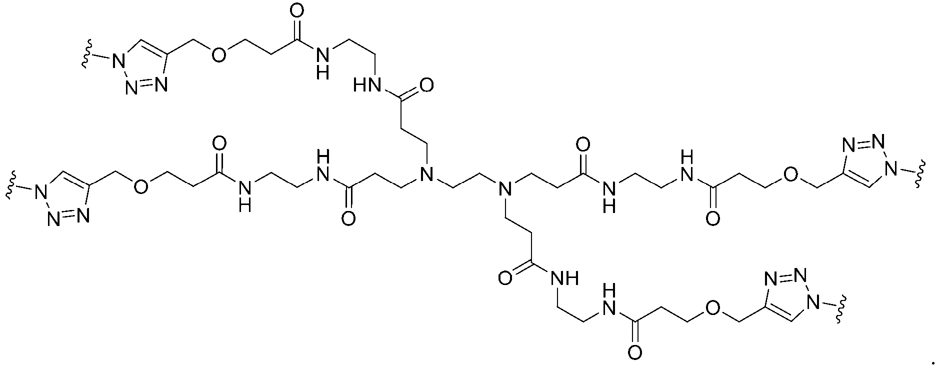
72. The modified mRNA of any one of clauses 50-71, wherein at least one instance of the second linker is covalently linked to a 5' or 3' nucleotide of the first 5' cap.
73. The modified mRNA of any one of clauses 50-72, wherein at least one instance of the second linker is covalently linked to a 5' or 3' nucleotide of the at least one instance of the second 5' caps.
75. The modified mRNA of any one of clauses 50-73, wherein the 5' cap region of the modified mRNA comprises one or more modified nucleotides.
75. The modified mRNA of clause 74, wherein one or more modified nucleotides comprise a modified nucleobase.
76. The modified mRNA of clause 75, wherein the modified nucleobase is selected from the group consisting of xanthine, allyaminouracil, allyaminothymidine, hypoxanthine, digoxigeninated adenine, digoxigeninated cytosine, digoxigeninated guanine, digoxigeninated uracil, 6-chloropurineriboside, N6-methyladenine, methylpseudouracil, 2-thiocytosine, 2-thiouracil, 5-methyluracil, 4-thiothymidine, 4-thiouracil, 5,6-dihydro-5-methyluracil, 5,6-dihydrouracil, 5-[(3-Indolyl)propionamide-N-allyl]uracil, 5-aminoallylcytosine, 5-aminoallyluracil, 5-bromouracil, 5-bromocytosine, 5-carboxycytosine, 5-carboxymethylesteruracil, 5-carboxyuracil, 5-fluorouracil, 5-formylcytosine, 5-formyluracil, 5-hydroxycytosine, 5-hydroxymethylcytosine, 5-hydroxymethyluracil, 5-hydroxyuracil, 5-iodocytosine, 5-iodouracil, 5-methoxycytosine, 5-methoxyuracil, 5-methylcytosine, 5-methyluracil, 5-propargylaminocytosine, 5-propargylaminouracil, 5-propynylcytosine, 5-propynyluracil, 6-azacytosine, 6-azauracil, 6-chloropurine, 6-thioguanine, 7-deazaadenine, 7-deazaguanine, 7-deaza-7-propargylaminoadenine, 7-deaza-7-propargylaminoguanine, 8-azaadenine, 8-azidoadenine, 8-chloroadenine, 8-oxoadenine, 8-oxoguanine, araadenine, aracytosine, araguanine, arauracil, biotin-16-7-deaza-7-propargylaminoguanine, biotin-16-aminoallylcytosine, biotin-16-aminoallyluracil, cyanine 3-5-propargylaminocytosine, cyanine 3-6-propargylaminouracil, cyanine 3-aminoallylcytosine, cyanine 3-aminoallyluracil, cyanine 5-6-propargylaminocytosine, cyanine 5-6-propargylaminouracil, cyanine 5-aminoallylcytosine, cyanine 5-aminoallyluracil, cyanine 7-aminoallyluracil, dabcyl-5-3-aminoallyluracil, desthiobiotin-16-aminoallyl-uracil, desthiobiotin-6-aminoallylcytosine, isoguanine, N1-ethylpseudouracil, N1-methoxymethylpseudouracil, N1-methyladenine, N1-methylpseudouracil, N1-propylpseudouracil, N2-methylguanine, N4-biotin-OBEA-cytosine, N4-methylcytosine, N6-methyladenine, O6-methylguanine, pseudoisocytosine, pseudouracil, thienocytosine, thienoguanine, thienouracil, xanthosine, 3-deazaadenine, 2,6-diaminoadenine, 2,6-daminoguanine, 5-carboxamide-uracil, 5-ethynyluracil, N6-isopentenyladenine (i6A), 2-methyl-thio-N6-isopentenyladenine (ms2i6A), 2-methylthio-N6-methyladenine (ms2m6A), N6-(cis-hydroxyisopentenyl)adenine (io6A), 2-methylthio-N6-(cis-hydroxyisopentenyl)adenine (ms2io6A), N6-glycinylcarbamoyladenine (g6A), N6-threonylcarbamoyladenine (t6A), 2-methylthio-N6-threonyl carbamoyladenine (ms2t6A), N6-methyl-N6-threonylcarbamoyladenine (m6t6A), N6-hydroxynorvalylcarbamoyladenine (hn6A), 2-methylthio-N6-hydroxynorvalyl carbamoyladenine (ms2hn6A), N6,N6-dimethyladenine (m62A), and N6-acetyladenine (ac6A).
77. The modified mRNA of any one of clauses 74-76, wherein one or more modified nucleotides comprise a modified sugar.
78. The modified mRNA of clause 77, wherein the modified sugar is selected from the group consisting of 2'-thioribose, 2',3'-dideoxyribose, 2'-amino-2'-deoxyribose, 2' deoxyribose, 2'-azido-2'-deoxyribose, 2'-fluoro-2'-deoxyribose, 2'-O-methylribose, 2'-O-methyldeoxyribose, 3'-amino-2',3'-dideoxyribose, 3'-azido-2',3'-dideoxyribose, 3'-deoxyribose, 3'-O-(2-nitrobenzyl)-2'-deoxyribose, 3'-O-methylribose, 5'-aminoribose, 5'-thioribose, 5-nitro-1-indolyl-2'-deoxyribose, 5'-biotin-ribose, 2'-O,4'-C-methylene-linked, 2'-O,4'-C-amino-linked ribose, and 2'-O,4'-C-thio-linked ribose.
79. The modified mRNA of any one of clauses 74-78, wherein a one or more modified nucleotides comprise a modified phosphate.
80. The modified mRNA of clause 79, wherein the modified phosphate is selected from the group consisting of phosphorothioate (PS), thiophosphate, 5'-O-methylphosphonate, 3'-O-methylphosphonate, 5'-hydroxyphosphonate, hydroxyphosphanate, phosphoroselenoate, selenophosphate, phosphoramidate, carbophosphonate, methylphosphonate, phenylphosphonate, ethylphosphonate, H-phosphonate, guanidinium ring, triazole ring, boranophosphate (BP), methylphosphonate, and guanidinopropyl phosphoramidate.
81. The modified mRNA of clause 79 or 80, wherein the 5' cap region comprises between 1 and 3, between 3 and 5, between 5 and 10, between 10 and 15, between 15 and 30, between 30 and 50, between 50 and 100, or between 100 and 200 phosphorothioates.
82. The modified mRNA of clause 81, wherein the 5' cap region comprises between 5 and 30 phosphorothioates.
83. The modified mRNA of any one of clauses 74-82, wherein the 5' cap region comprises between 3 and 5, between 5 and 10, between 10 and 15, between 15 and 30, between 30 and 50, between 50 and 100, or between 100 and 200 deoxyribose sugars.
84. The modified mRNA of clause 83, wherein the 5' cap region comprises between 5 and 30 deoxyribose sugars.
85. The modified mRNA of any one of clauses 74-84, wherein the mRNA comprises a 3' terminal nucleotide wherein the 3' terminal nucleotide is dideoxyadenosine, dideoxycytidine, dideoxyguanosine, dideoxythymidine, dideoxyuridine, or inverted-deoxythymidine.
86. The modified mRNA of any one of clauses 50-85, wherein the 5' cap region comprises between 25-500 nucleotides.
87. The modified mRNA of clause 86, wherein the 5' cap region comprises between 50 and 100, between 100 and 150, between 150 and 200, between 200 and 300, between 300 and 400, or between 400 and 500 nucleotides.
88. The modified mRNA of any one of clauses 50-87, wherein the 5' cap region comprises between 1 and 3, between 3 and 5, between 5 and 7, or between 7 and 10 5' caps.
89. The modified mRNA of any one of clauses 50-88, wherein the 5' cap region comprises one or more 7-methylguanylate caps comprising a 5'-5' triphosphate linkage.
90. The modified mRNA of any one of clauses 50-89, wherein the 5' cap region comprises one or more 2,2,7-trimethylguianosine caps comprising a 5'-5' triphosphate linkage.
91. The modified mRNA of any one of clauses 50-90, wherein the 5' cap region comprises one or more modified 5' caps.
92. The modified mRNA of clause 91, wherein one or more modified 5' caps are a locked nucleic acid (LNA)-modified cap, a 5' cap comprising a 5'-5' triphosphate linkage that is modified by 5'-phosphorothiolate, or a 5' cap comprising a 5'-5' tetraphosphate linkage.
93. The modified mRNA of clause 91 or 92, wherein the 5' cap region comprises one or more 5' caps comprising one or more modified nucleotides.
94. The modified mRNA of clause 93, wherein one or more modified nucleotides are a 2'-O-methylated nucleotide.
95. The modified mRNA of any one of clauses 50-94, wherein the first 5' cap comprises between 10 and 50 ribonucleotides, between 1 and 10 modified uridine deoxyribonucleotides comprising an attachment point, and a 5' cap; and the second 5' cap comprises between 10 and 50 ribonucleotides and a 5' cap.
96. The modified mRNA of clause 95, wherein:
   the first 5' cap comprises a nucleotide sequence set forth as: 
5′-/Cap/rGrGrGrArArAdU(c1)rArArGrArGrArGrArArArArGrArArGrArGdU(c1)
rArArGrArArGrArArAdU(c1)rA-3′ (SEQ ID NO: 4);
   the second 5' cap comprises a nucleotide sequence set forth as: 5'-/Cap/rGrGrGrArGrArCrTrGrCrCrArCrCrA*rA*rA*rA*rA*rA*(d1)-3' (SEQ ID NO: 5);
   each instance of the second linker is independently 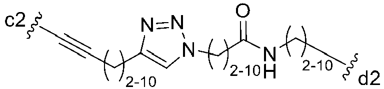 or 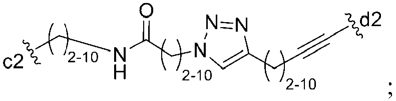
   "rA" represents an adenosine ribonucleotide;
   "rT" represents a thymidine ribonucleotide;
   "rC" represents a cytidine ribonucleotide;
   "rG" represents a guanosine ribonucleotide;
   "dU(c1)" represents a modified uridine deoxyribonucleotide;
   "*" represents a phosphorothioate linkage;
   "Cap" represents a 5' cap;
   the c1 of each instance of dU(c1) represents an attachment point on the uridine of the dU(c1);
   the d1 of rA*(d1) represents an attachment point on the * of the rA*(d1);
   each instance of c1 is attached to an instance of c2; and
   each instance of d1 is attached to an instance of d2.
97. The modified mRNA of clause 95 or 96, wherein the modified uridine deoxynucleotide is 5-octadiynyl deoxyuridine.
98. The modified mRNA of any one of clauses 50-94, wherein the first 5' cap comprises between 5 and 10 ribonucleotides and a 5' azide moiety; and the second 5' cap comprises between 10 and 50 ribonucleotides, a 3' azide moiety, and a 5' cap.
99. The modified mRNA of clause 98, wherein the first 5' cap comprises a nucleotide sequence set forth as:
   5'-//5AzideN/rArArA rArA-3';
   and the second 5' cap comprises a nucleotide sequence set forth as:
   5'-/Cap/rGrGrGrArGrArCrTrGrCrCrArCrCrA*rA*rA*rA*rA*rA*/3AzideN/-3' (SEQ ID NO: 6);
   wherein "rA" represents an adenosine ribonucleotide; "rT" represents a thymidine ribonucleotide; "rC" represents a cytidine ribonucleotide; "rG" represents a guanosine ribonucleotide; "5AzideN" represents a 5' azide moiety, "3AzideN" represents a 3' azide moiety, "*" represents a phosphorothioate linkage, and "Cap" represents a 5' cap.
100. A method of producing a modified mRNA of any one of clauses 1-99, the method comprising ligating a first RNA comprising an open reading frame (ORF) encoding a protein to a tailing nucleic acid comprising two or more poly-A tails and/or a capping nucleic acid comprising two or more 5' caps, in the presence of an RNA ligase, whereby the RNA ligase forms a covalent bond between the 3' nucleotide of the first RNA and the 5' nucleotide of the tailing nucleic acid and/or a covalent bond between the 5' nucleotide of the first RNA and the 3' nucleotide of the capping nucleic acid to produce the modified mRNA.
101. The method of clause 100, wherein the modified mRNA comprises a 5' untranslated region (5' UTR) and a 3' untranslated region (3' UTR), wherein the ORF is between the 5' UTR and the 3' UTR, wherein the 3' UTR is between the ORF and the ligated tailing nucleic acid, and wherein the 5' UTR is between the ORF and the ligated capping nucleic acid.
102. The method of clause 100 or 101, wherein the tailing nucleic acid comprises a first poly-A tail and one or more second poly-A tails, wherein the first poly-A tail is covalently linked to the 3' UTR.
103. A modified mRNA produced by the method of any one of clauses 98-100.
104. A delivery agent comprising the modified mRNA of any one of clauses 1-99, wherein the delivery agent comprises a lipid, a peptide, a protein, an antibody, a carbohydrate, a nanoparticle, or a microparticle.
105. The delivery agent of clause 104, wherein the nanoparticle or microparticle is a lipid nanoparticle or a lipid microparticle, a polymer nanoparticle or a polymer microparticle, a protein nanoparticle or a protein microparticle, or a solid nanoparticle or a solid microparticle.
106. A cell comprising the modified mRNA of any one of clauses 1-99.
107. The cell of clause 106, wherein the cell is a mammalian cell.
108. A composition comprising the modified mRNA of any one of clauses 1-99, the delivery agent of clause 104 or 105, or the cell of clause 106 or 107.
109. The composition of clause 108, wherein the composition further comprises an additional agent.
110. The composition of clause 109, wherein the additional agent is an agent which has a therapeutic effect when administered to a subject.
111. The composition of clause 109 or 110, wherein the additional agent is a nucleotide, a nucleic acid, an amino acid, a peptide, a protein, a small molecule, an aptamer, a lipid, or a carbohydrate.
112. The composition of clause 111, wherein the additional agent is a shRNA, a siRNA, or an ASO.
113. The composition of any one of clauses 109-111, wherein the additional agent is an antigen or adjuvant.
114. The composition of any one of clauses 108-113, wherein the composition is a pharmaceutical composition, wherein the pharmaceutical composition further comprises a pharmaceutically acceptable excipient.
115. A method comprising introducing the modified mRNA of any one of clauses 1-99 or the delivery agent of clause 104 or 105, into a cell.
116. A method comprising introducing the modified mRNA of any one of clauses 1-99, the delivery agent of clause 104 or 105, the cell of clause 106 or 107, or the composition of any one of clauses 108-114, into a subject.
117. A method of preventing a disease in a subject in need thereof comprising introducing an effective amount of the modified mRNA of any one of clauses 1-99, the delivery agent of clause 104 or 105, the cell of clause 105 or 107, or the composition of any one of clauses 108-114 into the subject, wherein the open reading frame of the modified mRNA encodes a protein.
118. A method of treating a disease in a subject in need thereof comprising introducing an effective amount of the modified mRNA of any one of clauses 1-99, the delivery agent of clause 104 or 105, the cell of clause 106 or 107, or the composition of any one of clauses 108-114 into the subject, wherein the open reading frame of the modified mRNA encodes a protein.
119. The method of clause 117 or clause 118, wherein the protein is a protein antigen or a fragment thereof from a cell or virus that causes the disease.
120. The method of clause 117 or clause 118, wherein the protein is a protein that is expressed in the subject at a level that is less than that of a reference value.
121. A method of replacing an enzyme in a subject comprising introducing the modified mRNA of any one of clauses 1-99, the delivery agent of clause 104 or 105, the cell of clause 106 or 107, or the composition of any one of clauses 108-114 into the subject, wherein the open reading frame of the modified mRNA encodes an enzyme.
122. The method of any one of clauses 116-117, wherein the subject is a human.
123. The modified mRNA of any one of clauses 1-99, the delivery agent of clause 104 or 105, the cell of clause 106 or 107, or the composition of any one of clauses 108-114 for use in preventing a disease in a subject in need thereof.
124. The modified mRNA of any one of clauses 1-99, the delivery agent of clause 104 or 105, the cell of clause 106 or 107, or the composition of any one of clauses 108-114 for use in treating a disease in a subject in need thereof.
125. A kit comprising the first RNA, and the tailing nucleic acid and/or capping nucleic acid of any one of clauses 100-102.
126. The kit of clause 125 further comprising an RNA ligase.
127. A kit comprising the pharmaceutical composition of clause 114, a device for administering the pharmaceutical composition to a subject, and instructions for administering the pharmaceutical composition to a subject.

## Claims

1. A modified mRNA comprising:
(i) an open reading frame (ORF) encoding a protein;
(ii) a poly-A region, and
(iii) a 5' cap region;
wherein the poly-A region is 3' to the ORF and comprises 10 or more nucleotides and the 5' cap region is 5' to the ORF; and
wherein the 5' cap region comprises two or more 5' caps.

2. The modified mRNA of claim 1, wherein the modified mRNA comprises a poly-A region comprising one poly-A tail and a 5' cap region comprising two or more 5' caps.

3. The modified mRNA of claim 1 or 2, wherein the modified mRNA comprises a 5' untranslated region (5' UTR) and a 3' untranslated region (3' UTR), wherein the ORF is between the 5' UTR and the 3' UTR, wherein the 3' UTR is between the ORF and the poly-A region, and wherein the 5' UTR is between the 5' cap region and the ORF.

4. The modified mRNA of any one of claims 1-3, wherein the modified mRNA is a linear mRNA, wherein the 5' cap region is at the 5' end of the modified mRNA and the poly-A region is at the 3' end of the modified mRNA, or wherein the modified mRNA is a circular mRNA, wherein the poly-A region is between the 3' UTR and the 5' cap region.

5. The modified mRNA of any one of claims 1-4, wherein the 5' cap region comprises two or more 5' caps, and at least two instances of the 5' caps are covalently linked by a linker.

6. The modified mRNA of claim 5, wherein:
the 5' cap region comprises -(a first 5' cap)-[(a linker)-(a second 5' cap)ₘ₁]ₘ₂;
each instance of m1 is independently an integer between 1 and 20, inclusive;
m2 is an integer between 2 and 10, inclusive;
the first 5' cap is covalently linked to the 5' UTR; and
the linker is covalently linked to the first 5' cap and to the second 5' cap.

7. The modified mRNA of claim 6, wherein each instance of m1 is 1 or is independently an integer between 2 and 20, inclusive, and optionally wherein at least one instance of the linker comprises a dendrimer.

8. The modified mRNA of any one of claims 5-7, wherein at least one instance of the linker is covalently linked to a 5' or 3' nucleotide of the first 5' cap.

9. The modified mRNA of any one of claims 5-8, wherein at least one instance of the linker is covalently linked to a 5' or 3' nucleotide of the at least one instance of the second 5' cap.

10. The modified mRNA of any one of claims 1-9, wherein the 5' cap region of the modified mRNA comprises one or more modified nucleotides.

11. The modified mRNA of any one of claims 1-9, wherein the 5' cap region comprises between 25-500 nucleotides.

12. The modified mRNA of any one of claims 1-10, wherein the 5' cap region comprises between 1 and 3, between 3 and 5, between 5 and 7, or between 7 and 10 5' caps.

13. The modified mRNA of any one of claims 1-12, wherein the 5' cap region comprises one or more modified 5' caps.

14. The modified mRNA of claim 13, wherein the one or more modified 5' caps are selected from a locked nucleic acid (LNA)-modified cap, a 5' cap comprising a 5'-5' triphosphate linkage that is modified by 5'-phosphorothiolate, and a 5' cap comprising a 5'-5' tetraphosphate linkage, and a 5' cap comprising one or more modified nucleotides.

15. The modified mRNA of any one of claims 1-14, for use in preventing or treating a disease in a subject in need thereof.
